# EUROPEAN PATENT APPLICATION

(11) **EP 4 643 838 A1**
(43) Date of publication of application: **05.11.2025**
(21) Application number: 24836190.9
(22) Date of filing: 21.05.2024
(51) Int. Cl.: A61H 1/02, A61H 3/00, A61B 5/11, A63B 21/065

(54) **WEARABLE APPARATUS PROVIDING EXERCISE MODE BASED ON DISEASE OF USER AND OPERATING METHOD OF WEARABLE APPARATUS**

(30) Priority: 05.07.2023 KR 20230087172
(71) Applicant: Samsung Electronics Co., Ltd., Suwon-si, Gyeonggi-do 16677 (KR)
(72) Inventor: KANG, Hyunjoo, Suwon-si Gyeonggi-do 16677 (KR); LEE, Seungjoon, Suwon-si Gyeonggi-do 16677 (KR); CHO, Heeyoung, Suwon-si Gyeonggi-do 16677 (KR)
(74) Representative: Gulde & Partner
(86) International application number: PCT/KR2024/006873
(87) International publication number: WO 2025/009741

(57) **Abstract**

This wearable apparatus can: acquire information related to recognizing a disease of a user; recognize the disease of the user through the acquired information; determine an exercise mode of the wearable apparatus on the basis of the recognized disease; determine a first torque value on the basis of the values of parameters and a first angle value obtained by measuring the angle of a joint of the user in the determined exercise mode; generate a first torque corresponding to the determined first torque value through a driving module of the wearable apparatus and provide the first torque to the user; and when the start of a specified movement is detected in the determined exercise mode on the basis of a second angle value obtained by measuring the angle of the joint, perform a control operation for generating a second torque, to be generated by the driving module, so that the second torque acts as resistance to the specified movement.

## Description

### BACKGROUND

### 1. Field

Certain embodiments relate to a wearable device for providing an exercise mode based on a disease and/or state of a user, and/or an operating method thereof.

### 2. Description of Related Art

In general, a walking assistance device refers to a mechanism or device that helps a person to exercise and/or which helps a patient, who cannot walk on his/her own due to various diseases, accidents, and the like, to perform walking exercises for rehabilitation treatment. With the recent intensifying aging societies, a growing number of people experience inconvenience in walking or have difficulty in normal walking due to malfunctioning joint issues, and there is increasing interest in walking assistance devices. A walking assistance device may be worn on a body of a user to assist the user with exercise and/or with walking by providing a necessary or desired muscular strength and to induce the user to walk in a normal walking pattern.

### SUMMARY

According to an embodiment, a wearable device may include an angle sensor configured to measure an angle of a joint of a user, a driving module, comprising a motor and/or circuitry, configured to generate a torque and provide the torque to the user, and at least one processor, comprising processing circuitry, configured to control the driving module. The at least one processor may obtain information for recognizing a disease of the user, and recognize a disease of the user based on the obtained information. The at least one processor may determine an exercise mode of the wearable device based on the recognized disease. The at least one processor may receive a first angle value obtained by measuring the angle in the determined exercise mode from the angle sensor. The at least one processor may determine a first torque value based on the received first angle value and values of parameters. The at least one processor may control the driving module to generate a first torque corresponding to the determined first torque value. When a start of a designated motion in the determined exercise mode is detected based on a second angle value obtained by measuring the angle, the at least one processor may perform a control operation for generating a second torque so that the second torque generated by the driving module may act as a resistance to the designated motion.

According to an embodiment, a wearable device may include a communication module, comprising communication circuitry, configured to communicate with a user terminal, an angle sensor configured to measure an angle of a joint of a user, a driving module, comprising a motor and/or circuitry, configured to generate a torque and provide the torque to the user, and at least one processor comprising processing circuitry. The processor(s) may receive a control signal indicating an exercise mode of the wearable device from the user terminal through the communication module. The processor(s) may determine an exercise mode of the wearable device based on the received control signal. The processor(s) may receive a first angle value obtained by measuring the angle in the determined exercise mode from the angle sensor. The processor(s) may determine a first torque value based on the received first angle value and values of parameters. The processor(s) may control the driving module to generate a first torque corresponding to the determined first torque value. When a start of a designated motion in the determined exercise mode is detected based on a second angle value obtained by measuring the angle, the processor(s) may perform a control operation for generating a second torque so that the second torque generated by the driving module may act as a resistance to the designated motion.

According to an embodiment, an operating method of a wearable device may include obtaining information necessary or helpful for recognizing a disease of a user and recognizing a disease of the user through the obtained information, determining an exercise mode of the wearable device based on the recognized disease, determining a first torque value based on a first angle value obtained by measuring an angle of a joint of the user in the determined exercise mode and values of parameters, generating a first torque corresponding to the determined first torque value and providing the first torque to the user, through a driving module of the wearable device, and when a start of a designated motion in the determined exercise mode is detected based on a second angle value obtained by measuring the angle of the joint, performing a control for generating a second torque so that the second torque generated by the driving module may act as a resistance to the designated motion.

According to an embodiment, a wearable device may provide an exercise mode based on a disease of a user, and determine whether the user is capable of performing an exercise through information (e.g., blood glucose data, blood pressure data, whether the user has eaten food, a pain state, etc.) that needs to be checked before starting exercising for each disease.

According to an embodiment, a wearable device may provide an exercise guide or exercise schedule specific to a user who has a disease.

### BRIEF DESCRIPTION OF THE DRAWINGS

The above and other aspects, features, and advantages of certain embodiments of the present disclosure will be more apparent from the following detailed description, taken in conjunction with the accompanying drawings, in which:
FIG. 1A is a diagram illustrating an overview of a wearable device worn on a body of a user according to an embodiment.
FIG. 1B is a diagram illustrating an example of a system including a wearable device according to an embodiment.
FIG. 2A is a rear schematic view of a wearable device according to an embodiment.
FIG. 2B is a left side view of a wearable device according to an embodiment.
FIGS. 3A and 3B are block diagrams illustrating examples of a configuration of a wearable device according to an embodiment.
FIG. 4 is a diagram illustrating an interaction between a wearable device and an electronic device according to an embodiment.
FIG. 5 is a diagram illustrating an example of a configuration of an electronic device according to an embodiment.
FIG. 6 is a diagram illustrating an example of an operation of a wearable device based on a disease of a user according to an embodiment.
FIG. 7 is a diagram illustrating another example of an operation of a wearable device based on a disease of a user according to an embodiment.
FIGS. 8A and 8B are diagrams illustrating other examples of an operation of a wearable device based on a disease of a user according to an embodiment.
FIG. 9 is a diagram illustrating another example of an operation of a wearable device based on a disease of a user according to an embodiment.
FIG. 10 is a diagram illustrating another example of an operation of a wearable device based on a disease of a user according to an embodiment.
FIGS. 11A, 11B, 11C, 12A, 12B, and 12C are diagrams illustrating examples of an operation of a wearable device in a damping exercise mode according to an embodiment(s).
FIGS. 13A, 13B, and 13C are diagrams illustrating examples of an operation of a wearable device in a damping exercise mode according to an embodiment(s).
FIGS. 14A, 14B, 15A, and 15B are diagrams illustrating examples of an operation of a wearable device in a muscle strengthening exercise mode according to an embodiment(s).
FIG. 16 is a diagram illustrating an example of an operation of a wearable device in a muscle strengthening exercise mode according to an embodiment.
FIG. 17 is a diagram illustrating an example of an operation of a wearable device in a muscle strengthening exercise mode according to an embodiment.
FIG. 18 is a flowchart illustrating an example of an exercise schedule generation method of a wearable device according to an embodiment.
FIG. 19 is a diagram illustrating an example of an operation of a wearable device according to an embodiment.
FIG. 20 is a diagram illustrating an example of an operation of a wearable device according to an embodiment.
FIG. 21 is a diagram illustrating an example of an operation of a wearable device according to an embodiment.
FIG. 22 is a diagram illustrating an example of an operation of a wearable device according to an embodiment.
FIG. 23 is a flowchart illustrating an example of an operating method of a wearable device according to an embodiment.
FIG. 24 is a flowchart illustrating an example of an operating method of a wearable device according to an embodiment.

### DETAILED DESCRIPTION

The following detailed structural or functional description is provided as an example only and various alterations and modifications may be made to the embodiments. Here, the embodiments are not construed as limited to the disclosure and should be understood to include all changes, equivalents, and replacements within the idea and the technical scope of the disclosure.

Terms, such as first, second, and the like, may be used herein to describe components. Each of these terminologies is not used to define an essence, order or sequence of a corresponding component but used merely to distinguish the corresponding component from other component(s). For example, a first component may be referred to as a second component, and similarly the second component may also be referred to as the first component.

It should be noted that if it is described that one component is "connected", "coupled", or "joined" to another component, at least a third component may be "connected", "coupled", and "joined" between the first and second components, although the first component may be directly connected, coupled, or joined to the second component.

As used herein, the singular forms "a", "an", and "the" include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms, including technical and scientific terms, used herein have the same meaning as commonly understood by one of ordinary skill in the art to which this disclosure pertains. Terms, such as those defined in commonly used dictionaries, are to be interpreted as having a meaning that is consistent with their meaning in the context of the relevant art, and are not to be interpreted in an idealized or overly formal sense unless expressly so defined herein.

Hereinafter, embodiments will be described in detail with reference to the accompanying drawings. When describing the embodiments with reference to the accompanying drawings, like reference numerals refer to like elements and any repeated description related thereto will be omitted.

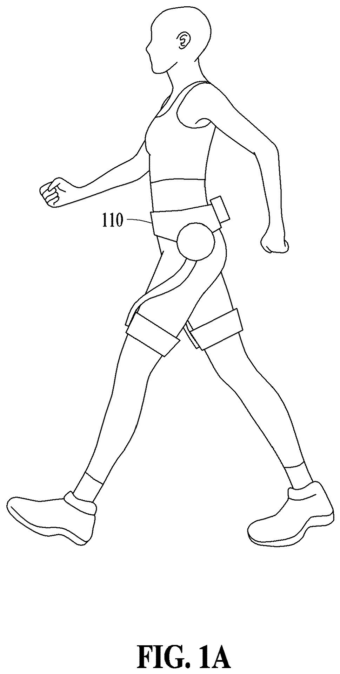

FIG. 1A is a diagram illustrating an overview of a wearable device worn on a body of a user according to an embodiment.

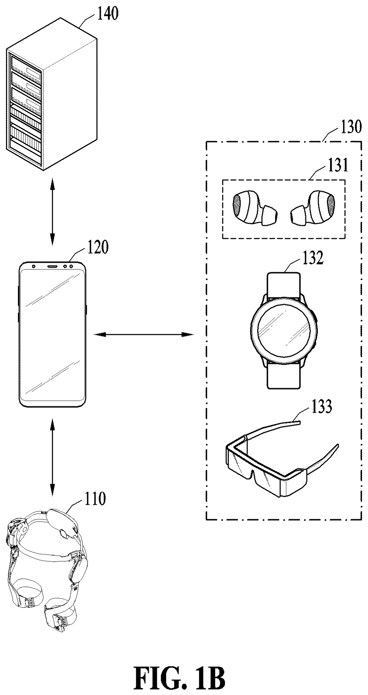

Referring to FIG. 1, a wearable device 110 may be a device worn on a body of a user to assist the user in walking, exercising, and/or working. In embodiments, the term "wearable device" may be replaced with "wearable robot," "walking assistance device," or "exercise assistance device". The user may be a human or an animal, but is not limited thereto. The wearable device 110 may be worn on a body (e.g., a lower body (the legs, ankles, knees, etc.), an upper body (the torso, arms, wrists, etc.), or the waist) of the user to provide an external force such as an assistance force and/or a resistance force to a body motion of the user. The assistance force may be a force applied in the same direction as the body motion direction of the user, and the resistance force may be a force applied in a direction opposite to the body motion direction of the user. The term "resistance force" may also be referred to as "exercise load".

When the wearable device 110 performs a walking assist function to assist the user in walking, the wearable device 110 may assist a portion or entirety of a leg of the user by providing an assistance force to the body of the user, thereby assisting the user in walking. The wearable device 110 may enable the user to walk independently or to walk for a long time by providing a force required for the user to walk, thereby extending the walking ability of the user. The wearable device 110 may help in improving an abnormal walking habit or gait posture of a walker.

When the wearable device 110 performs an exercise function to enhance the exercise effect of the user, the wearable device 110 may hinder a body motion of the user or provide resistance to a body motion of the user by providing a resistance force to the body of the user. When the wearable device 110 is, for example, a hip-type wearable device, the wearable device 110 may provide an exercise load to a body motion of the user while being worn on the legs, thereby enhancing the exercise effect of the user. The user may perform a walking motion while wearing the wearable device 110 for exercise. In this case, the wearable device 110 may apply a resistance force to the leg motion during the walking motion of the user.

In various embodiments of the present disclosure, an example of a hip-type wearable device 110 that is worn on the waist and legs is described for ease of description. However, as described above, the wearable device 110 may be worn on another body part (e.g., the upper arms, lower arms, hands, calves, and feet) other than the waist and legs (particularly, the thighs), and the shape and configuration of the wearable device 110 may vary depending on the body part on which the wearable device 110 is worn.

FIG. 1B is a diagram illustrating an example of a system including a wearable device according to an embodiment.

Referring to FIG. 1B, an electronic device 120 may communicate with the wearable device 110 and remotely control the wearable device 110. The electronic device 120 may be various types of devices. The electronic device 120 may include, for example, a portable communication device (e.g., a smart phone), a computer device, a portable multimedia device, or a home appliance, but is not limited thereto.

According to an embodiment, the electronic device 120 and/or the wearable device 110 may be connected to another wearable device 130. For example, the wearable device 110, the electronic device 120, and the other wearable device 130 may be connected to each other through a wireless communication link (e.g., a Bluetooth communication link). The other wearable device 130 may include, for example, wireless earphones 131, a smart watch 132, or smart glasses 133, but is not limited thereto. The smart watch 132 may be a watch-type wearable device (or a watch-type electronic device), and the smart glasses 133 may be an eyewear-type wearable device (or an eyewear-type electronic device).

In an embodiment, the smart watch 132 may control the wearable device 110. When the smart watch 132 is connected to the electronic device 120 through a wireless communication link, and the electronic device 120 is connected to the wearable device 110 through a wireless communication link, the smart watch 132 may control the wearable device 110 through the electronic device 120. Embodiments are not limited thereto, and the smart watch 132 may be directly connected to the wearable device 110 and control the wearable device 110.

In an embodiment, the electronic device 120 may transmit, to the other wearable device 130, a control signal to instruct to provide a user with feedback corresponding to a state of the wearable device 110. The other wearable device 130 may provide (or output) feedback (e.g., at least one of visual feedback, auditory feedback, or tactile feedback) corresponding to the state of the wearable device 110 in response to the reception of the control signal.

In an embodiment, the electronic device 120 may communicate with a server 140 using short-range wireless communication (e.g., Wi-Fi) or mobile communication (e.g., 4G, 5G, etc.).

In an embodiment, the electronic device 120 may receive profile information of the user from the user. The profile information may include, for example, at least one of the age, gender, height, weight, or body mass index (BMI), or a combination thereof. The electronic device 120 may transmit the profile information of the user to the server 140.

In an embodiment, the electronic device 120 and/or the wearable device 110 may request the user to perform one or more target motions to determine (or check) the exercise ability of the user. The one or more target motions may include, for example, a knee lift, a backward leg stretch, etc. The knee lift may be an exercise (or a motion) that starts from a position of the user standing straight with two feet on the ground and returns to the standing position after lifting a leg backward as much as possible without bending at the waist. The backward leg stretch may be an exercise (or a motion) that starts from a position of the user standing straight with the hands on the wall and returns to the standing position after lifting a leg backward as much as possible without bending at the waist.

In an embodiment, the wearable device 110 may obtain motion information of the user performing a target motion using a sensor (e.g., an inertial measurement unit (IMU)), and transmit the obtained motion information to the electronic device 120. The electronic device 120 may transmit the obtained motion information to the server 140.

In an embodiment, the server 140 may determine a target amount of exercise of the user for each of the exercise types (e.g., strength training, balance exercise, and aerobic exercise) through the profile information and motion information received from the electronic device 120. The server 140 may transmit the target amount of exercise for each exercise type to the electronic device 120.

In an embodiment, the server 140 may include a database in which information about a plurality of exercise programs to be provided to the user is stored. For example, the server 140 may manage a user account of the user of the electronic device 120 or the wearable device 110. The server 140 may store and manage a workout program performed by the user and a result of performance with respect to the workout program in link with the user account.

In an embodiment, at least one of the wearable device 110, the electronic device 120, or the server 140 may provide the user with various exercise programs to achieve an exercise goal in various exercise environments desired by the user. The exercise goal may include, for example, at least one of muscle strength improvement, physical strength improvement, cardiovascular endurance improvement, core stability improvement, flexibility improvement, or symmetry improvement, or a combination thereof.

In an embodiment, at least one of the wearable device 110, the electronic device 120, or the server 140 may recommend exercise programs to the user to achieve the exercise goal of the user. Each exercise program may include one or more exercise types (e.g., running, a lunge, etc.) to achieve an exercise goal. For example, running may be an exercise type for improving the cardiovascular endurance of the user. For example, a lunge may be an exercise type for improving the core stability of the user. A combination of exercise types forming each exercise program may vary according to the exercise goal of the user. The electronic device 120 may provide the user with various exercise programs according to the combination of the plurality of exercise types, even for the same exercise goal.

In an embodiment, the plurality of exercise types may be stored in at least one of the wearable device 110, the electronic device 120, or the server 140 as a database. At least one of the wearable device 110, the electronic device 120, or the server 140 may generate the plurality of exercise programs based on a variety of information about the user and recommend a target exercise program among the plurality of exercise programs to the user in consideration of the exercise goal or an exercise performance state of the user. For example, at least one of the wearable device 110, the electronic device 120, or the server 140 may determine the target exercise program to recommend to the user based on at least one of the exercise goal, an exercise history, or an exercise performance result of the user. Accordingly, a new exercise program may be recommended to the user even if the user performs an exercise every day under the same exercise goal, and the user may feel like performing a different exercise from the previous exercise by performing the new exercise program.

In an embodiment, the server 140 may store at least one or all of a disease code of the user, medical record information of the user, or exercise history information of the user.

For example, the server 140 may receive the disease code of the user from a hospital and/or insurance company, and store the disease code of the user. Each disease may be assigned a disease code (or a disease classification code) to classify the disease. If the user has a disease, the hospital and/or insurance company may have records of the disease code for the disease of the user. The server 140 may link with the hospital and/or insurance company to receive and store the disease code of the user.

The medical record information may include, for example, at least one of medical history (e.g., telemedicine history and/or history of treatment with hospital visits), medication history, or medical check-up history of the user. The medical history may include, for example, the blood pressure data, blood glucose data, and the like measured when the user visits the hospital. The medical check-up history may include, for example, at least one of the blood pressure data, blood glucose data, heart rate data, cholesterol level data, or triglyceride level data measured during a medical check-up. A health care application to manage medical record information may be installed on the electronic device 120 (or the smart watch 132). A server of the health care application may store the medical record information of the user, and the server 140 may link with the server of the health care application to store the medical record information of the user.

The exercise history information may include, for example, an evaluation result (e.g., an exercise duration, a walking symmetry, etc.) of exercises that the user has previously performed while wearing the wearable device 110. The server 140 may receive the evaluation result of the exercises from the wearable device 110 (or the electronic device 120) and store the evaluation result of the exercises.

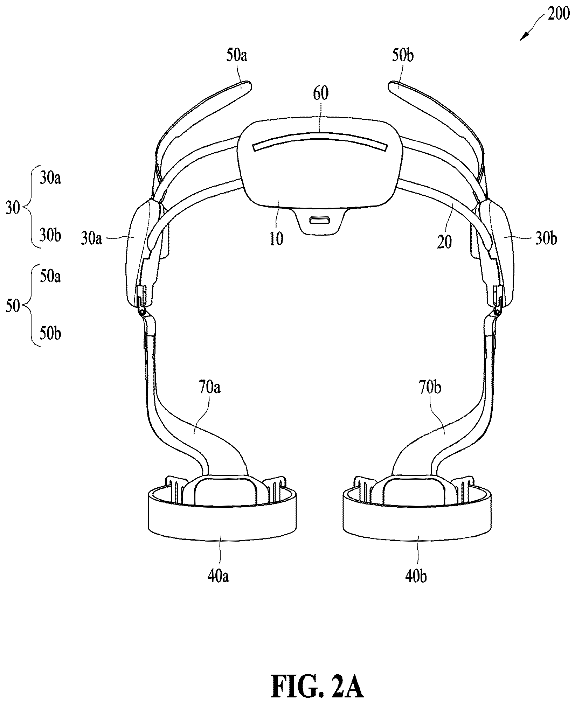

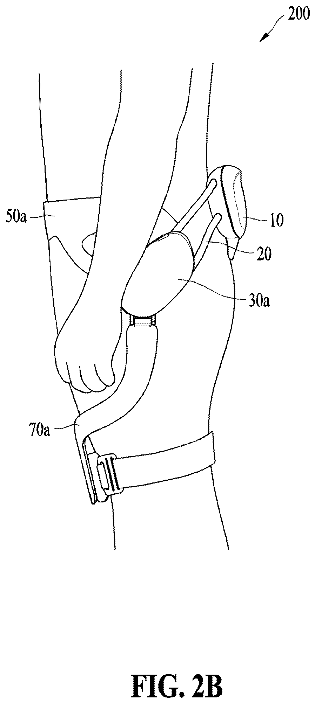

FIG. 2A is a rear schematic view of a wearable device according to an embodiment. FIG. 2B is a left side view of the wearable device according to an embodiment.

A wearable device 200 shown in FIGS. 2A and 2B may be an example of the wearable device 110.

Referring to FIG. 2A, according to an embodiment, the wearable device 200 may include a base body 10, a base frame 20, a driving module 30, thigh fastening portions 40a and 40b, a main belt 50, and leg driving frames 70a and 70b. Each "driving module" herein may comprise a motor and/or circuitry.

According to an embodiment, the base body 10 may be positioned on the lumbar region (an area of the lower back) of the user while the user is wearing the wearable device 200. The base body 10 may be mounted on the lumbar region of the user to provide a cushioning feeling to the lower back of the user and may support the lower back of the user. The base body 10 may be hung on the hip region (an area of the hips) of the user to prevent or reduce chances of the wearable device 200 from being separated downward due to gravity while the user is wearing the wearable device 200. The base body 10 may distribute a portion of the weight of the wearable device 200 to the lower back of the user while the user is wearing the wearable device 200. The base body 10 may be connected, directly or indirectly, to the base frame 20. Base frame connecting elements (not shown) to be connected, directly or indirectly, to the base frame 20 may be provided at both end portions of the base body 10.

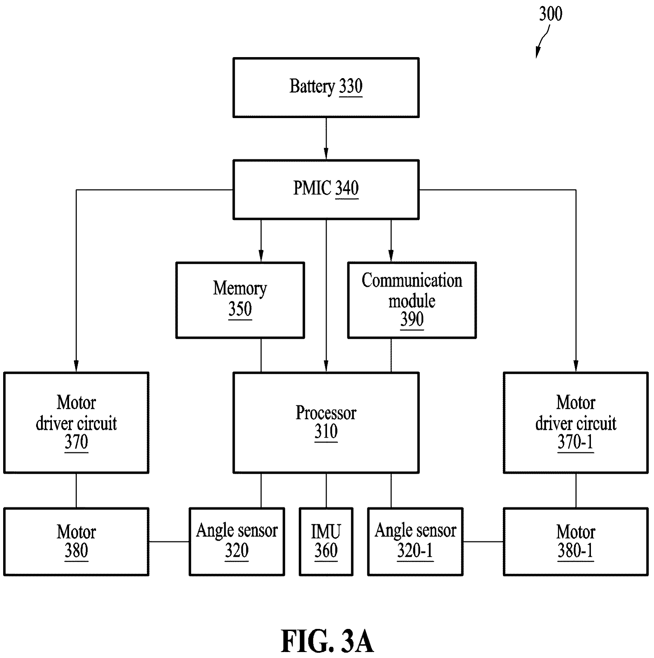

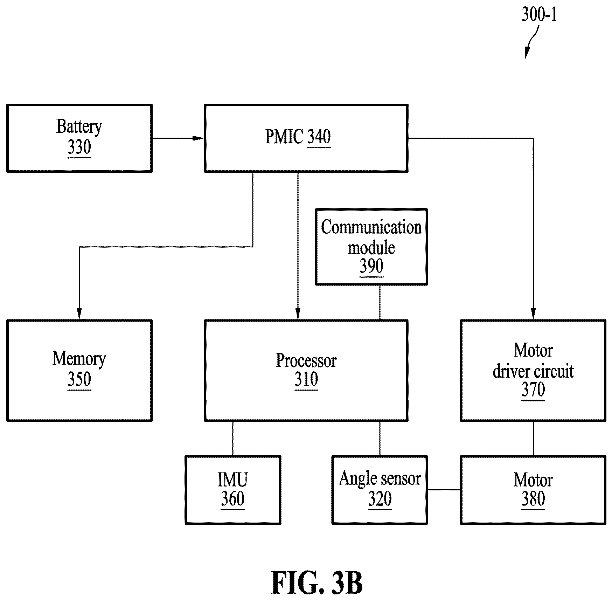

According to an embodiment, the base body 10 may include a lighting unit 60. The lighting unit 60 may include a plurality of light sources (e.g., light-emitting diodes (LEDs)). The lighting unit 60 may emit light by control of a processor (e.g., a processor 310 of FIGS. 3A and 3B). According to embodiments, the processor may control the lighting unit 60 such that visual feedback corresponding to the state of the wearable device 200 (e.g., a booting state, a sensing state, etc.) may be provided (or output) to the user through the lighting unit 60.

According to an embodiment, the base frame 20 may extend from both end portions of the base body 10. The lumbar region of the user may be accommodated inside the base frame 20. The base frame 20 may include at least one rigid body beam. Each beam may be in a curved shape having a preset curvature to enclose the lumbar region of the user. The main belt 50 may be connected, directly or indirectly, to an end portion of the base frame 20. The driving module 30 may be mounted on the base frame 20. The base frame 20 may include a connector (not shown) for mounting the driving module 30 thereon.

According to an embodiment, the driving module 30 may include a first driving module 30a positioned on the left side of the user while the user is wearing the wearable device 200, and a second driving module 30b positioned on the right side of the user while the user is wearing the wearable device 200.

According to an embodiment, the first driving module 30a may include a first angle sensor (e.g., a first encoder or a first Hall sensor) for measuring the left hip joint angle of the user. The second driving module 30b may include a second angle sensor (e.g., a second encoder or a second Hall sensor) for measuring the right hip joint angle of the user.

According to an embodiment, the first driving module 30a may include a first actuator and a first reducer, and the second driving module 30b may include a second actuator and a second reducer. An output end of the first actuator may be connected, directly or indirectly, to an input end of the first reducer, and an output end of the second actuator may be connected, directly or indirectly, to an input end of the second reducer.

According to an embodiment, the processor (e.g., the processor 310 described later) may determine a torque value (e.g., a torque value *τₗ*(*t*) to be described later) and control the first actuator to generate a torque based on the determined torque value (e.g., *τₗ*(*t*)). With this control, the first actuator may generate the torque, and the generated torque may be reduced by the first reducer. The torque reduced by the first reducer may rotate the first leg driving frame 70a. The torque reduced by the first reducer may be provided, for example, to the left leg of the user through the first leg driving frame 70a. The processor (e.g., the processor 310 described later) may determine a torque value (e.g., a torque value *τ_{R}*(*t*) to be described later) and control the second actuator to generate a torque based on the determined torque value (e.g., *τ_{R}*(*t*)). With this control, the second actuator may generate the torque, and the generated torque may be reduced by the second reducer. The torque reduced by the second reducer may rotate the second leg driving frame 70b. The torque reduced by the second reducer may be provided, for example, to the right leg of the user through the second leg driving frame 70b.

According to an embodiment, the leg driving frames 70a and 70b may support the legs (e.g., thighs) of the user when the wearable device 200 is worn on the legs of the user. The leg driving frames 70a and 70b may include the first leg driving frame 70a configured to support the left leg of the user and the second leg driving frame 70b configured to support the right leg of the user.

According to an embodiment, the leg driving frames 70a and 70b may, for example, transmit the torques generated by the driving modules 30a and 30b (e.g., the torques reduced by the reducers) to the thighs of the user. As one end portions of the leg driving frames 70a and 70b are connected, directly or indirectly, to the driving modules 30a and 30b to rotate, and the other end portions of the leg driving frames 70a and 70b are connected, directly or indirectly, to the thigh fastening portions 40a and 40b, the leg driving frames 70a and 70b may transmit the torques generated by the driving modules 30a and 30b to the thighs of the user while supporting the thighs of the user. For example, the leg driving frames 70a and 70b may push or pull the thighs of the user. The leg driving frames 70a and 70b may extend in the longitudinal direction of the thighs of the user. The leg driving frames 70a and 70b may be bent to surround at least a portion of the circumferences of the thighs of the user.

According to an embodiment, the thigh fastening portions 40a and 40b may be connected, directly or indirectly, to the leg driving frames 70a and 70b and may fasten the leg driving frames 70a and 70b to the thighs. The thigh fastening portions 40a and 40b may include a first thigh fastening portion 40a configured to fasten the first leg driving frame 70a to the left thigh of the user and a second thigh fastening portion 40b configured to fasten the second leg driving frame 70b to the right thigh of the user.

According to an embodiment, the first thigh fastening portion 40a may include a first cover, a first fastening frame, and a first strap, and the second thigh fastening portion 40b may include a second cover, a second fastening frame, and a second strap. The first cover and the second cover may be arranged on one sides of the thighs of the user. The first cover and the second cover may be arranged on the front surfaces of the thighs of the user. The first cover and the second cover may be arranged in the circumferential directions of the thighs of the user. The first cover and the second cover may extend to both sides from the other end portions of the leg driving frames 70a and 70b and may include curved surfaces corresponding to the thighs of the user. One ends of the first cover and the second cover may be connected, directly or indirectly, to the fastening frames, and the other ends thereof may be connected, directly or indirectly, to the straps.

According to an embodiment, the first fastening frame and the second fastening frame may be arranged, for example, to surround at least some portions of the circumferences of the thighs of the user, thereby preventing or reducing chances of the thighs of the user from being separated from the leg driving frames 70a and 70b. The first fastening frame may have a fastening structure that connects the first cover and the first strap, and the second fastening frame may have a fastening structure that connects the second cover and the second strap.

According to an embodiment, the first strap may enclose the remaining portion of the circumference of the left thigh of the user that is not covered by the first cover and the first fastening frame, and the second strap may enclose the remaining portion of the circumference of the right thigh of the user that is not covered by the second cover and the second fastening frame. The first strap and the second strap may include, for example, an elastic material (e.g., a band).

According to an embodiment, the main belt 50 may be connected, directly or indirectly, to the base frame 20. The main belt 50 may include a first main belt 50a configured to enclose the left abdomen of the user while the user is wearing the wearable device 200, and a second main belt 50b configured to enclose the right abdomen of the user while the user is wearing the wearable device 200. The first main belt 50a may be formed in a shape having a longer length than the second main belt 50b, but is not limited thereto, and the first main belt 50a may be formed in a shape having the same length as or a shorter length than the second main belt 50b. The first main belt 50a and the second main belt 50b may be connected, directly or indirectly, to both end portions of the base frame 20, respectively. The main belt 50 may be bent in a direction to surround the abdomen of the user when the body of the user is inserted in such a direction that it is accommodated in the wearable device 200. The first main belt 50a and the second main belt 50b may be connected, directly or indirectly, to each other while the user is wearing the wearable device 200. The main belt 50 may distribute a portion of the weight of the wearable device 200 to the abdomen of the user while the user is wearing the wearable device 200.

Referring to FIG. 2B, the base body 10 may be mounted on the back of the lumbar region of the user and be hung on the hip region of the user, thereby supporting a portion of the weight of the wearable device 200. The first driving module 30a may be arranged on the left lumbar region of the user. The base frame 20 may extend from the end portion of the base body 10 and be inclined in a direction toward the first driving module 30a. The first main belt 50a mounted on the base frame 20 may surround the left abdomen of the user.

FIGS. 3A and 3B are block diagrams illustrating examples of a configuration of a wearable device according to an embodiment.

According to an embodiment, a wearable device 300 of FIG. 3A may include the processor 310, angle sensors 320 and 320-1, a battery 330, a power management integrated circuit (PMIC) 340, a memory 350, an IMU 360, motor driver circuits 370 and 370-1, motors 380 and 380-1 (e.g., the first and second actuators described with reference to FIG. 2A), and a communication module 390.

Although the plurality of angle sensors 320 and 320-1, the plurality of motor driver circuits 370 and 370-1, and the plurality of motors 380 and 380-1 are shown in FIGs. 3A-3B, which is merely an example, the wearable device 300-1 in the example shown in FIG. 3B may include a single angle sensor 320, a single motor driver circuit 370, and a single motor 380. Also, according to the implementation, the wearable devices 300 and 300-1 may include a plurality of processors. The number of motor driver circuits, the number of motors, or the number of processors may vary depending on a body part on which the wearable devices 300 and 300-1 are worn.

The wearable device 300 in FIG. 3A and the wearable device 300-1 of FIG. 3B may be examples of the wearable device 110 of Figs. 1A-1B and the wearable device 200 of Figs. 2A-2B.

According to an embodiment, the angle sensor 320, the motor driver circuit 370, and the motor 380 may be included in the first driving module 30a of FIG. 2A, and the angle sensor 320-1, the motor driver circuit 370-1, and the motor 380-1 may be included in the second driving module 30b of FIG. 2A.

According to an embodiment, the angle sensor 320 and the angle sensor 320-1 may each correspond to a Hall sensor, but are not limited thereto.

According to an embodiment, the angle sensor 320 may measure or sense at least one of the angle, angular velocity, or angular acceleration of a first joint (e.g., the left hip joint, etc.) of the user. The angle sensor 320 may transmit the measurement result (e.g., at least one of the angle value, angular velocity value, or angular acceleration value of the first joint) to the processor 310. For example, the angle sensor 320 may obtain the angular acceleration value by measuring the angular acceleration of the left hip joint angle of the user, and transmit the obtained angular acceleration value to the processor 310. Embodiments are not limited thereto, and the angle sensor 320 may obtain the angle value or angular velocity value by measuring the angle or angular velocity of the left hip joint angle of the user, and transmit the obtained angle value or angle velocity value to the processor 310. The processor 310 may calculate the angular acceleration value of the left hip joint angle through the angle value or angular velocity value received from the angle sensor 320.

According to an embodiment, the angle sensor 320-1 may measure or sense at least one of the angle, angular velocity, or angular acceleration of a second joint (e.g., the right hip joint) of the user. The angle sensor 320-1 may transmit the measurement result (e.g., at least one of the angle value, angular velocity value, or angular acceleration value of the second joint) to the processor 310. For example, the angle sensor 320-1 may obtain the angular acceleration value by measuring the angular acceleration of the right hip joint angle of the user, and transmit the obtained angular acceleration value to the processor 310. Embodiments are not limited thereto, and the angle sensor 320-1 may obtain the angle value or angular velocity value by measuring the angle or angular velocity of the right hip joint angle of the user, and transmit the obtained angle value or angle velocity value to the processor 310. The processor 310 may calculate the angular acceleration value of the right hip joint angle through the angle value or angular velocity value received from the angle sensor 320-1.

According to an embodiment, the angle sensor 320 and the angle sensor 320-1 may additionally measure the knee angles and ankle angles of the user according to the positions of the angle sensor 320 and the angle sensor 320-1.

According to an embodiment, the wearable devices 300 and 300-1 may include a potentiometer. The potentiometer may sense an R-axis joint angle, an L-axis joint angle, an R-axis joint angular velocity, and an L-axis joint angular velocity according to a walking motion of the user. In this example, the R and L axes may be reference axes for the right leg and the left leg of the user, respectively. For example, the R/L axis may be set to be vertical to the ground and set such that a front side of a body of a person has a negative value and a rear side of the body has a positive value.

According to an embodiment, the PMIC 340 may charge the battery 330 using power supplied from an external power source. For example, the external power source and the wearable devices 300 and 300-1 may be connected through a cable (e.g., a universal serial bus (USB) cable, etc.). The PMIC 340 may receive power from the external power source through the cable, and charge the battery 330 using the received power. According to embodiments, the PMIC 340 may charge the battery 330 through a wireless charging method.

According to an embodiment, the PMIC 340 may transmit the power stored in the battery 330 to the components (e.g., the processor 310, the angle sensors 320 and 320-1, the memory 350, the IMU 360, the motors 380 and 380-1, etc.) in the wearable devices 300 and 300-1. The PMIC 340 may, for example, adjust the power stored in the battery 330 to a voltage or current level suitable for the components in the wearable device 300. The PMIC 340 may include, for example, a converter (e.g., a direct current (DC)-DC converter) or a regulator (e.g., a low-dropout (LDO) regulator or a switching regulator) configured to perform the adjustment described above.

According to an embodiment, the PMIC 340 may determine state information (e.g., a state of charge, a state of health, an overvoltage, a low voltage, an overcurrent, an overcharge, an overdischarge, an overheating, a short circuit, or a swelling) of the battery 330, and transmit the state information of the battery 330 to the processor 310. The processor 310 may control to provide the state information of the battery 330 to the user. For example, the processor 310 may output the state information of the battery 330 through at least one of a sound output module, a vibration output module, or a display module described below. For example, the processor 310 may transmit the state information of the battery 330 to the electronic device 120 through the communication module 390, and the electronic device 120 may display the state information of the battery 330 on the display.

According to an embodiment, the IMU 360 may obtain or measure motion information of the user. For example, the IMU 360 may measure or obtain 3-axis (e.g., x-axis, y-axis, and z-axis) accelerations and rotation angles (e.g., roll, pitch, and yaw) according to a walking motion of the user. The IMU 360 may transmit the obtained motion information (e.g., the measured 3-axis accelerations and rotation angles) to the processor 310.

According to an embodiment, the processor 310 may control the overall operation of the wearable devices 300 and 300-1.

Each "processor" herein includes processing circuitry, and/or may include multiple processors. For example, as used herein, including the claims, the term "processor" may include various processing circuitry, including at least one processor, wherein one or more of at least one processor, individually and/or collectively in a distributed manner, may be configured to perform various functions described herein. As used herein, when "a processor", "at least one processor", and "one or more processors" are described as being configured to perform numerous functions, these terms cover situations, for example and without limitation, in which one processor performs some of recited functions and another processor(s) performs other of recited functions, and also situations in which a single processor may perform all recited functions. Additionally, the at least one processor may include a combination of processors performing various of the recited /disclosed functions, e.g., in a distributed manner. At least one processor may execute program instructions to achieve or perform various functions.

According to an embodiment, the processor 310 may, for example, control the components (e.g., the motor driver circuits 370 and 370-1, etc.) in the wearable devices 300 and 300-1 by executing software (e.g., a program or instructions) stored in the memory 350, and perform various data processing or computation. As at least a portion of the data processing or computation, the processor 310 may store data received from other components (e.g., the IMU 360, the angle sensors 320 and 320-1, etc.) in the memory 350, and process the instructions or data stored in the memory 350.

According to an embodiment, the processor 310 may determine torque values for generating torques of the motors 380 and 380-1, and control the driving module 30 (e.g., the motor driver circuits 370 and 370-1) based on the determined torque values. For example, the processor 310 may determine a state factor *y*(*t*) indicating a state of a motion of the user according to an equation *y*(*t*) = *sin*(*q_r*(*t*)) - *sin*(*q*_*l*(*t*)). *q_l*(*t*) may denote the angle value of the first joint (e.g., the left hip joint), and *q_r*(*t*) may denote the angle value of the second joint (e.g., the right hip joint). The processor 310 may determine a torque value *τ*(*t*) according to an equation *τ*(*t*) = *κy*(*t -*△ *t*). A gain *k* may be a parameter indicating the magnitude and direction of a torque generated by each of the motors 380 and 380-1. As the value of the gain *κ* increases, a greater torque may be output. If the gain *κ* is negative, a torque (or a resistance torque) acting as a resistance force may be output to the user, and if the gain *κ* is positive, a torque (or an assistance torque) acting as an assistance force may be output to the user. A delay *Δt* may be a parameter associated with a torque output timing. The value of the gain *κ* and the value of the delay *Δt* may be, for example, preset, and may be adjustable by the user, the wearable device 300, or the electronic device 120 paired with the wearable device 300. The processor 310 may determine a torque value for generating a torque from the motor 380-1 (or a torque value for the right leg of the user) *τ_{R}*(*t*) according to an equation *τ_{R}*(*t*) = *τ*(*t*), and determine a torque for generating a torque from the motor 380 (or a torque value for the left leg of the user) *τ_{L}*(*t*) according to *τ_{L}*(*t*) = *-τ*(*t*).

According to an embodiment, the motor driver circuits 370 and 370-1 may control the motors 380 and 380-1 based on the torque values received from the processor 310, and the motors 380 and 380-1 may generate torques by this control. "Based on" as used herein covers based at least on.

According to an embodiment, the communication module 390, comprising communication circuitry, may support the establishment of a direct (or wired) communication channel or a wireless communication channel between the wearable device 300, 300-1 and an external electronic device, and support the communication through the established communication channel. The communication module 390 may include one or more communication processors configured to support direct (or wired) communication or wireless communication. According to an embodiment, the communication module 390 may include a wireless communication module (e.g., a cellular communication module, a short-range wireless communication module, or a global navigation satellite system (GNSS) communication module) or a wired communication module (e.g., a local area network (LAN) communication module or a power line communication (PLC) module). A corresponding one of these communication modules may communicate with the external electronic device via a first network (e.g., a short-range communication network such as Bluetooth^{™}, wireless-fidelity (Wi-Fi) direct, or infrared data association (IrDA)) or a second network (e.g., a legacy cellular network, a 5G network, a next-generation communication network, the Internet, or a computer network). These various types of communication modules may be implemented as a single component (e.g., a single chip), or may be implemented as multiple components (e.g., multiple chips) separate from each other.

According to an embodiment, the wearable devices 300 and 300-1 may include a display module. The display module may include, for example, a display and/or a lighting unit (e.g., the lighting unit 60 of FIG. 2A). The processor 310 may control the display module so that the display module may provide visual feedback to the user.

According to an embodiment, the wearable devices 300 and 300-1 may include a sound output module. The sound output module may include, for example, a speaker. The processor 310 may control the sound output module so that the sound output module may provide auditory feedback to the user.

According to an embodiment, the wearable devices 300 and 300-1 may include a vibration output module. The vibration output module may include, for example, a vibration motor. The processor 310 may control the vibration output module so that the vibration output module may provide tactile feedback (or haptic feedback) to the user.

According to an embodiment, at least one of the processor 310, the battery 330, the PMIC 340, the memory 350, the IMU 360, the communication module 390, the display module, the sound output module, or the vibration output module, or a combination thereof may be positioned in the base body 10 of FIGS. 2A and 2B.

Each embodiment herein may be used in combination with any other embodiment(s) described herein.

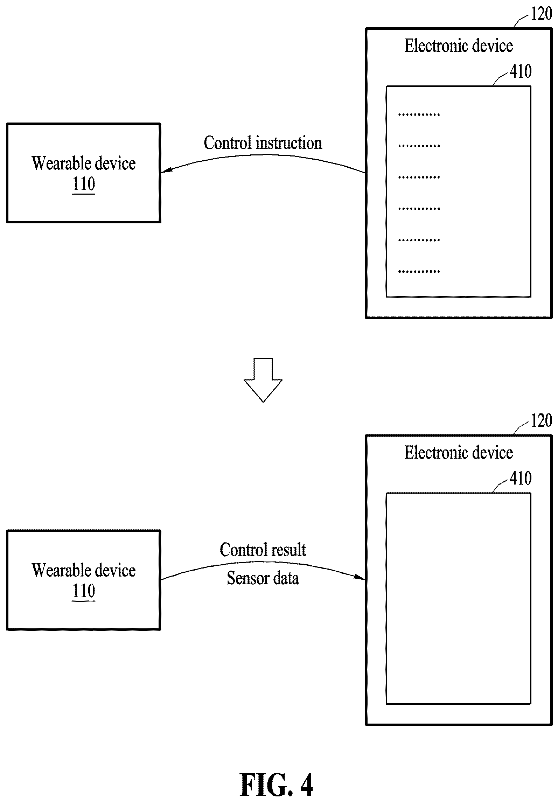

FIG. 4 is a diagram illustrating an interaction between a wearable device and an electronic device according to an embodiment.

Referring to FIG. 4, the wearable device 110 may communicate with the electronic device 120 (e.g., a smart phone or smart watch). For example, the electronic device 120 may be a user terminal of the user who uses the wearable device 110 or a controller device dedicated to the wearable device 110. According to an embodiment, the wearable device 110 and the electronic device 120 may be connected to each other through short-range wireless communication (e.g., Bluetooth^{™} or Wi-Fi communication).

According to an embodiment, the electronic device 120 may verify a state of the wearable device 110 or execute an application to control or operate the wearable device 110. A screen of a user interface (UI) may be displayed to control an operation of the wearable device 110 on a display 410 of the electronic device 120 through the execution of the application. The UI may be, for example, a graphical user interface (GUI).

According to an embodiment, the user may input an instruction to control an operation of the wearable device 110 through the GUI screen on the display 410 of the electronic device 120 (e.g., an instruction to instruct the wearable device 110 to operate in an assistance mode of generating an assistance force or an instruction to instruct the wearable device 110 to operate in a resistance mode of generating a resistance force) or change settings of the wearable device 110. The electronic device 120 may generate a control instruction (or control signal) corresponding to an operation control instruction or a setting change instruction input by the user and transmit the generated control instruction to the wearable device 110. The wearable device 110 may operate according to the received control instruction and transmit a control result according to the control instruction and/or sensor data measured by the sensor (e.g., the angle sensors 320 and 320-1 and/or the IMU 360) of the wearable device 110 to the electronic device 120. The electronic device 120 may provide the user with result information (e.g., walking ability information, exercise ability information, or exercise posture evaluation information) derived by analyzing the control result and/or the sensor data through the GUI screen.

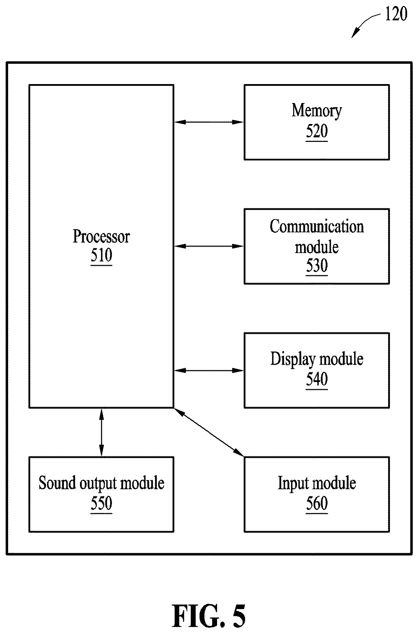

FIG. 5 is a diagram illustrating an example of a configuration of an electronic device according to an embodiment.

Referring to FIG. 5, the electronic device 120 may include a processor 510, a memory 520, a communication module 530, a display module 540, a sound output module 550, and an input module 560. In an embodiment, at least one (e.g., the sound output module 550) of these components may be omitted from the electronic device 120, or one or more other components (e.g., a sensor module, a haptic module, and a battery) may be added thereto.

The processor 510 may control at least one other component (e.g., a hardware or software component) of the electronic device 120, and may perform a variety of data processing or computation. According to an embodiment, as at least part of data processing or computation, the processor 510 may store instructions or data received from another component (e.g., the communication module 530, comprising communication circuitry) in the memory 520, process the instructions or data stored in the memory 520, and store result data obtained as a result of processing in the memory 520.

According to an embodiment, the processor 510 may include a main processor (e.g., a central processing unit (CPU) and/or an application processor (AP)) or an auxiliary processor (e.g., a graphics processing unit (GPU), a neural processing unit (NPU), an image signal processor (ISP), a sensor hub processor, and/or a communication processor (CP)) that is operable independently of, or in conjunction with the main processor.

The memory 520 may store a variety of data used by at least one component (e.g., the processor 510 or the communication module 530) of the electronic device 120. The data may include, for example, a program (e.g., an application), and input data or output data for instructions related thereto. The memory 520 may include at least one instruction executable by the processor 510. The memory 520 may include a volatile memory or a non-volatile memory.

The communication module 530 may support the establishment of a direct (e.g., wired) communication channel or a wireless communication channel between the electronic device 120 and another electronic device (e.g., the wearable device 110, the other wearable device 130, or the server 140), and support the communication through the established communication channel. The communication module 530 may include a communication circuit configured to perform a communication function. The communication module 530 may include one or more communication processors that are operable independently from the processor 510 (e.g., the application processor) and support direct (e.g., wired) communication or wireless communication. According to an embodiment, the communication module 530 may include a wireless communication module configured to perform wireless communication (e.g., a Bluetooth communication module, a cellular communication module, a Wi-Fi communication module, or a GNSS communication module) or a wired communication module (e.g., a LAN communication module or a power line communication (PLC) module). For example, the communication module 530 may transmit a control instruction to the wearable device 110 and receive, from the wearable device 110, at least one of sensor data including body motion information of the user who is wearing the wearable device 110, state data of the wearable device 110, or control result data corresponding to the control instruction.

The display module 540 may visually provide information to the outside (e.g., the user) of the electronic device 120. The display module 540 may include, for example, a liquid-crystal display (LCD) or organic light-emitting diode (OLED) display, a hologram device, or a projector device. The display module 540 may further include a control circuit configured to control the driving of a display. In an embodiment, the display module 540 may further include a touch sensor set to sense a touch or a pressure sensor set to sense the intensity of a force generated by the touch. The display module 540 may output a user interface screen for controlling the wearable device 110 or providing a variety of information (e.g., exercise evaluation information and setting information of the wearable device 110).

The sound output module 550 may output sound signals to the outside of the electronic device 120. The sound output module 550 may include a speaker configured to play back a guiding sound signal (e.g., an operation start sound or an operation error alarm), music content, or a guiding voice based on the state of the wearable device 110. For example, when it is determined that the wearable device 110 is not normally worn on the body of the user, the sound output module 550 may output a guiding voice to inform the user is wearing the wearable device 110 abnormally or to guide the user to wear the wearable device 110 normally.

The input module 560 may receive a command or data to be used by a component (e.g., the processor 510) of the electronic device 120 from the outside (e.g., the user) of the electronic device 120. The input module 560 may include an input component circuit and may receive a user input. The input module 560 may include, for example, a touch recognition circuit for recognizing a touch on a key (e.g., a button) and/or a screen.

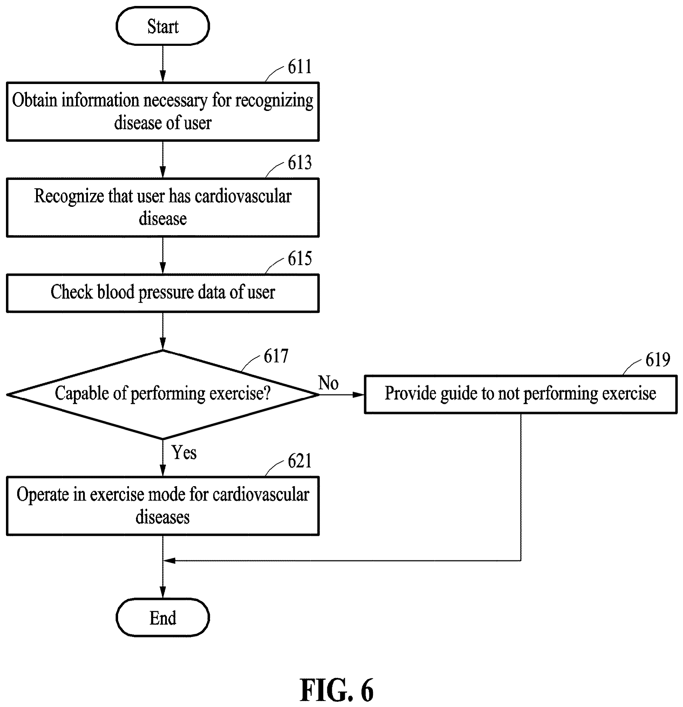

FIG. 6 is a diagram illustrating an example of an operation of a wearable device based on a disease of a user according to an embodiment.

Referring to FIG. 6, in operation 611, the wearable device 110 (e.g., the processor 310) may obtain information necessary (or to be used) or helpful for recognizing a disease (e.g., a chronic disease) of a user. The information necessary or helpful for recognizing the disease (e.g., a chronic disease) of the user may include, for example, at least one or all of a disease code (or a disease classification code) of the user, user input information, biometric information of the user, medical record information of the user, or exercise history information of the user.

For example, the wearable device 110 may request the disease code of the user from the server 140. When a request for the disease code of the user is received from the wearable device 110, the server 140 may transmit the disease code of the user to the wearable device 110. The wearable device 110 may receive the disease code of the user from the server 140, and recognize which disease (or chronic disease) (e.g., high blood pressure, diabetes, arthritis, back pain, etc.) the user has through the received disease code.

The user input information may include, for example, the name of the disease input by the user. The electronic device 120 (or the smart watch 132) may request the user to input the disease the use has, and receive the name of the disease (e.g., high blood pressure, diabetes, back pain, leg pain, etc.) from the user. The wearable device 110 may receive the user input information (e.g., the name of the disease input by the user) from the electronic device 120 (or the smart watch 132), and recognize which disease (e.g., high blood pressure, diabetes, hyperlipidemia, back pain, leg pain, etc.) the user has through the received user input information.

The biometric information may include, for example, at least one of blood pressure data, blood glucose data, or heart rate data of the user. The electronic device 120 (or the smart watch 132) may measure at least one of the blood pressure, blood glucose, or heart rate of the user, and store the measurement result (e.g., at least one of blood pressure data, blood glucose data, or heart rate data). The wearable device 110 may receive the biometric information of the user from the electronic device 120 (or the smart watch 132), and recognize which disease (e.g., diabetes, hypertension, etc.) the user has through the received biometric information.

For example, the wearable device 110 may receive the medical record information of the user from the server 140, and recognize which disease (e.g., high blood pressure, diabetes, hyperlipidemia, arthritis, back pain, leg pain, etc.) the user has through the received medical record information.

For example, the wearable device 110 may request a predetermined (or certain) item of the exercise history information (e.g., a walking symmetry) of the user from the server 140, and receive the predetermined item of the exercise history information (e.g., the walking symmetry) from the server 140. The wearable device 110 may recognize the disease (e.g., knee pain, leg pain, etc.) of the user through a change in the item (e.g., the walking symmetry) received from the server 140.

In operation 613, the wearable device 110 (e.g., the processor 310) may recognize that the user has a cardiovascular disease (e.g., hypertension, diabetes, hyperlipidemia, etc.) through the obtained information (e.g., the disease code of the user, the user input information, the biometric information of the user, the medical record information of the user, or the exercise history information of the user).

In operation 615, the wearable device 110 (e.g., the processor 310) may check the blood pressure data of the user. For example, the processor 310 may check whether the wearable device 110 (or the electronic device 120) is connected to a device (e.g., the smart watch 132 or a separate blood pressure measuring device) capable of measuring the blood pressure of the user. When the wearable device 110 (or the electronic device 120) is connected to the smart watch 132 capable of measuring the blood pressure of the user, the processor 310 may request the smart watch 132 to measure the blood pressure and receive the blood pressure data from the smart watch 132. When the wearable device 110 (or the electronic device 120) is not connected to a device capable of measuring the blood pressure of the user, the processor 310 may obtain the most recent blood pressure data of the user from the medical record information.

In operation 617, the wearable device 110 (e.g., the processor 310) may determine whether the user is capable of performing an exercise based on the blood pressure data of the user (e.g., the blood pressure data measured by the smart watch 132 or the blood pressure data obtained from the medical record information). The processor 310 may determine that the user is incapable of performing an exercise when the blood pressure data of the user exceeds a predetermined (or certain) level. The processor 310 may determine that the user is capable of performing an exercise when the blood pressure data of the user is less than the predetermined level. For example, the processor 310 may determine that the user is incapable of performing an exercise when the systolic blood pressure of the user is greater than or equal to a first blood pressure value (e.g., 200 millimeters of mercury (mmHg)) and/or the diastolic blood pressure of the user is greater than or equal to a second blood pressure value (e.g., 110 mmHg). The processor 310 may determine that the user is capable of performing an exercise when the systolic blood pressure of the user is less than the first blood pressure value (e.g., 200 mmHg) and the diastolic blood pressure of the user is less than the second blood pressure value (e.g., 110 mmHg). Examples of the first and second blood pressure values are not limited thereto.

When it is determined that the user is incapable of performing an exercise (if NO in operation 617), the wearable device 110 (e.g., the processor 310) may provide the user with a guide to not performing an exercise, in operation 619. For example, the processor 310 may provide the user with a guide to not performing an exercise through a voice output and/or a visual output. The voice output may include, for example, a voice output through a speaker of the wearable device 110 and/or a voice output through the wireless earphones 131, but is not limited thereto. The visual output may include, for example, at least one of a screen display of the electronic device 120, a screen display of the smart watch 132, or a screen display of the smart glasses 133.

When it is determined that the user is capable of performing an exercise (if YES in operation 617), the wearable device 110 (e.g., the processor 310) may operate in an exercise mode for cardiovascular diseases, in operation 621. For example, the processor 310 may control the wearable device 110 to operate in an exercise mode for cardiovascular diseases (e.g., an interval walking exercise mode). An interval walking exercise may be an exercise in which a user repeats walking and resting. In the interval walking exercise mode, the processor 310 may control the driving module 30 to provide a torque (e.g., an assistance torque or a resistance torque) to the user performing an interval walking exercise, and provide the user with a guide to an interval walking exercise (e.g., an exercise posture, an exercise time left, etc.).

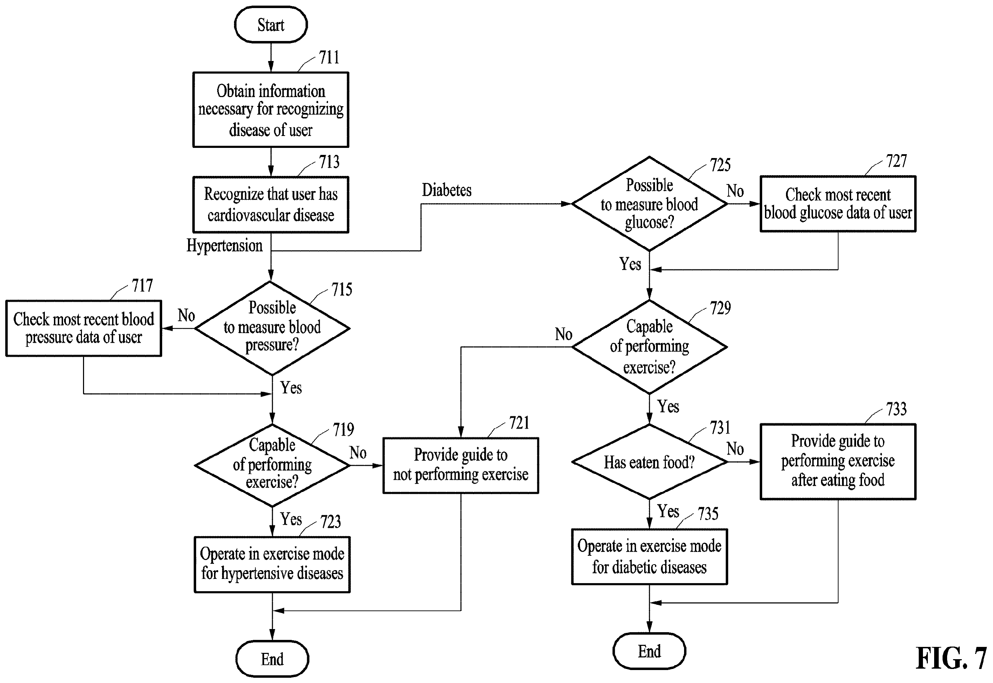

FIG. 7 is a diagram illustrating another example of an operation of a wearable device based on a disease of a user according to an embodiment.

Referring to FIG. 7, in operation 711, the wearable device 110 (e.g., the processor 310) may obtain information necessary or helpful for recognizing a disease of a user (e.g., at least one or all of a disease code of the user, user input information, biometric information of the user, medical record information of the user, or exercise history information of the user). The description of operation 611 may apply to the description of operation 711.

In operation 713, the wearable device 110 (e.g., the processor 310) may recognize that the user has a cardiovascular disease (e.g., hypertension, diabetes, etc.) through the obtained information (e.g., the disease code of the user, the user input information, the biometric information of the user, the medical record information of the user, or the exercise history information of the user).

When it is recognized that the user has hypertension, the wearable device 110 (e.g., the processor 310) may check whether it is possible to measure the blood pressure of the user, in operation 715. For example, the processor 310 may check whether the wearable device 110 (or the electronic device 120) is connectable (or already connected) to a device (e.g., the smart watch 132 or a separate blood pressure measuring device) capable of measuring the blood pressure of the user. When the wearable device 110 (or the electronic device 120) is connected to the smart watch 132 capable of measuring the blood pressure of the user, the processor 310 may request the smart watch 132 to measure the blood pressure and receive the blood pressure data from the smart watch 132. When the wearable device 110 (or the electronic device 120) is not connected to a device capable of measuring the blood pressure of the user (e.g., No in step/query 715), the processor 310 may check (or obtain) the most recent blood pressure data of the user from the medical record information in operation 717.

In operation 719, the wearable device 110 (e.g., the processor 310) may determine whether the user is capable of performing an exercise based on the blood pressure data of the user (e.g., the blood pressure data measured by the smart watch 132 or the blood pressure data checked from the medical record information). The processor 310 may determine that the user is incapable of performing an exercise when the blood pressure data of the user exceeds a predetermined (or certatin) level. The processor 310 may determine that the user is capable of performing an exercise when the blood pressure data of the user is less than the predetermined level. For example, the processor 310 may determine that the user is incapable of performing an exercise when the systolic blood pressure of the user is greater than or equal to a first blood pressure value (e.g., 200 mmHg) and/or the diastolic blood pressure of the user is greater than or equal to a second blood pressure value (e.g., 110 mmHg). The processor 310 may determine that the user is capable of performing an exercise when the systolic blood pressure of the user is less than the first blood pressure value and the diastolic blood pressure of the user is less than the second blood pressure value.

When it is determined that the user is incapable of performing an exercise (if NO in operation 719), the wearable device 110 (e.g., the processor 310) may provide the user with a guide to not performing an exercise, in operation 721. For example, the processor 310 may provide the user with a guide to not performing an exercise because the user has high blood pressure through a voice output and/or a visual output. The voice output may include a voice output of a guide to not performing an exercise through the speaker of the wearable device 110 and/or a voice output of a guide to not performing an exercise through the wireless earphones 131, but is not limited thereto. The visual output may include, for example, at least one of displaying a guide to not performing an exercise on a screen of the electronic device 120, displaying a guide to not performing an exercise on a screen of the smart watch 132, or displaying a guide to not performing an exercise on a screen of the smart glasses 133, but is not limited thereto.

When it is determined that the user is capable of performing an exercise (if YES in operation 719), the wearable device 110 (e.g., the processor 310) may operate in an exercise mode for hypertensive diseases, in operation 723. For example, the processor 310 may control the wearable device 110 to operate in an exercise mode for hypertensive diseases (e.g., a first interval walking exercise mode). The first interval walking exercise mode may be, for example, an interval walking exercise mode set with exercise intensity (e.g., level 1)/duration of exercise (e.g., 15 minutes)/number of sets (e.g., 2 sets). In the first interval walking exercise mode, the processor 310 may control the driving module 30 to provide a torque (e.g., an assistance torque or a resistance torque) to the user performing an interval walking exercise, and provide the user with a guide to an interval walking exercise (e.g., an exercise posture, an exercise time left, etc.).

When it is recognized in operation 713 that the user has diabetes, the wearable device 110 (e.g., the processor 310) may check whether it is possible to measure the blood glucose of the user, in operation 725. For example, the processor 310 may check whether the wearable device 110 (or the electronic device 120) is connectable (or already connected) to a device (e.g., the smart watch 132 or a separate blood glucose measuring device) capable of measuring the blood glucose of the user. When the wearable device 110 (or the electronic device 120) is connected to the smart watch 132 (or the blood glucose measuring device) capable of measuring the blood glucose of the user, the processor 310 may request the smart watch 132 (or the blood glucose measuring device) to measure the blood glucose and receive the blood glucose data from the smart watch 132 (or the blood glucose measuring device). When the wearable device 110 (or the electronic device 120) is not connected to a device capable of measuring the blood glucose of the user, the processor 310 may check (or obtain) the most recent blood glucose data of the user from the medical record information, in operation 727.

In operation 729, the wearable device 110 (e.g., the processor 310) may determine whether the user is capable of performing an exercise based on the blood glucose data of the user (e.g., the blood glucose data measured by the smart watch 132 (or the blood glucose measuring device) or the blood glucose data checked from the medical record information). The processor 310 may determine that the user is incapable of performing an exercise when the blood glucose data of the user exceeds a predetermined (or certain) level. The processor 310 may determine that the user is capable of performing an exercise when the blood glucose data of the user is less than the predetermined level. For example, the processor 310 may determine that the user is capable of performing an exercise when the blood glucose level of the user is within a predetermined (or certain) range (e.g., 100 to 250 milligrams per deciliter (mg/dL)). For example, the processor 310 may determine that the user is incapable of performing an exercise when the blood glucose level of the user is out of the predetermined range (e.g., 100 to 250 mg/dL).

When it is determined that the user is incapable of performing an exercise (if NO in operation 729), the wearable device 110 (e.g., the processor 310) may provide the user with a guide to not performing an exercise, in operation 721. For example, the processor 310 may provide the user with a guide to not performing an exercise because the user has high or low blood glucose through a voice output and/or a visual output. The voice output may include a voice output of a guide to not performing an exercise through the speaker of the wearable device 110 and/or a voice output of a guide to not performing an exercise through the wireless earphones 131, but is not limited thereto. The visual output may include, for example, at least one of displaying a guide to not performing an exercise on a screen of the electronic device 120, displaying a guide to not performing an exercise on a screen of the smart watch 132, or displaying a guide to not performing an exercise on a screen of the smart glasses 133, but is not limited thereto.

When it is determined that the user is capable of performing an exercise (if YES in operation 729), the wearable device 110 (e.g., the processor 310) may check whether the user has eaten food, in operation 731. For example, the processor 310 may provide the user with a voice output and/or visual output of an inquiry as to whether the user has eaten food through the wearable device 110 (or the electronic device 120, the smart watch 132, or the smart glasses 133). The voice output may include, for example, a voice output of an inquiry as to whether the user has eaten food through the speaker of the wearable device 110 and/or through the wireless earphones 131, but is not limited thereto. The visual output may include, for example, displaying a message indicating an inquiry as to whether the user has eaten food on at least one of the screen of the electronic device 120, the screen of the smart watch 132, or the screen of the smart glasses 133, but is not limited thereto. The processor 310 may receive a voice response as to whether the user has eaten food from the user through the microphone of the wearable device 110 and/or the wireless earphones 131. Alternatively, the user may input a response as to whether the user has eaten food to the electronic device 120 (or the smart watch 132 or the smart glasses 133), and the processor 310 may receive the response as to whether the user has eaten food from the electronic device 120 (or the smart watch 132 or the smart glasses 133).

When the user has not eaten food (if NO in operation 731), the wearable device 110 (e.g., the processor 310) may provide the user with a guide to performing an exercise after eating food, in operation 733. Such a guide may be provided to the user through a voice output and/or a visual output.

When the user has eaten food (if YES in operation 731), the wearable device 110 (e.g., the processor 310) may operate in an exercise mode for diabetic diseases, in operation 735. The exercise mode for diabetic diseases may differ from the exercise mode for hypertensive diseases in terms of at least one of the exercise intensity, duration of exercise, or number of sets. For example, the processor 310 may control the wearable device 110 to operate in an exercise mode for diabetic diseases (e.g., a second interval walking exercise mode). The second interval walking exercise mode may be, for example, an interval walking exercise mode set with exercise intensity (e.g., Level 2)/duration of exercise (e.g., 20 to 30 minutes)/number of sets (e.g., 1 set). In the second interval walking exercise mode, the processor 310 may control the driving module 30 to provide a torque (e.g., an assistance torque or a resistance torque) to the user performing an interval walking exercise, and provide the user with a guide to an interval walking exercise (e.g., an exercise posture, an exercise time left, etc.).

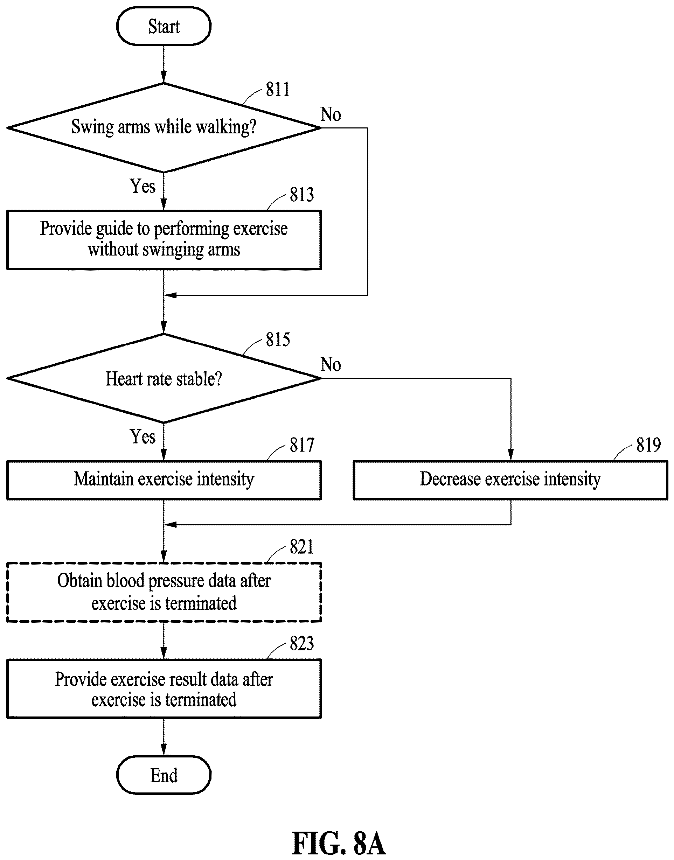

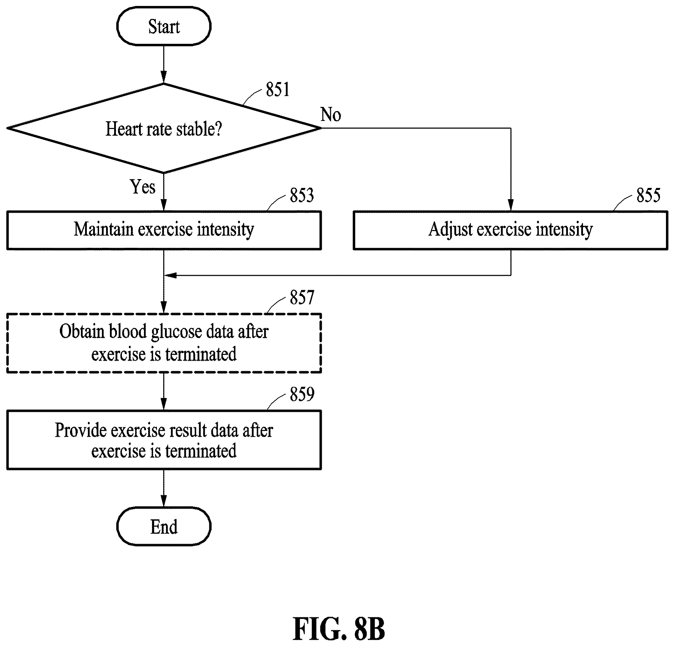

FIGS. 8A and 8B are diagrams illustrating other examples of an operation of a wearable device based on a disease of a user according to an embodiment.

The wearable device 110 may perform operations of FIG. 8A in the interval walking exercise mode described with reference to FIG. 6 or in the first interval walking exercise mode described with reference to FIG. 7.

Referring to FIG. 8, in operation 811, the wearable device 110 (e.g., the processor 310) may determine whether the user swings arms while walking (or performing an interval walking exercise). For example, the processor 310 may determine whether the user is walking (or performing an interval walking exercise) while swinging the arms, through the smart watch 132.

When it is determined that the user swings the arms while walking (if YES in operation 811), the wearable device 110 (e.g., the processor 310) may provide the user with a guide to performing an exercise without swinging the arms, in operation 813. Such a guide may be provided to the user through a voice output and/or a visual output.

In operation 815, the wearable device 110 (e.g., the processor 310) may determine whether the heart rate is stable. For example, when it is determined that the user is not walking while swinging the arms, the processor 310 may request the smart watch 132 to measure the heart rate, and receive heart rate data of the user performing an exercise (e.g., an interval walking exercise) from the smart watch 132. When the received heart rate data hardly changes, the processor 310 may determine that the heart rate of the user is stable.

When it is determined that the heart rate of the user performing the exercise is stable (if YES in operation 815), the wearable device 110 (e.g., the processor 310) may maintain the exercise intensity of the interval walking exercise mode (or the first interval walking exercise mode), in operation 817. Embodiments are not limited thereto, and when it is determined that the heart rate of the user performing the exercise is stable, the wearable device 110 (e.g., the processor 310) may increase the exercise intensity of the interval walking exercise mode (or the first interval walking exercise mode).

When it is determined that the heart rate of the user performing the exercise is unstable (if NO in operation 815), the wearable device 110 (e.g., the processor 310) may decrease the exercise intensity of the interval walking exercise mode (or the first interval walking exercise mode), in operation 819.

The interval walking exercise of the user may be terminated.

In operation 823, the wearable device 110 (e.g., the processor 310) may provide the user with exercise result data after the exercise is terminated. The exercise result data may include, for example, the duration of exercise, exercise capacity indicators (e.g., walking symmetry, walking speed, etc.), and the like, but is not limited thereto. The processor 310 may transmit the exercise result data to the electronic device 120 (or the smart watch 132), and the electronic device 120 (or the smart watch 132) may display the exercise result data on the display. According to embodiments, the processor 310 may transmit the exercise result data to the server 140. As the exercise result data is accumulated in the server 140, the exercise history information of the user may be generated.

According to the implementation, the wearable device 110 (e.g., the processor 310) may obtain blood pressure data after the exercise is terminated, in operation 821, prior to operation 823. For example, the processor 310 may determine whether the wearable device 110 is connectable (or already connected) to a device capable of measuring the blood pressure of the user. When the wearable device 110 is connected to the smart watch 132 (or a separate blood pressure measuring device), the processor 310 may request the smart watch 132 to measure the blood pressure and receive the blood pressure data of the user terminating the exercise from the smart watch 132 (or the blood pressure measuring device). The processor 310 may include the blood pressure data obtained after the exercise is terminated in the exercise result data and provide the exercise result data to the user.

The wearable device 110 may perform operations of FIG. 8B in the interval walking exercise mode described with reference to FIG. 6 or in the second interval walking exercise mode described with reference to FIG. 7.

Referring to FIG. 8B, in operation 851, the wearable device 110 (e.g., the processor 310) may determine whether the heart rate of the user performing an exercise (e.g., an interval walking exercise) is stable. For example, the processor 310 may request the smart watch 132 to measure the heart rate and receive heart rate data from the smart watch 132. When the received heart rate data hardly changes, the processor 310 may determine that the heart rate of the user performing the exercise is stable.

When it is determined that the heart rate of the user performing the exercise is stable (if YES in operation 851), the wearable device 110 (e.g., the processor 310) may maintain the exercise intensity of the interval walking exercise mode (or the second interval walking exercise mode), in operation 853. Embodiments are not limited thereto, and when it is determined that the heart rate of the user performing the exercise is stable, the wearable device 110 (e.g., the processor 310) may increase the exercise intensity of the interval walking exercise mode (or the second interval walking exercise mode).

When it is determined that the heart rate of the user performing the exercise is unstable (if NO in operation 851), the wearable device 110 (e.g., the processor 310) may decrease the exercise intensity of the interval walking exercise mode (or the second interval walking exercise mode), in operation 855.

The interval walking exercise of the user may be terminated.

In operation 859, the wearable device 110 (e.g., the processor 310) may provide the user with exercise result data after the exercise is terminated. The exercise result data may include, for example, the duration of exercise, exercise capacity indicators (e.g., walking symmetry, walking speed, etc.), and the like, but is not limited thereto. The processor 310 may transmit the exercise result data to the electronic device 120 (or the smart watch 132), and the electronic device 120 (or the smart watch 132) may display the exercise result data on the display. According to embodiments, the processor 310 may transmit the exercise result data to the server 140.

According to the implementation, the wearable device 110 (e.g., the processor 310) may obtain blood glucose data after the exercise is terminated, in operation 857 prior to operation 859. For example, the processor 310 may determine whether the wearable device 110 is connectable (or already connected) to a device capable of measuring the blood glucose of the user after the exercise is terminated. When the wearable device 110 is connected, directly or indirectly, to the smart watch 132 (or a separate blood glucose measuring device), the processor 310 may request the smart watch 132 (or the separate blood glucose measuring device) to measure the blood glucose and receive the blood glucose data of the user terminating the exercise from the smart watch 132 (or the separate blood glucose measuring device). The processor 310 may include the blood glucose data obtained after the exercise is terminated in the exercise result data and provide the exercise result data to the user.

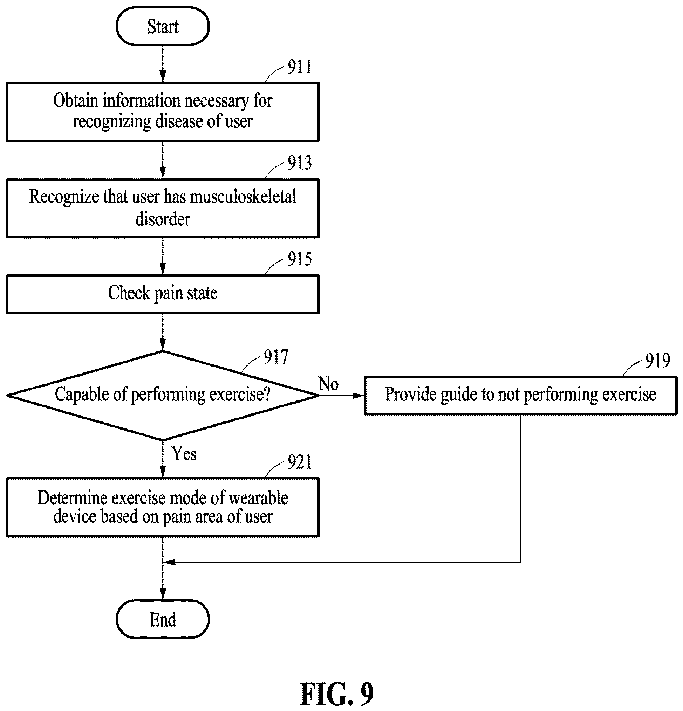

FIG. 9 is a diagram illustrating another example of an operation of a wearable device based on a disease of a user according to an embodiment.

Referring to FIG. 9, in operation 911, the wearable device 110 (e.g., the processor 310) may obtain information necessary or helpful for recognizing a disease of a user (e.g., at least one or all of a disease code of the user, user input information, biometric information of the user, medical record information of the user, or exercise history information of the user). The description of operation 611 may apply to the description of operation 911.

In operation 913, the wearable device 110 (e.g., the processor 310) may recognize that the user has a musculoskeletal disorder (e.g., arthritis, back pain, etc.) through the obtained information (e.g., the disease code of the user, the user input information, the biometric information of the user, the medical record information of the user, or the exercise history information of the user).

In operation 915, the wearable device 110 (e.g., the processor 310) may check a pain state (e.g., a pain area and/or a pain intensity) of the user. For example, the processor 310 may inquire the user about the pain area and/or the pain intensity in a voice through the speaker of the wearable device 110 or the wireless earphones 131. At this time, the processor 310 may inquire the user about a score of the pain intensity that the user feels on the numeric rating scale (e.g., 1 to 10 points). The processor 310 may receive a voice signal from the user and check the pain area and/or a score corresponding to the pain intensity that the user feels by processing the voice signal. As another example, the processor 310 may inquire the user about the pain area and/or the pain intensity through the display of the electronic device 120 (or the smart watch 132 or the smart glasses 133). The electronic device 120 (or the smart watch 132 or the smart glasses 133) may receive the pain area and/or the score corresponding to the pain intensity that the user feels from the user. The processor 310 may receive the pain area of the user and/or the score corresponding to the pain intensity that the user feels from the electronic device 120 (or the smart watch 132 or the smart glasses 133).

The processor 310 may determine that the user feels a mild pain intensity when the score corresponding to pain intensity falls within, for example, a first score range (e.g., 1 to 4 points), and determine that the user feels a moderate pain intensity when the score corresponding to pain intensity falls within, for example, a second score range (e.g., 5 to 6 points). The processor 310 may determine that the user feels a severe pain intensity when the score corresponding to the pain intensity falls within, for example, a third score range (e.g., 7 to 10 points).

In operation 917, the wearable device 110 (e.g., the processor 310) may determine whether the user is capable of performing an exercise based on the pain state (e.g., the pain area and/or the pain intensity) of the user. For example, the processor 310 may determine that the user is capable of performing an exercise when it is determined that the user feels a mild pain intensity or moderate pain intensity. The processor 310 may determine that the user is incapable of performing an exercise when it is determined that the user feels a severe pain intensity.

When it is determined that the user is incapable of performing an exercise (if NO in operation 917), the wearable device 110 (e.g., the processor 310) may provide the user with a guide to not performing an exercise, in operation 919. For example, the processor 310 may provide the user with a guide to not performing an exercise through a voice output and/or a visual output. The voice output may include, for example, a voice output of a guide to not performing an exercise through the speaker of the wearable device 110 and/or through the wireless earphones 131, but is not limited thereto. The visual output may include, for example, displaying a guide to not performing an exercise on at least one of the screen of the electronic device 120, the screen of the smart watch 132, or the screen of the smart glasses 133, but is not limited thereto.

According to embodiments, the processor 310 may recommend the user to go see a doctor when it is determined that the user is incapable of performing an exercise.

When it is determined that the user is capable of performing an exercise (if YES in operation 917), the wearable device 110 (e.g., the processor 310) may determine an exercise mode (e.g., a damping exercise mode or a muscle strengthening exercise mode) of the wearable device 110 based on the pain area of the user, in operation 921. For example, the processor 310 may determine the exercise mode of the wearable device 110 to be a damping exercise mode when the pain area of the user is a first body part (e.g., the knees), or determine the exercise mode of the wearable device 110 to be a muscle strengthening exercise mode when the pain area of the user is a second body part (e.g., the back).

The damping exercise mode may be, for example, an exercise mode for mitigating an impact on the first body part (e.g., the knees) of the user performing an exercise, and may be an exercise mode in which a torque control operation (e.g., an operation of multiplying a torque value by a predetermined (or certain) value or an operation of changing the value of a first parameter (e.g., a gain) among parameters for generating a torque of the wearable device 110) is performable. When a foot of the user touches the ground while the user is performing a walking exercise, it may cause an impact on the knee of the user. In addition, the user's bending or extending the knees in place may cause an impact on the knees of the user. The wearable device 110 may operate in the damping exercise mode to mitigate the impact on the first body part (e.g., the knees). The greater the magnitude of the resistance torque, the greater the impact mitigation intensity provided by the wearable device 110 to the user.

The muscle strengthening exercise mode may be, for example, an exercise mode for strengthening the muscles of the second body part (e.g., the back) of the user performing an exercise, and may be an exercise mode in which a torque control operation (e.g., an operation of multiplying a torque value by a predetermined (or certain) value or an operation of changing the value of the first parameter (e.g., the gain) among the parameters (e.g., the gain, delay, etc.) for generating a torque of the wearable device 110) is performable.

According to an embodiment, the processor 310 may recommend an exercise based on the pain intensity of the user or adjust the impact mitigation intensity of the wearable device 110 based on the pain intensity in the damping exercise mode. For example, when it is determined that the user feels mild knee pain, the processor 310 may operate in the damping exercise mode and recommend a forward walking exercise to the user. When it is determined that the user feels moderate knee pain, the processor 310 may operate in the damping exercise mode and recommend an in-place exercise (e.g., squats, lunges, a walking-in-place exercise, etc.) and/or a forward walking exercise to the user. The greater the pain intensity, the greater the processor 310 may adjust the impact mitigation intensity (e.g., the predetermined value by which the torque value is multiplied or the absolute value of the gain).

According to an embodiment, the processor 310 may recommend an exercise based on the pain intensity of the user or adjust the exercise intensity based on the pain intensity in the muscle strengthening exercise mode. For example, when it is determined that the user feels mild back pain, the processor 310 may operate in the muscle strengthening exercise mode and recommend an in-place exercise for strengthening the lower back muscles (e.g., a bird dog exercise and/or a skating exercise described later) and/or a walking exercise (e.g., a forward walking exercise and/or a slope walking exercise) to the user. When it is determined that the user feels moderate back pain, the processor 310 may operate in the muscle strengthening exercise mode and recommend the user to perform an in-place exercise for strengthening the lower back muscles (e.g., a bird dog exercise and/or a skating exercise) with a reduced exercise intensity and/or number of sets.

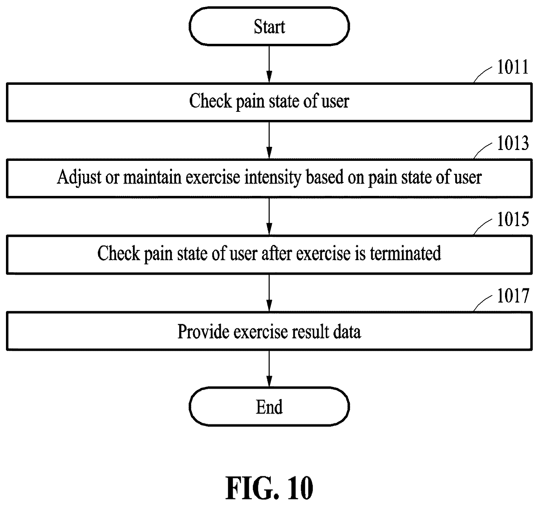

FIG. 10 is a diagram illustrating another example of an operation of a wearable device based on a disease of a user according to an embodiment.

The wearable device 110 may perform operations of FIG. 10 in the damping exercise mode and/or muscle strengthening exercise mode described with reference to FIG. 9.

Referring to FIG. 10, in operation 1011, the wearable device 110 (e.g., the processor 310) may check a pain state (e.g., a pain area and/or a pain intensity) of a user performing an exercise. For example, the processor 310 may inquire the user performing the exercise about the pain area and/or the pain intensity in a voice through the speaker of the wearable device 110 or the wireless earphones 131. At this time, the processor 310 may inquire the user about a score of the pain intensity that the user feels on the numeric rating scale (e.g., 1 to 10 points). The processor 310 may receive a voice signal about the score corresponding to the pain intensity that the user feels from the user and check the pain area and/or the score corresponding to the pain intensity that the user feels by processing the voice signal. As another example, the processor 310 may inquire the user about the pain area and/or the pain intensity through the display of the electronic device 120 (or the smart watch 132 or the smart glasses 133). The electronic device 120 (or the smart watch 132 or the smart glasses 133) may receive the pain area and/or the score corresponding to the pain intensity that the user feels from the user. The processor 310 may receive the pain area of the user and/or the score corresponding to the pain intensity that the user feels from the electronic device 120 (or the smart watch 132 or the smart glasses 133).

In operation 1013, the wearable device 110 (e.g., the processor 310) may adjust or maintain the exercise intensity (and/or the impact mitigation intensity) based on the pain state of the user performing the exercise.

For example, when there is no difference between the pain intensity of the user before an exercise (e.g., the pain intensity checked in operation 915 of FIG. 9) and the pain intensity of the user performing the exercise (e.g., the pain intensity checked in operation 1011), the processor 310 may maintain the exercise intensity and/or the impact mitigation intensity. The processor 310 may decrease the exercise intensity and/or increase the impact mitigation intensity if the pain intensity of the user performing the exercise is greater than the pain intensity of the user before an exercise. The processor 310 may increase the impact mitigation intensity by increasing the value of the first parameter in a negative direction (or increasing the absolute value of the first parameter). The processor 310 may increase the exercise intensity and/or decrease the impact mitigation intensity if the pain intensity of the user performing the exercise is less than the pain intensity of the user before an exercise. The processor 310 may decrease the impact mitigation intensity by decreasing the value of the first parameter in a negative direction (or decreasing the absolute value of the first parameter).

According to embodiments, when the wearable device 110 is connected, directly or indirectly, to the smart watch 132, the wearable device 110 (e.g., the processor 310) may request the smart watch 132 to measure the heart rate and receive the heart rate data of the user during exercise from the smart watch 132. The processor 310 may determine whether the heart rate of the user performing the exercise is stable through the heart rate data of the user performing the exercise. The processor 310 may increase the exercise intensity and/or decrease the impact mitigation intensity when it is determined that the heart rate of the user performing the exercise is stable. The processor 310 may decrease the exercise intensity and/or increase the impact mitigation intensity when it is determined that the heart rate of the user performing the exercise is unstable.

In operation 1015, the wearable device 110 (e.g., the processor 310) may check a pain state (e.g., a pain area and/or a pain intensity) of the user after the exercise is terminated. The description of operation 1011 may apply to the description of operation 1015.

In operation 1017, the wearable device 110 (e.g., the processor 310) may provide exercise result data. The exercise result data may include, for example, the duration of exercise, exercise capacity indicators (e.g., walking symmetry, walking speed, etc.), a pain state change, and the like, but is not limited thereto. The processor 310 may transmit the exercise result data to the electronic device 120 (or the smart watch 132), and the electronic device 120 (or the smart watch 132) may display the exercise result data on the display. According to embodiments, the processor 310 may transmit the exercise result data to the server 140.

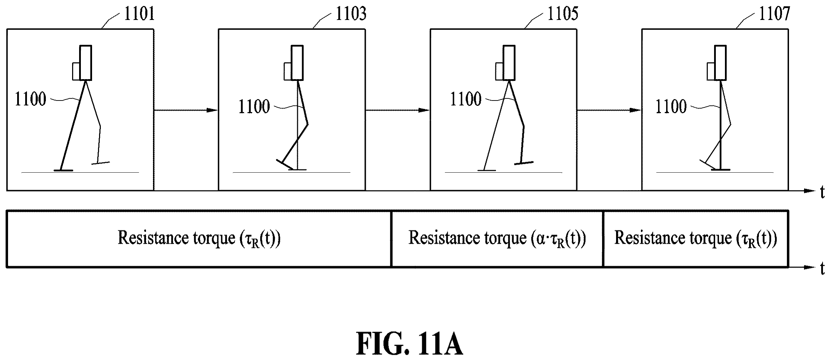

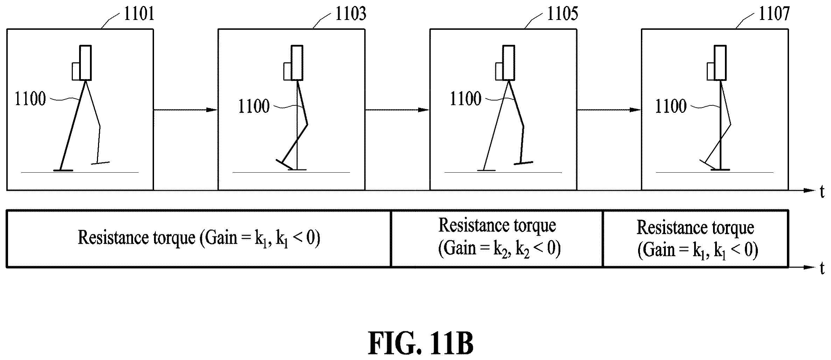

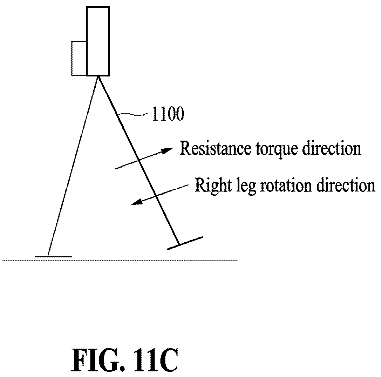

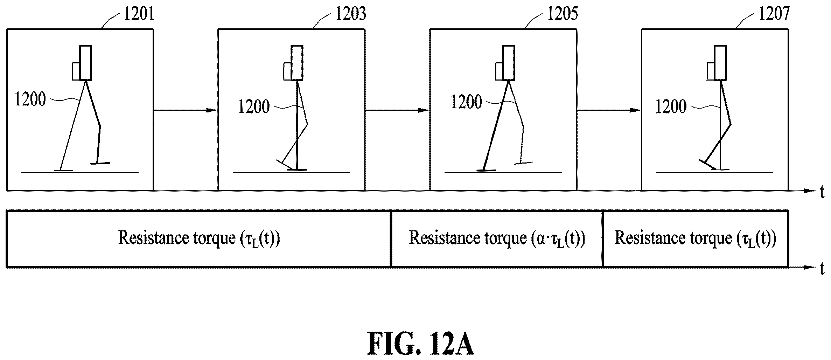

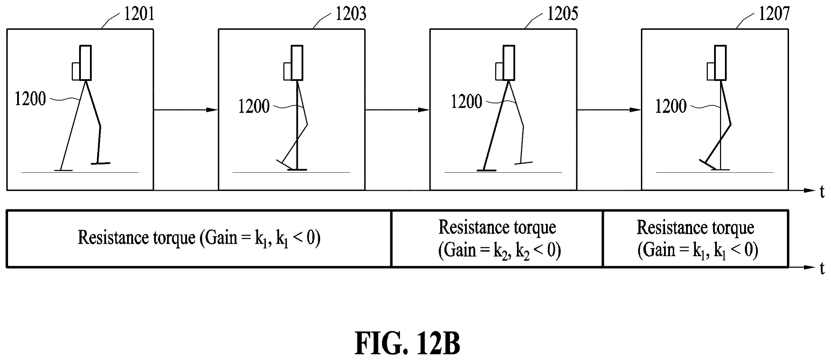

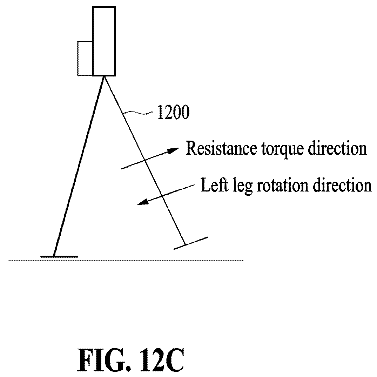

FIGS. 11A, 11B, 11C, 12A, 12B, and 12C are diagrams illustrating examples of an operation of a wearable device in a damping exercise mode according to an embodiment.

In the examples shown in FIGS. 11A, 11B, 11C, 12A, 12B, and 12C, a user wearing the wearable device 110 may perform a forward walking exercise.

FIG. 11A illustrates an example of postures 1101 to 1107 of the user while the user is performing a forward walking exercise, and illustrates an embodiment of an operation of performing torque control while the wearable device 110 provides a resistance torque to the user (e.g., an operation of multiplying a torque value for one leg by a predetermined (or certain) value) in a damping exercise mode.

According to an embodiment, the wearable device 110 may provide a resistance torque (or resistance force) to a motion of the user in a damping exercise mode. For example, the wearable device 110 may determine torque values (e.g., *τ_{R}*(*t*) and *τ_{L}*(*t*)) for both legs based on the difference value between the angle values of both hip joint angles of the user and the values of the parameters. The wearable device 110 may provide a resistance torque corresponding to the torque value (e.g., *τ_{R}*(*t*)) for the right leg 1100 to the right leg 1100 of the user, and provide a resistance torque corresponding to the torque value (e.g., *τ_{L}*(*t*)) for the left leg to the left leg of the user. At this time, the wearable device 110 may provide a greater magnitude of resistance torque to the leg (e.g., the right leg 1100) by multiplying the torque value (e.g., *τ_{R}*(*t*) of FIG. 11A) for the leg (e.g., the right leg 1100) by a predetermined (or certain) value (e.g., α of FIG. 11A) before the foot in the air (e.g., the right foot) touches the ground. Hereinafter, an embodiment of torque control in a damping exercise mode is described with reference to the example shown in FIG. 11A.

In the example shown in FIG. 11A, the posture 1101 may be a posture in which the right foot touches the ground in the back. In the posture 1101, the rotation direction of the right hip joint may be the forward direction (e.g., the counterclockwise direction in FIG. 11A). The posture 1103 may be a posture in which the right leg 1100 and the left leg cross and then, the right hip joint is extended forward. In the posture 1103, the rotation direction of the right hip joint may be the forward direction. A posture in which the right hip joint is maximally or largely extended forward may be present between the posture 1103 and the posture 1105, and after the right hip joint is maximally or largely extended forward, the rotation direction of the right hip joint may change from the forward direction to the backward direction (e.g., the clockwise direction in FIG. 11A). The posture 1105 may be a posture before the right foot touches the ground, and the rotation direction of the right hip joint may be the backward direction. A posture in which the right foot touches the ground may be present between the posture 1105 and the posture 1107. The posture 1107 may be a posture in which the right foot of the user touches the ground and then, the left leg rotates in the forward direction.

During the motion of the posture 1101 and the posture 1103 of FIG. 11A, the processor 310 may determine the torque values (e.g., *τ_{R}*(*t*) and *τ_{L}*(*t*)) for both legs. The processor 310 may control the driving modules 30a and 30b based on the determined torque values, respectively, so that resistance torques (or resistive forces) corresponding to the determined torque values may be provided to the user (e.g., both legs of the user).

After the posture 1103, the processor 310 may detect the start of a designated motion of the right leg 1100 (e.g., a motion from when the rotation direction of the right hip joint of the user changes to when the right foot of the user touches the ground) in the damping exercise mode. For example, the processor 310 may detect that the right hip joint maximally or highly rotates in the forward direction (or that the rotation direction of the right hip joint changes from the forward direction to the backward direction) through the angle values and/or angular velocity values of the right hip joint.

When the start of the designated motion of the right leg 1100 is detected, the processor 310 may multiply the torque value (e.g., the torque value *τ_{R}*(*t*)) for the right leg 1100 by the predetermined (or certain) value (e.g., α), and control the second driving module 30b so that the resistance torque corresponding to the value (e.g., α·*τ_{R}*(*t*)) obtained by multiplying the torque value for the right leg 1100 by the predetermined value may be provided to the right leg 1100. When the start of the designated motion of the right leg 1100 is detected, the processor 310 may not multiply the torque value for the left leg by the predetermined value. Embodiments are not limited thereto, and according to embodiments, the processor 310 may multiply the torque value for the left leg by the predetermined value, even when the start of the designated motion of the right leg 1100 is detected.

According to an embodiment, the predetermined (or certain) value may be a value that enables a greater magnitude of resistance torque to be provided to the user during the designated motion of the right leg 1100. The predetermined value may be greater than, for example, "1". The greater the predetermined value is than "1", the greater the magnitude of the resistance torque may be provided during the designated motion of the right leg 1100. The processor 310 may determine (or adjust) the predetermined value based on a control signal received from the electronic device 120 or the other wearable device 130. For example, the processor 310 may receive a control signal to determine the predetermined value to be "1.2" from the electronic device 120 or the other wearable device 130. The processor 310 may determine (or adjust) the predetermined value to be "1.2" based on the received control signal.

During the designated motion of the right leg 1100, the wearable device 110 may provide a resistance torque of a greater magnitude in a direction opposite to the rotation direction of the right leg 1100 to the right leg 1100, thereby mitigating the impact when the right foot touches the ground. Impact mitigation is described in detail with reference to FIG. 11C.

The processor 310 may detect the end of the designated motion of the right leg 1100 (e.g., a motion from when the rotation direction of the right hip joint of the user changes to when the right foot of the user touches the ground) in the damping exercise mode. For example, the processor 310 may check whether the right foot of the user touches the ground through at least some or all of the motion information (e.g., 3-axis accelerations and rotation angles) obtained by the IMU 360.

When it is checked that the right foot touches the ground, the processor 310 may not multiply the torque value for the right leg 1100 by the predetermined value. The processor 310 may control the driving modules 30a and 30b so that resistance torques corresponding to the torque values (e.g., *τ_{R}*(*t*) and *τ_{L}*(*t*)) for both legs may be provided to the user (e.g., both legs of the user).

FIG. 11B illustrates an example of postures 1101 to 1107 of the user while the user is performing a forward walking exercise, and illustrates an embodiment of an operation of performing torque control while the wearable device 110 provides a resistance torque to the user (e.g., an operation of changing the value of a gain) in a damping exercise mode.

According to an embodiment, the wearable device 110 may provide a resistance torque (or resistance force) to a motion of the user in a damping exercise mode. For example, the wearable device 110 may determine torque values (e.g., *τ_{R}*(*t*) and *τ_{L}*(*t*)) based on the difference value between the angle values of both hip joint angles of the user and the values of the parameters. The wearable device 110 may provide resistance torques corresponding to the determined torque values to the user. At this time, the wearable device 110 may increase the value of the gain before the foot in the air (e.g., the right foot) touches the ground. Hereinafter, an embodiment of torque control in a damping exercise mode is described with reference to the example shown in FIG. 11B.

In the example shown in FIG. 11B, during the motion of the posture 1101 and the posture 1103, the processor 310 may determine the torque values (e.g., *τ_{R}*(*t*) and *τ_{L}*(*t*)) based on the difference value between the angle values of both hip joints of the user and the values of the parameters, and control the driving module 30 based on the determined torque values so that the resistance torques (or resistive forces) corresponding to the determined torque values may be provided to the user. The value of the gain may be k₁, and k₁ may be negative.

The processor 310 may detect the start of the designated motion of the right leg 1100 (e.g., a motion from when the rotation direction of the right hip joint of the user changes to when the right foot of the user touches the ground) in the damping exercise mode. For example, the processor 310 may detect that the right hip joint maximally or highly rotates in the forward direction (or that the rotation direction of the right hip joint changes from the forward direction to the backward direction) through the angle values and/or angular velocity values of the right hip joint. In this case, the processor 310 may change the value of the gain from k₁ to k₂. k₂ may be negative, and the absolute value of k₂ may be greater than the absolute value of k₁. During the designated motion of the right leg 1100, the processor 310 may control the driving module 30 to provide a greater magnitude of resistance torque by increasing the value of the gain. Accordingly, the impact on the right foot touching the ground may be mitigated. Impact mitigation is described below with reference to FIG. 11C.

The processor 310 may detect the end of the designated motion of the right leg 1100 (e.g., a motion from when the rotation direction of the right hip joint of the user changes to when the right foot of the user touches the ground) in the damping exercise mode. For example, the processor 310 may check whether the right foot of the user touches the ground through at least some or all of the motion information (e.g., 3-axis accelerations and rotation angles) obtained by the IMU 360. The processor 310 may set the changed value (=k₂) of the gain back to the original value (=k₁) when it is checked that the right foot touches the ground. The processor 310 may control the driving module 30 so that a resistance torque with the value of the gain of k₁ may be provided to the user, in the posture 1107.

According to embodiments, the processor 310 may determine (or calculate) the impact strength when the right foot and the left foot touch the ground through at least some or all of the motion information (e.g., 3-axis accelerations and rotation angles) obtained by the IMU 360 before operating in the damping exercise mode. The processor 310 may calculate the average step length value of one step of the user through previous exercise result data, and set a length value corresponding to a predetermined (or certain) ratio (e.g., 0.9) of the step length value as a reference value for changing the value of the gain. For example, when the average step length value of one step of the user is 65 cm, the reference value may be 58.5 cm. The processor 310 may obtain the angle values of the right hip joint of the user performing the exercise in the damping exercise mode, and check whether the step length of one step of the user reaches the reference value through the angle values of the right hip joint. When the step length of one step of the user reaches the reference value, the processor 310 may multiply the torque value (e.g., *τ_{R}*(*t*)) for the right leg 1100 by the predetermined value (e.g., α) or change the value of the gain (e.g., from k₁ to k₂). The processor 310 may obtain the motion information (e.g., 3-axis accelerations and rotation angles) from the IMU 360, and determine (or calculate) the impact strength when the right foot touches the ground through at least some or all of the obtained motion information. The processor 310 may determine that the damping exercise mode operates properly when the impact strength of the right foot when operating in the damping exercise mode is less than the impact strength of the right foot before operating in the damping exercise mode.

FIG. 11C illustrates a posture in which the right leg 1100 of the user touches the ground.

In the example shown in FIG. 11C, the rotation direction of the right leg 1100 may be the clockwise direction.

When the processor 310 does not multiply the torque value by a predetermined (or certain) value at the start of the designated motion of the right leg 1100, the magnitude of the resistance torque provided to the user immediately before the right foot touches the ground may be, for example, 1 newton-meter (Nm). As described with reference to FIG. 11A, when the processor 310 detects the start of the designated motion of the right leg 1100 and then multiplies the torque value (e.g., *τ_{R}*(*t*)) by the predetermined value, the magnitude of the resistance torque provided to the user immediately before the right foot touches the ground may be 1.2 to 1.5 Nm, which is 20 to 50% greater than the magnitude of the resistance torque (e.g., 1 Nm) when the torque value (e.g., *τ_{R}*(*t*)) is not multiplied by the predetermined value. In the case of multiplying the torque value (e.g., *τ_{R}*(*t*)) by the predetermined value, compared to the case of not multiplying the torque value (e.g., *τ_{R}*(*t*)) by the predetermined value, a greater magnitude of resistance torque may be provided in a direction opposite to the rotation direction of the right leg 1100 during the designated motion of the right leg 1100. Accordingly, in the case of multiplying the torque value (e.g., *τ_{R}*(*t*)) by the predetermined value, compared to the case of not multiplying the torque value (e.g., *τ_{R}*(*t*)) by the predetermined value, the rotation velocity of the right leg 1100 of the user may decrease and the velocity at which the right foot touches the ground may decrease, during the designated motion of the right leg 1100. Accordingly, in the case of multiplying the torque value with the predetermined value, compared to the case of not multiplying the torque value by the predetermined value, the impact on the right foot touching the ground may be mitigated.

When the processor 310 maintains the value of the gain to be k₁ at the start of the designated motion of the right leg 1100, the magnitude of the resistance torque provided to the user immediately before the right foot touches the ground may be, for example, 1 Nm. As described with reference to FIG. 11B, when the processor 310 detects the start of the designated motion of the right leg 1100 and then changes the value of the gain from k₁ to k₂, the magnitude of the resistance torque provided to the user immediately before the right foot touches the ground may be 1.2 to 1.5 Nm, which is 20 to 50% greater than the magnitude of the resistance torque (e.g., 1 Nm) having the value of the gain of k₁. When the value of the gain is k₂, compared to when the value of the gain is k₁, a greater magnitude of resistance torque may be provided in a direction opposite to the rotation direction of the right leg 1100 during the designated motion of the right leg 1100. Accordingly, when the value of the gain is k₂, compared to when the value of the gain is k₁, the rotation velocity of the right leg 1100 of the user may decrease and the velocity at which the right foot touches the ground may decrease, during the designated motion of the right leg 1100. Accordingly, when the value of the gain is k₂, compared to when the value of the gain is k₁, the impact on the right foot touching the ground may be mitigated.

FIG. 12A illustrates an example of postures 1201 to 1207 while the user is performing a forward walking exercise, and illustrates an embodiment of an operation of performing torque control while the wearable device 110 provides a resistance torque to the left leg 1200 (e.g., an operation of multiplying a torque value for the other one leg by a predetermined (or certain) value) in a damping exercise mode.

According to an embodiment, the wearable device 110 may provide a resistance torque (or resistance force) to a motion of the user in a damping exercise mode. For example, the wearable device 110 may determine torque values (e.g., *τ_{R}*(*t*) and *τ_{L}*(*t*)) for both legs based on the difference value between the angle values of both hip joint angles of the user and the values of the parameters. The wearable device 110 may provide a resistance torque corresponding to the torque value (e.g., *τ_{R}*(*t*)) for the right leg to the right leg of the user, and provide a resistance torque corresponding to the torque value (e.g., *τ_{L}*(*t*)) for the left leg 1200 to the left leg 1200 of the user. At this time, the wearable device 110 may provide a greater magnitude of resistance torque to the leg (e.g., the left leg 1200) by multiplying the torque value (e.g., *τ_{L}*(*t*) of FIG. 12A) for the leg (e.g., the left leg 1200) by a predetermined value (e.g., α of FIG. 12A) before the foot in the air (e.g., the left foot) touches the ground. Hereinafter, an embodiment of torque control is described with reference to the example shown in FIG. 12A.

In the example shown in FIG. 12A, the posture 1201 may be a posture in which the left foot touches the ground in the back. In the posture 1201, the rotation direction of the left hip joint may be the forward direction (e.g., the counterclockwise direction in FIG. 12A). The posture 1203 may be a posture in which the left leg 1200 and the right leg cross and then, the left hip joint is extended forward. In the posture 1203, the rotation direction of the left hip joint may be the forward direction. A posture in which the left hip joint is maximally or largely extended forward may be present between the posture 1203 and the posture 1205, and after the left hip joint is maximally or highly extended forward, the rotation direction of the left hip joint may change from the forward direction to the backward direction (e.g., the clockwise direction in FIG. 12A). A posture in which the left foot touches the ground may be present between the posture 1205 and the posture 1207. The posture 1207 may be a posture in which the left foot of the user touches the ground and then, the right leg rotates in the forward direction.

During the motion of the posture 1201 and the posture 1203 of FIG. 12A, the processor 310 may determine the torque values (e.g., *τ_{R}*(*t*) and *τ_{L}*(*t*)) for both legs. The processor 310 may control the driving modules 30a and 30b based on the determined torque values, respectively, so that resistance torques (or resistive forces) corresponding to the determined torque values may be provided to the user (e.g., both legs of the user).

After the posture 1203, the processor 310 may detect the start of a designated motion of the left leg 1200 (e.g., a motion from when the rotation direction of the left hip joint of the user changes to when the right foot of the user touches the ground) in the damping exercise mode. For example, the processor 310 may detect that the left hip joint maximally or largely rotates in the forward direction (or that the rotation direction of the left hip joint changes from the forward direction to the backward direction) through the angle values and/or angular velocity values of the left hip joint.

When the start of the designated motion of the left leg 1200 is detected, the processor 310 may multiply the torque value (e.g., the torque value *τ_{L}*(*t*)) for the left leg 1200 by the predetermined (or certain) value, and control the first driving module 30a so that the resistance torque corresponding to the value (e.g., *α·τ_{L}*(*t*)) obtained by multiplying the torque value for the left leg 1200 by the predetermined value may be provided to the left leg 1200. The predetermined value may be a value that enables a greater magnitude of resistance torque to be provided to the user during the designated motion of the left leg 1200. The predetermined value may be greater than, for example, "1" and may be adjusted. When the start of the designated motion of the left leg 1200 is detected, the processor 310 may not multiply the torque value for the right leg by the predetermined value. Embodiments are not limited thereto, and according to embodiments, the processor 310 may multiply the torque value for the right leg by the predetermined value, even when the start of the designated motion of the left leg 1200 is detected.

During the designated motion of the left leg 1200, the wearable device 110 may provide a resistance torque of a greater magnitude in a direction opposite to the rotation direction of the left leg 1200 to the left leg 1200, thereby mitigating the impact when the left foot touches the ground. Impact mitigation is described in detail with reference to FIG. 12C.

The processor 310 may detect the end of the designated motion of the left leg 1200 (e.g., a motion from when the rotation direction of the left hip joint of the user changes to when the left foot of the user touches the ground) in the damping exercise mode. For example, the processor 310 may check whether the left foot of the user touches the ground through at least some or all of the motion information (e.g., 3-axis accelerations and rotation angles) obtained by the IMU 360.

When it is checked that the left foot touches the ground, the processor 310 may not multiply the torque value for the left leg by the predetermined (or certain) value (e.g., α). The processor 310 may control the driving modules 30a and 30b so that resistance torques corresponding to the torque values (e.g., *τ_{R}*(*t*) and *τ_{L}*(*t*)) for both legs may be provided to the user (e.g., both legs of the user).

FIG. 12B illustrates an example of postures 1201 to 1207 of the user while the user is performing a forward walking exercise, and an example of an embodiment of an operation of performing torque control while the wearable device 110 provides a resistance torque to the user (e.g., an operation of changing the value of a gain) in a damping exercise mode.

According to an embodiment, the wearable device 110 may provide a resistance torque (or resistance force) to a motion of the user in a damping exercise mode. For example, the wearable device 110 may determine torque values (e.g., *τ_{R}*(*t*) and *τ_{L}*(*t*)) based on the difference value between the angle values of both hip joint angles of the user and the values of the parameters. The wearable device 110 may provide resistance torques corresponding to the determined torque values to the user. At this time, the wearable device 110 may provide a greater magnitude of resistance torque by increasing the value of the gain before the foot in the air (e.g., the left foot) touches the ground. Hereinafter, an embodiment of torque control is described with reference to the example shown in FIG. 12B.

In the example shown in FIG. 12, during the motion of the posture 1201 and the posture 1203, the processor 310 may determine the torque values (e.g., *τ_{R}*(*t*) and *τ_{L}*(*t*)) based on the difference value between the angle values of both hip joints of the user and the values of the parameters, and control the driving module 30 based on the determined torque values so that the resistance torques (or resistive forces) corresponding to the determined torque values may be provided to the user. The value of the gain may be k₁, and k₁ may be negative.

The processor 310 may detect the start of the designated motion of the left leg 1200 (e.g., a motion from when the rotation direction of the left hip joint of the user changes to when the left foot of the user touches the ground) in the damping exercise mode. For example, the processor 310 may detect that the left hip joint maximally or largely rotates in the forward direction (or that the rotation direction of the left hip joint changes from the forward direction to the backward direction) through the angle values and/or angular velocity values of the left hip joint. In this case, the processor 310 may change the value of the gain from k₁ to k₂. k₂ may be negative, and the absolute value of k₂ may be greater than the absolute value of k₁. During the designated motion of the left leg 1200, the processor 310 may control the driving module 30 to provide a greater magnitude of resistance torque by increasing the value of the gain. Accordingly, the impact on the left foot touching the ground may be mitigated. Impact mitigation is described below with reference to FIG. 12C.

The processor 310 may detect the end of the designated motion of the left leg 1200 (e.g., a motion from when the rotation direction of the left hip joint of the user changes to when the left foot of the user touches the ground). For example, the processor 310 may check whether the left foot of the user touches the ground through at least some or all of the motion information (e.g., 3-axis accelerations and rotation angles) obtained by the IMU 360. The processor 310 may set the changed value (=k₂) of the gain back to the original value (=k₁) when it is checked that the left foot touches the ground. The processor 310 may control the driving module 30 so that a resistance torque with the value of the gain of k₁ may be provided to the user, in the posture 1207.

According to embodiments, the processor 310 may obtain the angle values of the left hip joint of the user performing the exercise in the damping exercise mode, and check whether the step length of one step of the user reaches the reference value through the angle values of the left hip joint. When the step length of one step of the user reaches the reference value, the processor 310 may multiply the torque value for the left leg by the predetermined (or certain) value as described with reference to FIG. 12A or change the value of the gain as described with reference to FIG. 12B. The processor 310 may obtain the motion information (e.g., 3-axis accelerations and rotation angles) from the IMU 360, and determine the impact strength when the left foot touches the ground through at least some or all of the obtained motion information. The processor 310 may determine that the damping exercise mode operates properly when the impact strength of the left foot when operating in the damping exercise mode is less than the impact strength of the left foot before operating in the damping exercise mode.

FIG. 12C illustrates a posture in which the left leg 1200 of the user touches the ground.

In the example shown in FIG. 12C, the rotation direction of the left leg 1200 may be the clockwise direction.

When the processor 310 does not multiply the torque value by a predetermined (or certain) value at the start of the designated motion of the left leg 1200, the magnitude of the resistance torque provided to the user immediately before the left foot touches the ground may be, for example, 1 Nm. As described with reference to FIG. 12A, when the processor 310 detects the start of the designated motion of the left leg 1200 and then multiplies the torque value (e.g., *τ_{L}*(*t*)) for the left leg by the predetermined value, the magnitude of the resistance torque provided to the user immediately before the left foot touches the ground may be 1.2 to 1.5 Nm, which is 20 to 50% greater than the magnitude of the resistance torque (e.g., 1 Nm) when the torque value (e.g., *τ_{L}*(*t*)) is not multiplied by the predetermined value. In the case of multiplying the torque value (e.g., *τ_{L}*(*t*)) by the predetermined value, compared to the case of not multiplying the torque value (e.g., *τ_{L}*(*t*)) by the predetermined value, a greater magnitude of resistance torque may be provided in a direction opposite to the rotation direction of the left leg 1200 during the designated motion of the left leg 1200. Accordingly, in the case of multiplying the torque value (e.g., *τ_{L}*(*t*)) by the predetermined value, compared to the case of not multiplying the torque value (e.g., *τ_{L}*(*t*)) by the predetermined value, the rotation velocity of the left leg 1200 of the user may decrease and the velocity at which the left foot touches the ground may decrease, during the designated motion of the left leg 1200. Accordingly, in the case of multiplying the torque value (e.g., *τ_{L}*(*t*)) with the predetermined value, compared to the case of not multiplying the torque value (e.g., *τ_{L}*(*t*)) by the predetermined value, the impact on the left foot touching the ground may be mitigated.

When the processor 310 maintains the value of the gain to be k₁ at the start of the designated motion of the left leg 1200, the magnitude of the resistance torque provided to the user immediately before the left foot touches the ground may be, for example, 1 Nm. As described with reference to FIG. 11B, when the processor 310 detects the start of the designated motion of the left leg 1200 and then changes the value of the gain from k₁ to k₂, the magnitude of the resistance torque provided to the user immediately before the left foot touches the ground may be 1.2 to 1.5 Nm, which is 20 to 50% greater than the magnitude of the resistance torque (e.g., 1 Nm) having the value of the gain of k₁. When the value of the gain is k₂, compared to when the value of the gain is k₁, a greater magnitude of resistance torque may be provided in a direction opposite to the rotation direction of the left leg 1200 during the designated motion of the left leg 1200. Accordingly, when the value of the gain is k₂, compared to when the value of the gain is k₁, the rotation velocity of the left leg 1200 of the user may decrease and the velocity at which the left foot touches the ground may decrease, during the designated motion of the left leg 1200. Accordingly, when the value of the gain is k₂, compared to when the value of the gain is k₁, the impact on the right foot touching the ground may be mitigated.

The examples of providing a resistance torque to the user during motions other than the designated motion in the damping exercise mode have been described with reference to FIGS. 11A to 12C, but embodiments are not limited thereto. The processor 310 may control the driving module 30 so that an assistance torque may be provided to the user during a motion other than the designated motion (e.g., the designated motion of the right leg 1100 and/or the designated motion of the left leg 1200 described above) in the damping exercise mode. When the start of the designated motion is detected, the processor 310 may multiply the torque value for a leg performing the designated motion by a predetermined (or certain) value (e.g., a negative value less than "-1") and control the driving module 30 so that a resistance torque may be provided to the leg performing the designated motion. When the end of the designated motion is detected, the processor 310 may not multiply the torque value by the negative value, and may control the driving module 30 so that an assistance torque may be provided to the user. According to embodiments, when the start of the designated motion is detected, the processor 310 may change the value of the gain (e.g., from a positive gain value to a negative gain value) and control the driving module 30 so that a resistance torque may be provided to the user during the designated motion. When the end of the designated motion is detected, the processor 310 may set the changed value (e.g., the negative gain value) of the gain back to the original value (e.g., the positive gain value), and control the driving module 30 so that an assistance torque may be provided to the user.

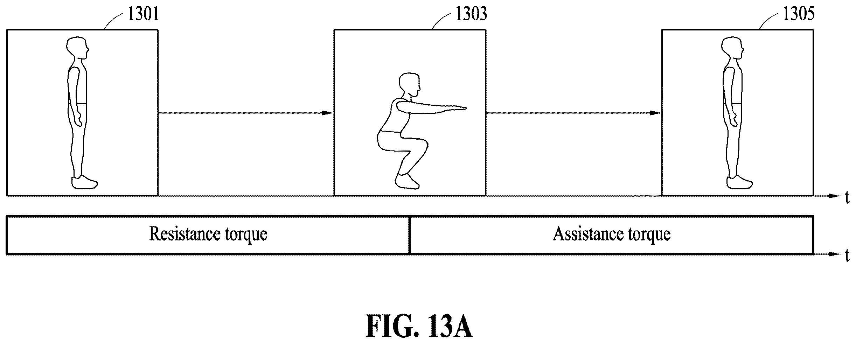

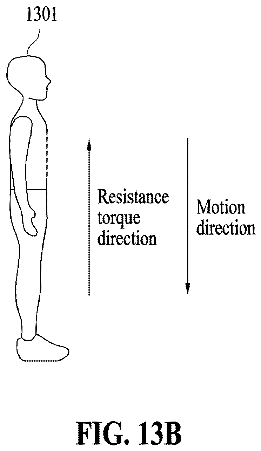

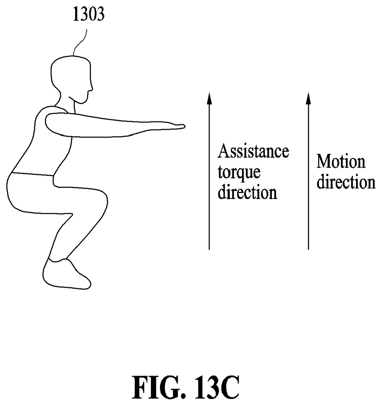

FIGS. 13A, 13B, and 13C are diagrams illustrating examples of an operation of a wearable device in a damping exercise mode according to an embodiment.

In the example shown in FIG. 13A, a user wearing the wearable device 110 may perform an in-place exercise (e.g., squats). The embodiment described with reference to FIG. 13A may also apply even when the user performs other in-place exercises (e.g., lunges, a walking-in-place exercise, etc.).

FIG. 13A illustrates an example of postures 1301 to 1305 of a squat exercise of the user and an embodiment of an operation of performing torque control of the wearable device 110 (e.g., an operation of changing the value of a gain) in a damping exercise mode.

In the example shown in FIG. 13A, the posture 1301 may be a posture in which the user is standing, the posture 1303 may be a posture in which the user maximally or largely bends both knees in the squat exercise, and the posture 1305 may be a posture in which the user is standing.

According to an embodiment, the wearable device 110 (e.g., the processor 310) may provide a resistance torque to the user when the user starts bending the knees, and provide an assistance torque to the user when the user starts extending the maximally or largely bent knees, in the damping exercise mode.

For example, the processor 310 may detect that the joints (e.g., the hip joints or the knee joints) are bent through the angle values of the joints (e.g., the hip joints or the knee joints). When it is detected that the joints (e.g., the hip joints or the knee joints) are bent, the processor 310 may determine the torque values based on the difference value between the angle values of both hip joints and the values of parameters (e.g., a gain and a delay), and provide resistance torques (or resistance forces) corresponding to the determined torque values to the user. As in the example shown in FIG. 13B, the processor 310 may control the driving module 30 to provide a resistance torque to the user in a direction opposite to the motion direction of the thighs while the thighs (or the hip joints) of the user go down (e.g., during the motion starting from the posture 1301 to the posture 1303) in the damping exercise mode, thereby helping the thighs (or the hip joints) of the user to go down slowly. Accordingly, less load may be provided to the knees of the user, and the impact on the knees may be mitigated.

The processor 310 may detect that the knees of the user start to go up through the angle values (or angular velocity values) of the joints (e.g., the hip joints or the knee joints). The processor 310 may detect that the rotation direction of the joints changes through the angular velocity values of the joints. In this case, the processor 310 may change the value of the gain from a negative value to a positive value, and control the driving module 30 so that an assistance torque may be provided to the user. When the thighs (or the hip joints) of the user start to go up in the posture 1303, the processor 310 may determine the torque values based on the difference value between the angle values of both hip joints of the user and the values of the parameters (e.g., the gain and the delay), and control the driving module 30 so that assistance torques (or assistance forces) corresponding to the determined torque values may be provided. As in the example shown in FIG. 13C, the processor 310 may control the driving module 30 so that assistance torques may be provided to the user in a direction the same as the motion direction of the thighs while the thighs of the user go up (e.g., during the motion starting from the posture 1303 to the posture 1305) in the damping exercise mode. Accordingly, less load may be provided to the knees of the user, and the impact on the knees of the user may be mitigated.

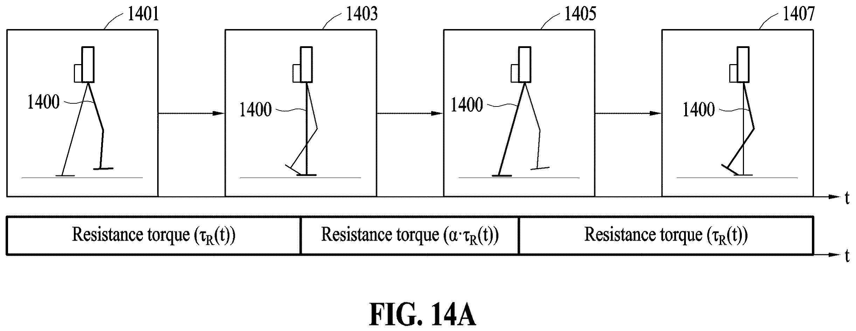

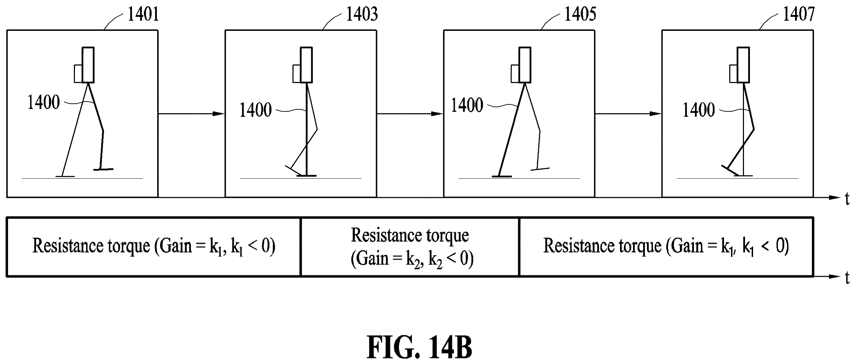

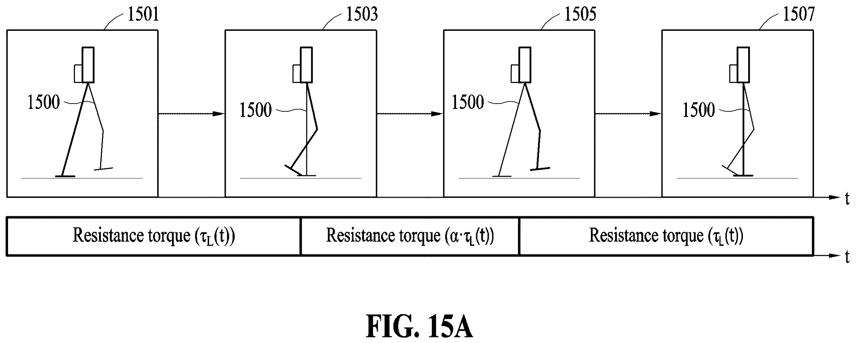

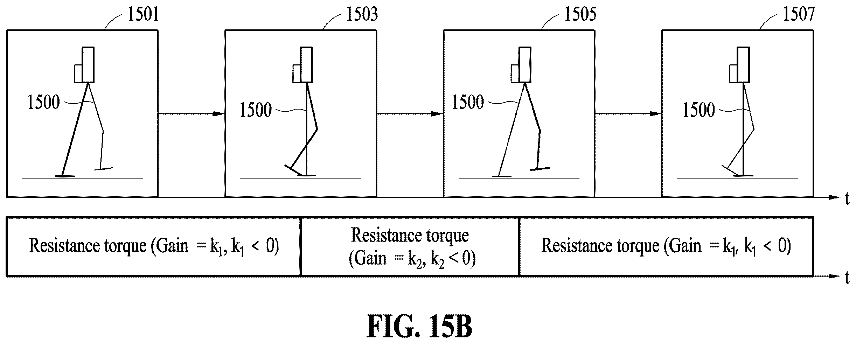

FIGS. 14A, 14B, 15A, and 15B are diagrams illustrating examples of an operation of a wearable device in a muscle strengthening exercise mode according to an embodiment.

FIG. 14A illustrates an example of postures 1401 to 1407 of the user while the user is performing a forward walking exercise, and illustrates an embodiment of an operation of performing torque control while the wearable device 110 provides a resistance torque to the user (e.g., an operation of multiplying a torque value for one leg by a predetermined (or certain) value) in a muscle strengthening exercise mode.

According to an embodiment, the wearable device 110 may provide a resistance torque (or resistance force) to a motion of the user in a muscle strengthening exercise mode. For example, the wearable device 110 may determine torque values (e.g., *τ_{R}*(*t*) and *τ_{L}*(*t*)) for both legs based on the difference value between the angle values of both hip joint angles of the user and the values of the parameters. At this time, the wearable device 110 may multiply the torque value for the right leg 1400 by a predetermined (or certain) value when the right hip joint angle has the first value while the right hip joint of the user rotates in the backward direction. The wearable device 110 may multiply the torque value for the right leg 1400 by the predetermined (or certain) value so that a greater magnitude of resistance torque may be provided for a motion of pushing the right leg 1400 backward. Hereinafter, an embodiment of torque control in a muscle strengthening exercise mode is described with reference to the example shown in FIG. 14A.

In the example shown in FIG. 14A, the posture 1401 may be a posture before the right foot touches the ground, and the rotation direction of the right hip joint may be the backward direction (e.g., the clockwise direction in FIG. 14A). The posture 1403 may be a posture in which the right foot of the user touches the ground and then, the left leg rotates in the forward direction (e.g., the counterclockwise direction in FIG. 14A). The angle value of the right hip joint angle in the posture 1403 may be a first value (e.g., 0 degrees), and the rotation direction of the right hip joint may be the backward direction. The posture 1405 may indicate a posture in which the right hip joint of the user is maximally or largely extended in the backward direction. Before and after the posture 1405, the rotation direction of the right hip joint may change from the backward direction to the forward direction. The posture 1407 may be a posture in which the left foot touches the ground and then, the right leg 1400 rotates in the forward direction.

In the posture 1401 of FIG. 14A, the processor 310 may determine the torque values (e.g., *τ_{R}*(*t*) and *τ_{L}*(*t*)) for both legs. The processor 310 may control the driving modules 30a and 30b based on the determined torque values, respectively, so that resistance torques (or resistive forces) corresponding to the determined torque values may be provided to the user (e.g., both legs of the user).

The processor 310 may detect the start of a designated motion of the right leg 1400 (e.g., a motion from when the first value of the right hip joint angle is the first value to when the rotation direction of the right hip joint changes from the backward direction to the forward direction) in the muscle strengthening exercise mode through the angle values (or angular velocity values) of the right hip joint angle. The processor 310 may detect that the rotation direction of the right hip joint is the backward direction and that the angle value of the right hip joint angle is the first value (e.g., 0 degrees), through the angle values (or angular velocity values) of the right hip joint angle.

When the start of the designated motion of the right leg 1400 is detected, the processor 310 may multiply the torque value (e.g., *τ_{R}*(*t*)) for the right leg 1400 by a predetermined (or certain) value (e.g., α). The predetermined value may be a value that enables a greater magnitude of resistance torque to be provided to the user during the designated motion of the right leg 1400. The predetermined value may be greater than, for example, "1" and may be adjusted. During the designated motion of the right leg 1400, the magnitude of the resistance torque when the torque value for the right leg 1400 is multiplied by the predetermined value may be greater than the magnitude of the resistance torque when the torque value for the right leg 1400 is not multiplied by the predetermined value. During the designated motion of the right leg 1400, the processor 310 may multiply the torque value for the right leg 1400 by the predetermined value (e.g., α) so that a greater magnitude of resistance torque may be provided for the motion of the user pushing the right leg 1400 backward. Accordingly, the processor 310 may help strengthen the muscles (e.g., the lower back muscles) of the user.

When the start of the designated motion of the right leg 1400 is detected, the processor 310 may not multiply the torque value for the left leg by the predetermined value. Embodiments are not limited thereto, and according to embodiments, the processor 310 may multiply the torque value for the left leg by the predetermined value, even when the start of the designated motion of the right leg 1400 is detected.

The processor 310 may detect the end of the designated motion of the right leg 1400 (e.g., the motion from when the angle value of the right hip joint angle is the first value to when the rotation direction of the right hip joint changes from the backward direction to the forward direction). The processor 310 may detect that the right hip joint maximally or largely extended backward returns (or that the rotation direction of the right hip joint changes from the backward direction to the forward direction) through the angle values (or angular velocity values) of the right hip joint. In this case, the processor 310 may not multiply the torque value for the right leg 1400 by the predetermined value. The processor 310 may control the driving modules 30a and 30b so that resistance torques corresponding to the torque values (e.g., *τ_{R}*(*t*) and *τ_{L}*(*t*)) for both legs may be provided to both legs of the user.

FIG. 14B illustrates an example of postures 1401 to 1407 of the user while the user is performing a forward walking exercise, and an embodiment of an operation of performing torque control while the wearable device 110 provides a resistance torque to the user (e.g., an operation of changing the value of a gain) in a muscle strengthening exercise mode.

According to an embodiment, the wearable device 110 may provide a resistance torque (or resistance force) to a motion of the user in a muscle strengthening exercise mode. For example, the wearable device 110 may determine torque values (e.g., *τ_{R}*(*t*) and *τ_{L}*(*t*)) based on the difference value between the angle values of both hip joint angles of the user and the values of the parameters. The wearable device 110 may provide resistance torques corresponding to the determined torque values to the user. At this time, the wearable device 110 may change the value of the gain when the right hip joint angle has the first value while the right hip joint of the user rotates in the backward direction. The wearable device 110 may increase the value of the gain so that a greater magnitude of resistance torque may be provided for the motion of pushing the right leg 1400 backward. Hereinafter, an embodiment of torque control in a muscle strengthening exercise mode is described with reference to the example shown in FIG. 14B.

In the posture 1401 of FIG. 14B, the processor 310 may determine the torque values (e.g., *τ_{R}*(*t*) and *τ_{L}*(*t*)) based on the difference value between the angle values of both hip joints of the user and the values of the parameters, and control the driving module 30 based on the determined torque values so that the resistance torques (or resistive forces) corresponding to the determined torque values may be provided to the user. The value of the gain may be k₁, and k₁ may be negative.

The processor 310 may detect the start of a designated motion of the right leg 1400 (e.g., a motion from when the angle value of the right hip joint angle is the first value to when the rotation direction of the right hip joint changes from the backward direction to the forward direction) in the muscle strengthening exercise mode through the angle values (or angular velocity values) of the right hip joint angle. The processor 310 may detect that the rotation direction of the right hip joint is the backward direction and that the angle value of the right hip joint angle is the first value (e.g., 0 degrees), through the angle values (or angular velocity values) of the right hip joint angle. In this case, the processor 310 may change the value of the gain from k₁ to k₂. k₂ may be negative, and the absolute value of k₂ may be greater than the absolute value of k₁. During the designated motion of the right leg 1400 (e.g., the motion from when the angle value of the right hip joint angle is the first value to when the rotation direction of the right hip joint changes from the backward direction to the forward direction), the processor 310 may increase the value of the gain, thereby controlling the driving module 30 so that a greater magnitude of resistance torque may be provided. The processor 310 may change the value of the gain so that a greater magnitude of resistance torque may be provided for the motion of the user pushing the right leg 1400 backward, thereby helping strengthen the muscles (e.g., the lower back muscles) of the user.

The processor 310 may detect the end of the designated motion of the right leg 1400 (e.g., the motion from when the angle value of the right hip joint angle is the first value to when the rotation direction of the right hip joint changes from the backward direction to the forward direction). The processor 310 may detect that the right hip joint maximally or largely extended backward returns (or that the rotation direction of the right hip joint changes from the backward direction to the forward direction) through the angle values (or angular velocity values) of the right hip joint. In this case, the processor 310 may return the changed value (=k₂) of the gain to the original value (=k₁). The processor 310 may control the driving module 30 so that a resistance torque with the value of the gain of k₁ may be provided to the user, in the posture 1407.

FIG. 15A illustrates an example of postures 1501 to 1507 of the user while the user is performing a forward walking exercise, and an embodiment of an operation of performing torque control while the wearable device 110 provides a resistance torque to the user (e.g., an operation of multiplying a torque value for the other leg by a predetermined (or certain) value) in a muscle strengthening exercise mode.

According to an embodiment, the wearable device 110 may provide a resistance torque (or resistance force) to a motion of the user in a muscle strengthening exercise mode. For example, the wearable device 110 may determine torque values (e.g., *τ_{R}*(*t*) and *τ_{L}*(*t*)) for both legs based on the difference value between the angle values of both hip joint angles of the user and the values of the parameters. At this time, the wearable device 110 may multiply the torque value for the left leg 1500 by a predetermined value when the left hip joint angle has the first value while the left hip joint of the user rotates in the backward direction. The wearable device 110 may multiply the torque value for the left leg 1500 by the predetermined value so that a greater magnitude of resistance torque may be provided for a motion of pushing the left leg 1500 backward. Hereinafter, an embodiment of torque control in a muscle strengthening exercise mode is described with reference to the example shown in FIG. 15A.

In the example shown in FIG. 15A, the posture 1501 may be a posture before the left foot touches the ground, and the rotation direction of the left hip joint may be the backward direction (e.g., the clockwise direction in FIG. 15A). The posture 1503 may be a posture in which the left foot of the user touches the ground and then, the right leg rotates in the forward direction (e.g., the counterclockwise direction in FIG. 15A). The angle value of the left hip joint angle in the posture 1503 may be a first value (e.g., 0 degrees), and the rotation direction of the left hip joint may be the backward direction. The posture 1505 may indicate a posture in which the left hip joint of the user is maximally or highly extended in the backward direction. Before and after the posture 1505, the rotation direction of the left hip joint may change from the backward direction to the forward direction. The posture 1507 may be a posture in which the right foot touches the ground and then, the left leg 1500 rotates in the forward direction.

In the posture 1501 of FIG. 15A, the processor 310 may determine the torque values (e.g., *τ_{R}*(*t*) and *τ_{L}*(*t*)) for both legs. The processor 310 may control the driving modules 30a and 30b based on the determined torque values, respectively, so that resistance torques (or resistive forces) corresponding to the determined torque values may be provided to the user (e.g., both legs of the user).

The processor 310 may detect the start of a designated motion of the left leg 1500 (e.g., a motion from when the angle value of the left hip joint angle is the first value to when the rotation direction of the left hip joint changes from the backward direction to the forward direction) in the muscle strengthening exercise mode through the angle values (or angular velocity values) of the left hip joint angle. The processor 310 may detect that the rotation direction of the left hip joint is the backward direction and that the angle value of the left hip joint angle is the first value (e.g., 0 degrees), through the angle values (or angular velocity values) of the left hip joint angle.

When the start of the designated motion of the left leg 1500 is detected, the processor 310 may multiply the torque value (e.g., *τ_{L}*(*t*)) for the left leg 1500 by a predetermined (or certain) value (e.g., α). The predetermined value may be a value that enables a greater magnitude of resistance torque to be provided to the user during the designated motion of the left leg 1500. The predetermined value may be greater than, for example, "1" and may be adjusted. During the designated motion of the left leg 1500, the magnitude of the resistance torque when the torque value for the left leg 1500 is multiplied by the predetermined value may be greater than the magnitude of the resistance torque when the torque value for the left leg 1500 is not multiplied by the predetermined value. During the designated motion of the left leg 1500, the processor 310 may multiply the torque value for the left leg 1500 by the predetermined value (e.g., α) so that a greater magnitude of resistance torque may be provided for the motion of the user pushing the left leg 1500 backward. Accordingly, the processor 310 may help strengthen the muscles (e.g., the lower back muscles) of the user.

When the start of the designated motion of the left leg 1500 is detected, the processor 310 may not multiply the torque value for the right leg by the predetermined value. Embodiments are not limited thereto, and according to embodiments, the processor 310 may multiply the torque value for the right leg by the predetermined value, even when the start of the designated motion of the left leg 1500 is detected.

The processor 310 may detect the end of the designated motion of the left leg 1500 (e.g., the motion from when the angle value of the right hip joint angle is the first value to when the rotation direction of the right hip joint changes from the backward direction to the forward direction). The processor 310 may detect that the left hip joint maximally or significantly extended backward returns (or that the rotation direction of the left hip joint changes from the backward direction to the forward direction) through the angle values (or angular velocity values) of the left hip joint. In this case, the processor 310 may not multiply the torque value for the left leg 1500 by the predetermined value. The processor 310 may control the driving modules 30a and 30b so that resistance torques corresponding to the torque values (e.g., *τ_{R}*(*t*) and *τ_{L}*(*t*)) for both legs may be provided to both legs of the user.

FIG. 15B illustrates an example of postures 1501 to 1507 of the user while the user is performing a forward walking exercise, and an embodiment of an operation of performing torque control while the wearable device 110 provides a resistance torque to the user (e.g., an operation of changing the value of a gain) in a muscle strengthening exercise mode.

According to an embodiment, the wearable device 110 may provide a resistance torque (or resistance force) to a motion of the user in a muscle strengthening exercise mode. For example, the wearable device 110 may determine torque values (e.g., *τ_{R}*(*t*) and *τ_{L}*(*t*)) based on the difference value between the angle values of both hip joint angles of the user and the values of the parameters. The wearable device 110 may provide resistance torques corresponding to the determined torque values to the user. At this time, the wearable device 110 may change the value of the gain when the left hip joint angle has the first value while the left hip joint of the user rotates in the backward direction. The wearable device 110 may increase the value of the gain so that a greater magnitude of resistance torque may be provided for the motion of pushing the left leg 1500 backward. Hereinafter, an embodiment of torque control in a muscle strengthening exercise mode is described with reference to the example shown in FIG. 15B.

In the posture 1501 of FIG. 15B, the processor 310 may determine the torque values (e.g., *τ_{R}*(*t*) and *τ_{L}*(*t*)) based on the difference value between the angle values of both hip joints of the user and the values of the parameters, and control the driving module 30 based on the determined torque values so that the resistance torques (or resistive forces) corresponding to the determined torque values may be provided to the user. The value of the gain may be k₁, and k₁ may be negative.

The processor 310 may detect the start of a designated motion of the left leg 1500 (e.g., a motion from when the angle value of the left hip joint angle is the first value to when the rotation direction of the left hip joint changes from the backward direction to the forward direction) in the muscle strengthening exercise mode through the angle values (or angular velocity values) of the left hip joint angle. The processor 310 may detect that the rotation direction of the left hip joint is the backward direction and that the angle value of the left hip joint angle is the first value (e.g., 0 degrees), through the angle values (or angular velocity values) of the left hip joint angle. In this case, the processor 310 may change the value of the gain from k₁ to k₂. K₂ may be negative, and the absolute value of k₂ may be greater than the absolute value of k₁. During the designated motion of the left leg 1500 (e.g., the motion from when the angle value of the left hip joint angle is the first value to when the rotation direction of the left hip joint changes from the backward direction to the forward direction), the processor 310 may increase the value of the gain, thereby controlling the driving module 30 so that a greater magnitude of resistance torque may be provided. The processor 310 may change the value of the gain so that a greater magnitude of resistance torque may be provided for the motion of the user pushing the left leg 1500 backward, thereby strengthening the muscles (e.g., the lower back muscles) of the user.

The processor 310 may detect the end of the designated motion of the left leg 1500 (e.g., the motion from when the angle value of the left hip joint angle is the first value to when the rotation direction of the left hip joint changes from the backward direction to the forward direction). The processor 310 may detect that the left hip joint maximally or significantly extended backward returns (or that the rotation direction of the left hip joint changes from the backward direction to the forward direction) through the angle values (or angular velocity values) of the left hip joint. In this case, the processor 310 may return the changed value (=k₂) of the gain to the original value (=k₁). The processor 310 may control the driving module 30 so that a resistance torque with the value of the gain of k₁ may be provided to the user, in the posture 1507.

The examples of providing a resistance torque to the user during motions other than the designated motion in the muscle strengthening exercise mode are described with reference to FIGS. 14A to 15B, but embodiments are not limited thereto. The processor 310 may control the driving module 30 so that an assistance torque may be provided to the user during a motion other than the designated motion (e.g., the designated motion of the right leg 1400 and/or the designated motion of the left leg 1500 described above) in the muscle strengthening exercise mode. When the start of the designated motion is detected, the processor 310 may multiply the torque value for a leg performing the designated motion by a predetermined (or certain) value (e.g., a negative value less than "-1"), and control the driving module 30 so that a resistance torque may be provided to the leg performing the designated motion. When the end of the designated motion is detected, the processor 310 may not multiply the torque value by the predetermined value (e.g., the negative value less than "-1"), and may control the driving module 30 so that an assistance torque may be provided to the user. According to embodiments, when the start of the designated motion is detected, the processor 310 may change the value of the gain (e.g., from a positive gain value to a negative gain value) and control the driving module 30 so that a resistance torque may be provided to the user during the designated motion. When the end of the designated motion is detected, the processor 310 may set the changed value (e.g., the negative gain value) of the gain back to the original value (e.g., the positive gain value), and control the driving module 30 so that an assistance torque may be provided to the user.

According to an embodiment, the wearable device 110 may be wirelessly connected to a treadmill of the user. In the muscle strengthening exercise mode, the wearable device 110 may increase the slope of the treadmill to strengthen the lower back muscles of the user. The user may perform a walking exercise on the treadmill with the adjusted slope. The wearable device 110 may provide a torque (e.g., an assistance torque or a resistance torque) to the user performing a walking exercise on the treadmill with the adjusted slope. At this time, the wearable device 110 may multiply the torque value by a predetermined value or change the value of the gain. Embodiments are not limited thereto, and the wearable device 110 may not multiply the torque value by the predetermined value and may not change the value of the gain, when providing the torque to the user performing a walking exercise on the treadmill with the adjusted slope.

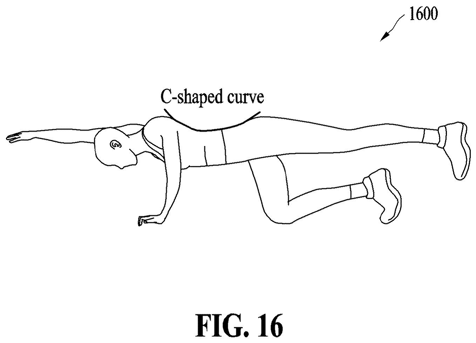

FIG. 16 is a diagram illustrating an example of an operation of a wearable device in a muscle strengthening exercise mode according to an embodiment.

FIG. 16 shows an example of a posture 1600 of an in-place exercise (e.g., a bird dog exercise) to strengthen the lower back muscles. The wearable device 110 may provide a guide and/or a torque for a bird dog exercise to the user in the muscle strengthening exercise mode so that the user may perform a bird dog exercise.

In the example shown in FIG. 16, the user may perform a bird dog exercise while wearing the wearable device 110 and the smart watch 132. The wearable device 110 may perform wireless communication with the smart watch 132. Embodiments are not limited thereto, and the wearable device 110 may perform wireless communication with the smart watch 132 through the electronic device 120.

According to an embodiment, the processor 310 of the wearable device 110 may determine whether the body of the user is parallel to the floor, through at least some or all of the motion information (e.g., 3-axis accelerations and rotation angles) obtained by the IMU 360. The processor 310 may receive angle values from the angle sensors 320 and 320-1, respectively. The processor 310 may receive sensing data obtained by a sensor (e.g., a gyro sensor) in the smart watch 132 from the smart watch 132.

According to an embodiment, the processor 310 of the wearable device 110 may determine whether the arm with the smart watch 132 on and the opposite knee are in contact with the ground, through at least part of the motion information obtained by the IMU 360, the angle values obtained by the angle sensors 320 and 320-1, or the sensing data received from the smart watch 132. In the example shown in FIG. 16, when the user wears the smart watch 132 on the left arm, the processor 310 may determine whether the left arm and the right knee are in contact with the ground, through at least part of the motion information obtained by the IMU 360, the angle values obtained by the angle sensors 320 and 320-1, or the sensing data received from the smart watch 132.

According to an embodiment, the processor 310 of the wearable device 110 may control to provide a guide to the posture of the bird dog exercise to the user. For example, in the example shown in FIG. 16, the processor 310 may control to provide the user with a guide to extending the right arm forward and extending the left leg backward while maintaining a C-shaped curve of the lower back of the user. The processor 310 may determine whether the body of the user is tilting or moving through the motion information obtained by the IMU 360 and control to provide a notification when the body of the user is tilting or moving.

According to an embodiment, the processor 310 may determine whether the user maintains the posture 1600 for a predetermined (or certain) period of time (e.g., 5 seconds).

The user may perform the opposite posture (e.g., a posture of extending the left arm forward and extending the right leg backward with the right arm and the left knee on the ground). The processor 310 may determine whether the body of the user is tilting or moving when the user performs the opposite posture, and control to provide a notification when the body of the user is tilting or moving.

According to an embodiment, the processor 310 may control the driving module 30 so that the driving module 30 may provide a torque (e.g., an assistance torque or a resistance torque) to the user while the user performs a bird dog exercise. For example, the wearable device 110 may provide a torque to the user when the user lifts the leg, and provide a torque to the user so that the user may maintain the leg lift posture.

According to an embodiment, the processor 310 may not change the value of the gain while the user performs a bird dog exercise. Embodiments are not limited thereto, and for example, the processor 310 may maintain the value of the gain when the leg of the user is lifted, and change the value of the gain (e.g., increase the value of the gain or decrease the value of the gain) when the user maintains the leg lift posture. As another example, the processor 310 may change the value of the gain (e.g., increase the value of the gain or decrease the value of the gain) when the leg of the user is lifted, and return the changed value of the gain to the original value when the user maintains the leg lift posture.

According to an embodiment, the processor 310 may control to provide the user with a guide to lifting one of the arm and the leg if it is difficult for the user to lift the arm and the leg at the same time.

According to an embodiment, the processor 310 may control to provide the user with a guide to stopping the exercise when the user feels pain during bird dog exercise.

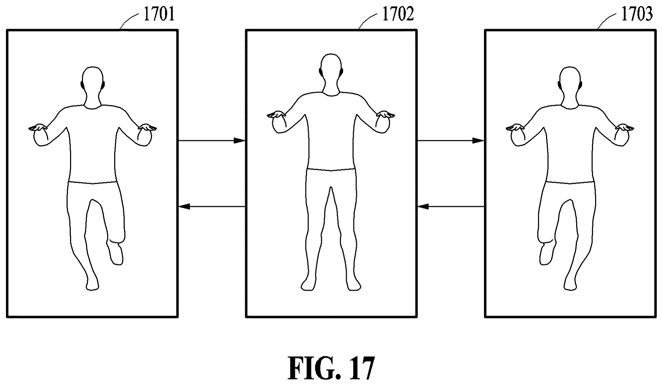

FIG. 17 is a diagram illustrating an example of an operation of a wearable device in a muscle strengthening exercise mode according to an embodiment.

Examples of postures 1701, 1702, and 1703 of an in-place exercise (e.g., a skating exercise) for strengthening the lower back muscles are shown in FIG. 17. The wearable device 110 may provide a guide and/or a torque to the user in the muscle strengthening exercise mode so that the user may perform a skating exercise.

In the example shown in FIG. 17, the user may perform a skating exercise while wearing the wearable device 110.

The user may perform a standing posture 1702.

The user may perform a posture 1703 by lifting one leg (e.g., the right leg) and bending the knee of the other leg (e.g., the left leg). The processor 310 of the wearable device 110 may determine whether the user maintains the posture 1703 for a predetermined (or certain) period of time (e.g., 2 seconds) through at least some or all of the motion information (e.g., 3-axis accelerations and rotation angles) obtained by the IMU 360. The processor 310 may determine whether the user maintains balance without falling in the posture 1703 through at least some or all of the motion information (e.g., 3-axis accelerations and rotation angles) obtained by the IMU 360.

The posture of the user may be changed from the posture 1703 to the posture 1702, and the user may perform the posture 1701 by bending the knee of one leg (e.g., the right leg) and lifting the other leg (e.g., the left leg). The processor 310 of the wearable device 110 may determine whether the user maintains the posture 1701 for a predetermined (or certain) period of time (e.g., 2 seconds) through at least some or all of the motion information (e.g., 3-axis accelerations and rotation angles) obtained by the IMU 360. The processor 310 may determine whether the user maintains balance without falling in the posture 1701 through at least some or all of the motion information (e.g., 3-axis accelerations and rotation angles) obtained by the IMU 360.

According to an embodiment, the processor 310 of the wearable device 110 may control to provide a guide to not bending the waist of the user when the user performs a skating exercise.

According to an embodiment, the processor 310 may control the driving module 30 so that a torque may be provided to the user while the user performs a skating exercise. At this time, the processor 310 may not change the value of the gain. Embodiments are not limited thereto, and for example, the processor 310 may maintain the value of the gain when the user bends one knee, and change the value of the gain (e.g., increase the value of the gain or decrease the value of the gain) when the user extends the bent knee. As another example, the processor 310 may change the value of the gain (e.g., increase the value of the gain or decrease the value of the gain) when the user bends one knee, and return the changed value of the gain to the original value when the user extends the bent knee.

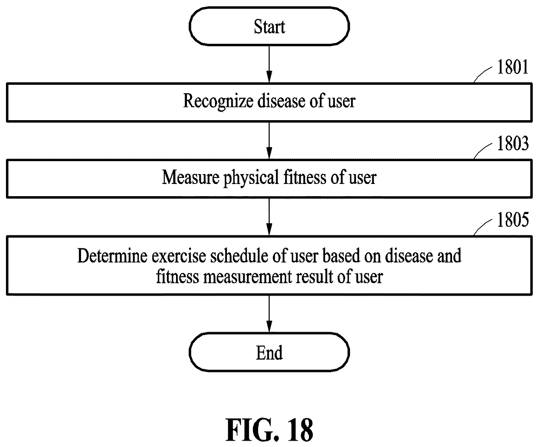

FIG. 18 is a flowchart illustrating an example of an exercise schedule generation method of a wearable device according to an embodiment.

Referring to FIG. 18, in operation 1801, the wearable device 110 (e.g., the processor 310) may recognize a disease of a user. The processor 310 may obtain information necessary or helpful for recognizing the disease of the user (e.g., at least one of a disease code of the user, user input information, biometric information of the user, medical record information of the user, or exercise history information of the user), and recognize the disease of the user through the obtained information. For example, the processor 310 may recognize that the user has a cardiovascular disease and/or a musculoskeletal disorder through at least one or a combination of two or more of the disease code of the user, the user input information, the biometric information of the user, the medical record information of the user, or the exercise history information of the user.

In operation 1803, the wearable device 110 (e.g., the processor 310) may measure (or estimate) the physical fitness of the user. The processor 310 may control the wearable device 110 to operate in a physical ability measurement mode to measure the physical fitness (or the physical ability) (e.g., the walking ability, muscle strength, balance, possibility of falls, etc.) of the user. For example, the processor 310 may obtain sensor data (e.g., the angle values obtained by the angle sensors 320 and 320-1 and/or the motion information obtained by the IMU 360) according to a motion of the user. The processor 310 may measure or estimate the physical fitness (or the physical ability) of the user by analyzing the sensor data. As another example, the processor 310 may cause the user to get a Timed Up and Go (TUG) test. In the TUG test, the time that the user sitting on a chair wearing the wearable device 110 takes to rise from the chair, walk a predetermined (or certain) distance (e.g., "3" meters), walk back to the chair, and sit down on the chair may be measured. The processor 310 may measure or estimate the physical ability (e.g., the mobility, walking ability, fall risk, etc.) of the user based on the measured time. As another example, the processor 310 may cause the user to get a 5 Times Sit to Stand (5xSTS) test. In the 5xSTS test, the time that the user sitting on a chair wearing the wearable device 110 takes to perform a motion of standing and sitting a predetermined (or a certain) number of times (e.g., "5" times) may be measured. The processor 310 may measure or estimate the physical ability (e.g., the leg strength) of the user based on the measured time. Examples of measuring the physical fitness of the user are not limited to the examples described above.

In operation 1805, the wearable device 110 (e.g., the processor 310) may determine an exercise schedule (or an exercise program) of the user based on the disease and fitness measurement result of the user. The processor 310 may generate and provide an exercise schedule customized to the user based on the disease and fitness measurement result of the user. The processor 310 may further consider the age of the user when determining the exercise schedule (or the exercise program).

Tables 1 to 4 below show examples of exercise schedules for diseases.

**[Table 1]**

| | First period of time (e.g., 1 to 2 weeks) | | | | Wearable device | |
|---|---|---|---|---|---|---|
| Disease | Number of sets | Time | Distance | Speed | Exercise mode | Exercise intensity |
| Hypertension | 3 | 20 min | 1.6 km | 80 m/min | Interval walking exercise mode | 1 |
| Diabetes | 3 | 10 min | 1 km | 100 m/min | Interval walking exercise mode | 2 |
| Hyperlipidemia | 3 | 20 min | 1.6 km | 80 m/min | Interval walking exercise mode | 2 |
| Arthritis | 3 | 20 min | 1.6 km | 80 m/min | Damping exercise mode | 1 |
| Back pain | 3 | 20 min | 1.6 km | 80 m/min | Muscle strengthening exercise mode | 1 |

**[Table 2]**

| | Second period of time (e.g., 3 to 6 weeks) | | | | Wearable device | |
|---|---|---|---|---|---|---|
| Disease | Number of sets | Time | Distance | Speed | Exercise mode | Exercise intensity |
| Hypertension | 4 | 20 min | 2 km | 100 m/min | Interval walking exercise mode | 1 |
| Diabetes | 4 | 20 min | 2 km | 100 m/min | Interval walking exercise mode | 2 |
| Hyperlipidemia | 4 | 30 min | 2.4 km | 80 m/min | Interval walking exercise mode | 2 |
| Arthritis | 4 | 30 min | 2.4 km | 80 m/min | Damping exercise mode | 1 |
| Back pain | 4 | 30 min | 2.4 km | 80 m/min | Muscle strengthening exercise mode | 1 |

**[Table 3]**

| | Third period of time (e.g., 7 to 10 weeks) | | | | Wearable device | |
|---|---|---|---|---|---|---|
| Disease | Number of sets | Time | Distance | Speed | Exercise mode | Exercise intensity |
| Hypertension | 4 | 30 min | 3 km | 100 m/min | Interval walking exercise mode | 2 |
| Diabetes | 5 | 30 min | 3 km | 100 m/min | Interval walking exercise mode | 2 |
| Hyperlipidemia | 5 | 40 min | 4 km | 100 m/min | Interval walking exercise mode | 3 |
| Arthritis | 5 | 40 min | 4 km | 100 m/min | Damping exercise mode | 2 |
| Back pain | 5 | 40 min | 3 km | 70 m/min | Muscle strengthening exercise mode | 2 |

**[Table 4]**

| | Fourth period of time (e.g., 11 to 12 weeks) | | | | Wearable device | |
|---|---|---|---|---|---|---|
| Disease | Number of sets | Time | Distance | Speed | Exercise mode | Exercise intensity |
| Hypertension | 5 | 40 min | 4 km | 100 m/min | Interval walking exercise mode | 3 |
| Diabetes | 5 | 40 min | 4 km | 100 m/min | Interval walking exercise mode | 2 |
| Hyperlipidemia | 5 | 50 min | 5 km | 100 m/min | Interval walking exercise mode | 3 |
| Arthritis | 5 | 50 min | 5 km | 100 m/min | Damping exercise mode | 3 |
| Back pain | 5 | 50 min | 5 km | 80 m/min | Muscle strengthening exercise mode | 3 |

When the disease of the user is hypertension, diabetes, and/or hyperlipidemia, the processor 310 may generate a schedule for an interval walking exercise, provide the schedule to the user, and operate in an interval walking exercise mode when the user performs an interval walking exercise.

When the disease of the user is arthritis (e.g., knee pain), the processor 310 may generate a schedule for a walking exercise, provide the schedule to the user, and operate in a damping exercise mode when the user performs a walking exercise.

When the disease of the user is back pain, the processor 310 may generate a schedule for a walking exercise, provide the schedule to the user, and operate in a muscle strengthening exercise mode when the user performs a walking exercise.

The embodiment in which the wearable device generates an exercise schedule has been described with reference to FIG. 18. However, embodiments are not limited thereto, and an exercise schedule of the user may be generated by the electronic device 120 and/or the server 140. For example, the electronic device 120 (or the server 140) may obtain information necessary or helpful for recognizing the disease of the user (e.g., at least one of a disease code of the user, user input information, biometric information of the user, medical record information of the user, or exercise history information of the user), and recognize the disease of the user using the obtained information. The electronic device 120 (or the server 140) may link with the wearable device 110 to obtain fitness measurement information (or physical ability information) obtained by measuring the physical fitness of the user. The electronic device 120 (or the server 140) may request the wearable device 110 to measure the physical fitness of the user, and the wearable device 110 may perform operation 1803 described above, and transmit the fitness measurement result to the electronic device 120 (or the server 140). The electronic device 120 (or the server 140) may receive sensor data according to the motion of the user from the wearable device 110, and measure or estimate the physical fitness (or the physical ability) of the user by analyzing the received sensor data. The electronic device 120 (or the server 140) may determine an exercise schedule (or an exercise program) of the user based on the recognized disease and the fitness measurement result. The electronic device 120 (or the server 140) may control the wearable device 110 to operate in an exercise mode according to the exercise schedule when the user performs an exercise according to the exercise schedule.

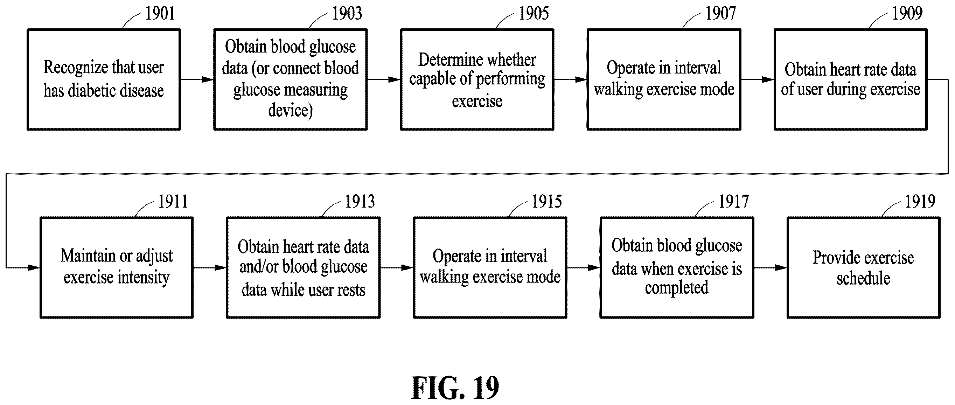

FIG. 19 is a diagram illustrating an example of an operation of a wearable device according to an embodiment.

Referring to FIG. 19, in operation 1901, the wearable device 110 may recognize that a user has a diabetic disease. For example, the wearable device 110 may recognize that the user has a diabetic disease through at least one of the diabetic disease code of the user received from the server 140, the name of the disease (e.g., diabetes) input by the user, or medical record information of the user.

In operation 1903, the wearable device 110 may obtain blood glucose data of the user. For example, the wearable device 110 may check whether the wearable device 110 is connectable to the smart watch 132 (or a blood glucose measuring device) capable of measuring blood glucose. The wearable device 110 may be connectable to the smart watch 132 (or the blood glucose measuring device), request the smart watch 132 (or the blood glucose measuring device) to measure the blood glucose, and receive the blood glucose data of the user before starting an exercise from the smart watch 132 (or the blood glucose measuring device). When the wearable device 110 is not connected to the smart watch 132 (or a blood glucose measuring device) capable of measuring blood glucose, the wearable device 110 may check recent blood glucose data of the user from the medical record information.

In operation 1905, the wearable device 110 may determine whether the user is capable of performing an exercise. For example, the wearable device 110 may check whether the user has eaten food, and when the user has not eaten food, provide the user with a guide to performing an exercise after eating food. When it is checked that the user has eaten food, and the blood glucose data checked in operation 1903 is within a predetermined (or certain) range (e.g., 100 to 250 mg/d), the wearable device 110 may determine that the user is capable of performing an exercise. When the blood glucose data checked in operation 1903 is out of the predetermined (or certain) range (e.g., 100 to 250 mg/d), the wearable device 110 may determine that the user is incapable of performing an exercise. The wearable device 110 may check that the user is in a hypoglycemic state when the blood glucose data checked in operation 1903 is lower than the predetermined range. In this case, the wearable device 110 may determine that the user is incapable of performing an exercise.

When it is determined that the user is capable of performing an exercise, the wearable device 110 may operate in an interval walking exercise mode, in operation 1907. For example, the wearable device 110 may recommend an exercise (e.g., an interval walking exercise) based on the diabetic disease of the user, and operate in an interval walking exercise mode to provide a torque (e.g., an assistance torque or a resistance torque) to the interval walking exercise of the user. The interval walking exercise mode in operation 1907 may be, for example, an interval walking exercise mode set with exercise intensity (e.g., Level 1)/duration of exercise (e.g., 15 minutes per set)/number of sets (e.g., 2 sets). The wearable device 110 may provide the user with a torque corresponding to the exercise intensity (e.g., Level 1), and provide the user with the exercise time left and the number of sets left.

In operation 1907, the wearable device 110 may provide the user with a guide to an interval walking exercise (e.g., a guide to walking while tightening the hips and thighs). Such a guide may include, for example, at least one of an auditory guide (e.g., a voice output), a visual guide (e.g., a visual output), or a tactile guide (e.g., a vibration output).

For example, when the wearable device 110 is connected to the wireless earphones 131, the wearable device 110 may generate a voice signal for the guide to an interval walking exercise, and transmit the generated voice signal to the wireless earphones 131. The wireless earphones 131 may output the received voice signal. Accordingly, the user may be provided with an auditory guide to an interval walking exercise.

For example, when the wearable device 110 is connected to the smart watch 132 and/or the smart glasses 133, the wearable device 110 may generate a message for the guide to an interval walking exercise, and transmit the generated message to the smart watch 132 and/or the smart glasses 133. The smart watch 132 and/or the smart glasses 133 may display the received message on the display. Accordingly, the user may be provided with a visual guide to an interval walking exercise.

For example, the wearable device 110 may include a vibration module (or a haptic module). The wearable device 110 may generate a vibration pattern (or a haptic pattern) for the guide to an interval walking exercise, and control the vibration module (or the haptic module) so that the vibration module (or the haptic module) may output vibration according to the vibration pattern (or the haptic pattern). Accordingly, the user may be provided with a tactile guide to an interval walking exercise.

In operation 1909, the wearable device 110 may obtain heart rate data of the user during exercise. For example, the wearable device 110 may request the connected smart watch 132 to measure the heart rate of the user, and receive the heart rate data of the user during exercise from the smart watch 132.

In operation 1911, the wearable device 110 may maintain or adjust the exercise intensity. For example, the wearable device 110 may increase the exercise intensity (e.g., adjust the exercise intensity from Level 1 to Level 2) when the heart rate data obtained in operation 1909 is stable. When the exercise intensity is adjusted to Level 2, the magnitude of a torque (e.g., a resistance torque) provided to the user may be increased to the magnitude corresponding to Level 2. The wearable device 110 may maintain the exercise intensity when the heart rate data obtained in operation 1909 is unstable.

The wearable device 110 may provide the user with a guide to rest. For example, the wearable device 110 may provide the user with a guide to rest when one set of interval walking exercises is terminated.

In operation 1913, the wearable device 110 may obtain the heart rate data and/or blood glucose data of the user while the user rests. While the user rests, the wearable device 110 may temporarily stop providing the torque to the user.

In operation 1915, the wearable device 110 may operate in an interval walking exercise mode. The number of sets of interval walking exercises may be set to "2", for example. In this case, the wearable device 110 may provide the user with a guide to the last set of interval walking exercises. When the exercise intensity is increased in operation 1911, the wearable device 110 may operate in an interval walking exercise mode with the increased exercise intensity.

The description of operation 1907 may apply to the description of operation 1915.

The wearable device 110 may obtain the heart rate data of the user performing the exercise, increase the exercise intensity when the heart rate data is stable, and maintain the exercise intensity when the heart rate data is unstable.

The interval walking exercise of the user may be terminated. In this case, the wearable device 110 may obtain blood glucose data (and/or heart rate data) of the user having completed the exercise, in operation 1917.

The wearable device 110 may transmit, to the server 140, the evaluation result of the interval walking exercise of the user, the blood glucose data (e.g., the blood glucose data before exercise, during rest, and after exercise), and the heart rate data (e.g., the heart rate data during exercise, during rest, and after exercise is terminated).

In operation 1919, the wearable device 110 may generate an exercise schedule for the user and provide the exercise schedule to the user. For example, the wearable device 110 may determine the physical fitness (or physical ability) of the user through the evaluation result of the interval walking exercise of the user, generate an exercise schedule (e.g., the exercise schedule for diabetic diseases in Tables 1 to 4) according to the physical fitness and the diabetic disease of the user, and provide the exercise schedule to the user. The wearable device 110 may guide the user to perform an exercise according to the exercise schedule of the user.

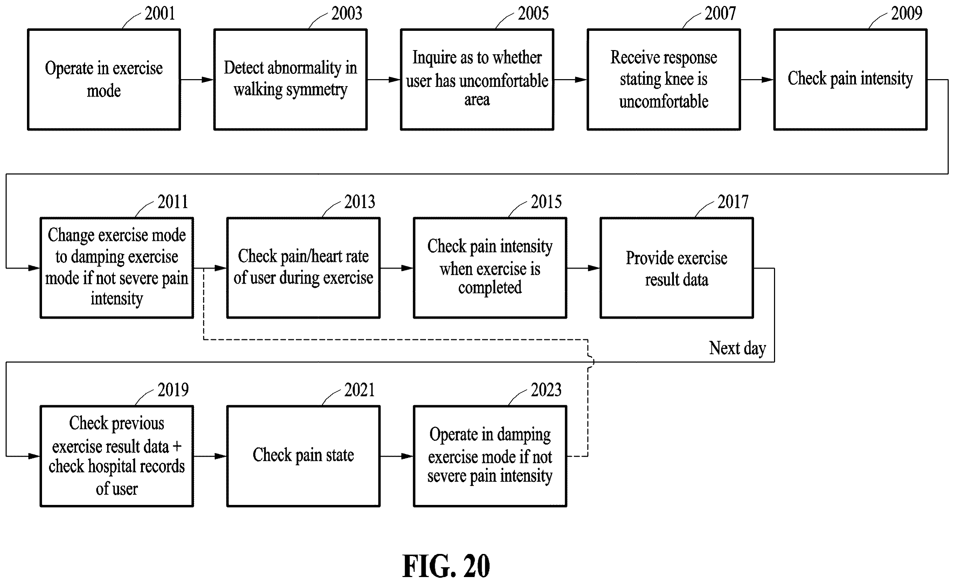

FIG. 20 is a diagram illustrating an example of an operation of a wearable device according to an embodiment.

Referring to FIG. 20, in operation 2001, the wearable device 110 may operate in an exercise mode (e.g., an assistance mode or a resistance mode). The wearable device 110 may provide a torque (e.g., an assistance torque or a resistance torque) to the user performing an exercise (e.g., a walking exercise). The exercise intensity may be, for example, Level 4. The wearable device 110 may calculate the walking time of a left step and the walking time of a right step of the user based on sensor data according to the walking exercise of the user (e.g., sensor data measured by the angle sensors 320 and 320-1 and/or the IMU 360), and calculate a walking symmetry based on the difference between the walking times. According to embodiments, the wearable device 110 may transmit the sensor data according to the walking exercise of the user (e.g., the sensor data measured by the angle sensors 320 and 320-1 and/or the IMU 360) to the electronic device 120 and/or the server 140. The electronic device 120 and/or the server 140 may calculate the walking time of a left step and the walking time of a right step of the user based on the received sensor data, and calculate the walking symmetry based on the difference between the walking times. The wearable device 110 may receive the walking symmetry from the electronic device 120 and/or the server 140.

The calculated walking symmetry may be, for example, "Right 2%". The walking symmetry of Right 2% may indicate that the walking time of the right foot is 2% longer than the walking time of the left foot. "Right 2%" may be within the range of balance (e.g., 0 to 6%).

In operation 2003, the wearable device 110 may detect an abnormality in the walking symmetry. For example, the wearable device 110 may check that the walking symmetry of the user changes from "Right 2%" to "Right 5%", and check that the walking symmetry changes to "Right 10%" which is out of the range of balance. The wearable device 110 may determine that the walking symmetry has an abnormality when the walking symmetry of the user is out of the range of balance (or the walking symmetry of the user is out of the range of balance for a predetermined (or certain) period of time).

When an abnormality in the walking symmetry is detected, the wearable device 110 may inquire as to whether the user has any uncomfortable area, in operation 2005, and receive a response stating that the knee is uncomfortable, in operation 2007.

For example, the wearable device 110 may be wirelessly connected to the wireless earphones 131. When an abnormality in the walking symmetry is detected, the wearable device 110 may generate a voice signal to inquire whether the user has any uncomfortable area, and transmit the generated voice signal to the wireless earphones 131. The wireless earphones 131 may output the received voice signal. The user may hear the output voice signal and say that the knee is uncomfortable. In other words, the user may perform an utterance stating that the knee is uncomfortable. The wireless earphones 131 may obtain a voice signal of the user by receiving the utterance stating that the knee is uncomfortable from the user, and transmit the voice signal of the user to the wearable device 110. The wearable device 110 may process the voice signal of the user received from the wireless earphones 131, and recognize that the knee of the user is uncomfortable through the processing result.

For example, the wearable device 110 may not be connected to the wireless earphones 131. In this case, the wearable device 110 may output a voice signal to inquire whether the user has any uncomfortable area through the speaker. The user may perform an utterance stating that the knee is uncomfortable. The wearable device 110 may obtain a voice signal of the user by receiving the utterance stating that the knee is uncomfortable from the user through the microphone. The wearable device 110 may process the voice signal of the user, and recognize that the knee of the user is uncomfortable through the processing result.

For example, the wearable device 110 may be wirelessly connected to at least one of the electronic device 120, the smart watch 132, or the smart glasses 133. When an abnormality in the walking symmetry is detected, the wearable device 110 may generate a message to inquire whether the user has any uncomfortable area, and transmit the generated message to at least one of the electronic device 120, the smart watch 132, or the smart glasses 133. At least one of the electronic device 120, the smart watch 132, or the smart glasses 133 may display the received message on the display. The user may input that the knee is uncomfortable into at least one of the electronic device 120, the smart watch 132, or the smart glasses 133, and at least one of the electronic device 120, the smart watch 132, or the smart glasses 133 may transmit, to the wearable device 110, the input indicating that the knee of the user is uncomfortable. The wearable device 110 may receive the input indicating that the knee of the user is uncomfortable, thereby recognizing that the knee of the user is uncomfortable.

In operation 2009, the wearable device 110 may check a pain intensity.

For example, the wearable device 110 may inquire the user about the pain area and/or the pain intensity in a voice through the speaker of the wearable device 110 or the wireless earphones 131. At this time, the wearable device 110 may inquire the user about a score of the pain intensity that the user feels on the numeric rating scale (e.g., 1 to 10 points). The wearable device 110 may receive a voice signal uttering the score corresponding to the pain intensity that the user feels from the user and check the score corresponding to the pain intensity that the user feels by processing the voice signal. As another example, the wearable device 110 may inquire the user about the pain intensity through the display of the electronic device 120 (or the smart watch 132 or the smart glasses 133). The electronic device 120 (or the smart watch 132 or the smart glasses 133) may receive the score corresponding to the pain intensity that the user feels from the user. The wearable device 110 may receive the score corresponding to the pain intensity that the user feels from the electronic device 120 (or the smart watch 132 or the smart glasses 133).

The wearable device 110 may determine that the user feels a mild pain intensity when the score corresponding to pain intensity falls within, for example, a first score range (e.g., 1 to 4 points), and determine that the user feels a moderate pain intensity when the score corresponding to pain intensity falls within, for example, a second score range (e.g., 5 to 6 points). The processor 310 may determine that the user feels a severe pain intensity when the score corresponding to the pain intensity falls within, for example, a third score range (e.g., 7 to 10 points).

When it is determined that the user feels a severe pain intensity, the wearable device 110 may provide a guide indicating that the user needs to see a doctor to the user.

In operation 2011, the wearable device 110 may change the exercise mode (e.g., the assistance mode or the resistance mode) to a damping exercise mode if the pain intensity of the user is not a severe pain intensity. The wearable device 110 may operate in the damping exercise mode for the remaining exercise interval so that the user may perform the exercise feeling less pain. The wearable device 110 may recommend a forward walking exercise to the user if the pain intensity of the user is a mild pain intensity. The wearable device 110 may recommend an in-place exercise (e.g., a squat exercise, a walking-in-place exercise, etc.) to the user if the pain intensity of the user is a moderate pain intensity. According to embodiments, the wearable device 110 may determine the exercise intensity when the pain intensity of the user is a mild pain intensity to be higher than the exercise intensity when the pain intensity of the user is a moderate pain intensity.

In operation 2013, the wearable device 110 may check the pain intensity and heart rate of the user performing the exercise. The description of operation 2009 may apply to checking the pain intensity in operation 2013. For example, the wearable device 110 may transmit a request for measuring the heart rate to the smart watch 132, and receive the heart rate data of the user during exercise from the smart watch 132. According to embodiments, the wearable device 110 may increase the exercise intensity when the heart rate data of the user is stable, and maintain the exercise intensity when the heart rate data of the user is unstable.

The exercise of the user may be terminated. In operation 2015, the wearable device 110 may check the pain intensity of the user when the exercise of the user is completed. The description of operation 2009 may apply to checking the pain intensity in operation 2015.

In operation 2017, the wearable device 110 may provide the user with exercise result data (e.g., the evaluation result of the exercise). The wearable device 110 may store the exercise result data (e.g., the evaluation result of the exercise) in the memory 350 and/or a cloud server (e.g., the server 140). The exercise result data of the user may be accumulated in the memory 350 and/or the cloud server (e.g., the server 140), and thus, the history of exercises performed by the user may be recorded. The wearable device 110 may store the pain state of the user during exercise (e.g., the pain area and/or the pain intensity) and the pain state after the exercise is terminated in the memory 350 and/or the cloud server (e.g., the server 140).

In operation 2019, the wearable device 110 may check the hospital records (or medical history) of the user and previous exercise result data from the memory 350 and/or the cloud server (e.g., the server 140). For example, the wearable device 110 may access the server 140 to check the previous exercise result data. When the user visits a hospital, the server 140 may link with the hospital visited by the user and store the hospital records (or medical history) of the user in the server 140. The wearable device 110 may access the server 140 to check the hospital records (or medical history) of the user.

In operation 2021, the wearable device 110 may check a pain state (e.g., a pain area and/or a pain intensity) of the user.

In operation 2023, the wearable device 110 may operate in the damping exercise mode if the pain intensity of the user is not a severe pain intensity. The wearable device 110 may perform operations 2013 to 2017.

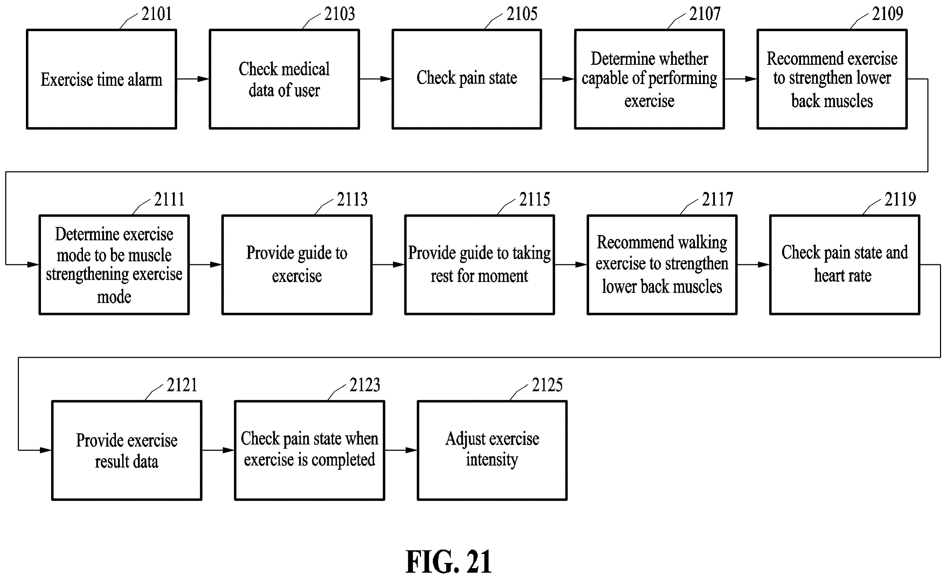

FIG. 21 is a diagram illustrating an example of an operation of a wearable device according to an embodiment.

Referring to FIG. 21, in operation 2101, the wearable device 110 may provide a user with an alarm of a scheduled exercise time (e.g., an exercise time alarm). The exercise time alarm may be performed by the electronic device 120 and/or the smart watch 132.

In operation 2103, the wearable device 110 may check medical data (or medical record information) of the user. For example, the wearable device 110 may check whether the server 140 has medical records (or updated medical record information) from a recent visit to a hospital. The wearable device 110 may identify the name of the disease of the user and the prescribed medication through the medical records.

In operation 2105, the wearable device 110 may check a pain state (e.g., a pain area and/or a pain intensity). The description of operations 915 and 2009 may apply to operation 2105. In the example shown in FIG. 21, the wearable device 110 may check that the pain area of the user is the lower back and the pain intensity is "2" on the numeric rating scale.

In operation 2107, the wearable device 110 may determine whether the user is capable of performing an exercise. The wearable device 110 may recognize that the pain intensity of the user is "2" and is not a severe pain intensity, and determine that the user is capable of performing an exercise.

In operation 2109, the wearable device 110 may recommend an exercise (e.g., an in-place muscle strengthening exercise and/or a walking exercise) to strengthen the lower back muscles. In the example shown in FIG. 21, the wearable device 110 may recommend a skating exercise (e.g., the skating exercise of FIG. 17) to the user.

In operation 2111, the wearable device 110 may determine the exercise mode to be a muscle strengthening exercise mode because the pain area of the user is the lower back. The wearable device 110 may provide a torque to the skating exercise of the user. At this time, the wearable device 110 may provide an appropriate magnitude of torque so that the user may maintain the posture of the skating exercise.

In operation 2113, the wearable device 110 may provide a guide to the exercise (e.g., the skating exercise). The guide may include, for example, at least one of an auditory guide, a visual guide, or a tactile guide. In operation 2113, the wearable device 110 may check at least one of the number of sets of the exercise, the exercise posture, or the duration of exercise. The wearable device 110 may provide the user with a guide to the exercise posture when the exercise posture of the user is incorrect.

In operation 2115, the wearable device 110 may provide the user with a guide to taking a rest for a moment.

In operation 2117, the wearable device 110 may recommend a walking exercise (e.g., a forward walking exercise) to strengthen the lower back muscles. The wearable device 110 may operate in the muscle strengthening exercise mode to strengthen the posterior thigh and lower back muscles of the user.

In operation 2119, the wearable device 110 may check the pain state and heart rate of the user during exercise.

The forward walking exercise of the user may be terminated.

In operation 2121, the wearable device 110 may provide the user with exercise result data (e.g., the evaluation result of the skating exercise and the evaluation result of the forward walking exercise), and store the exercise result data in the memory 350 and/or the cloud server (e.g., the server 140). The wearable device 110 may check a walking symmetry when the user performs a forward walking exercise.

In operation 2123, the wearable device 110 may check the pain state of the user when the exercise is completed. The wearable device 110 may check that the pain intensity checked in operation 2123 is the same as the pain intensity checked during exercise (e.g., the pain intensity checked in operation 2119) and the pain intensity checked before the start of the exercise (e.g., the pain intensity checked in operation 2105). In this case, the wearable device 110 may adjust the exercise intensity in operation 2125. For example, the wearable device 110 may provide the user with a lower exercise intensity when the user next performs a lower back muscle strengthening exercise. According to embodiments, the pain intensity checked after the exercise is terminated may be lower than the pain intensity checked during exercise (e.g., the pain intensity checked in operation 2119) and the pain intensity checked before the start of the exercise (e.g., the pain intensity checked in operation 2105). In this case, the wearable device 110 may maintain the exercise intensity or provide the user with a higher exercise intensity. The pain intensity checked after the exercise is terminated may be higher than the pain intensity checked during exercise (e.g., the pain intensity checked in operation 2119) and the pain intensity checked before the start of the exercise (e.g., the pain intensity checked in operation 2105). In this case, the wearable device 110 may provide the user with a lower exercise intensity or request the user to see a doctor.

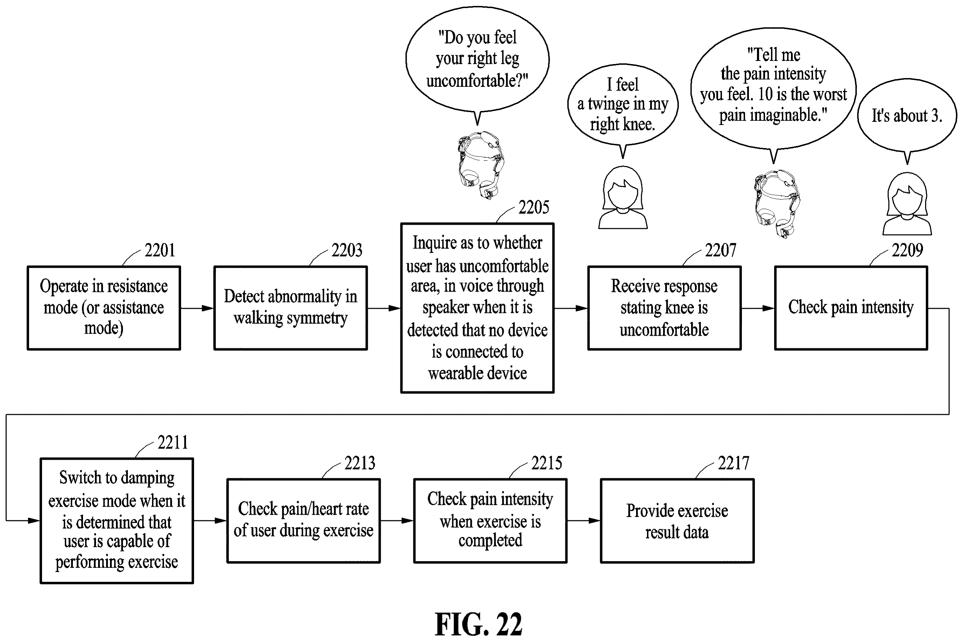

FIG. 22 is a diagram illustrating an example of an operation of a wearable device according to an embodiment.

Referring to FIG. 22, the wearable device 110 may operate in a resistance mode (or an assistance mode) in operation 2201 and, detect an abnormality in the walking symmetry in operation 2203. The description of operations 2001 and 2003 may apply to operations 2201 and 2203, respectively.

In operation 2205, the wearable device 110 may inquire as to whether the user has any uncomfortable area, in a voice through the speaker when it is detected that no device is connected to the wearable device 110.

In operation 2207, the wearable device 110 may receive a response stating that the knee is uncomfortable from the user. For example, the user may perform an utterance stating that the knee is uncomfortable. The wearable device 110 may obtain a voice signal of the user by receiving the utterance stating that the knee is uncomfortable from the user through the microphone. The wearable device 110 may process the voice signal of the user, and recognize that the knee of the user is uncomfortable through the processing result.

In operation 2209, the wearable device 110 may check a pain intensity of the user. In the example shown in FIG. 22, the wearable device 110 may check that the pain intensity of the user is "3" on the numeric rating scale.

In operation 2211, the wearable device 110 may switch the exercise mode in operation 2201 to a damping exercise mode when it is determined through the pain intensity of the user that the user is capable of performing an exercise. The description of operation 2011 may apply to operation 2211.

In operation 2213, the wearable device 110 may check the pain intensity and heart rate of the user performing the exercise. The description of operation 2013 may apply to operation 2213.

The exercise of the user may be terminated. In operation 2215, the wearable device 110 may check the pain intensity of the user when the exercise of the user is completed. The description of operation 2015 may apply to operation 2215.

In operation 2217, the wearable device 110 may provide the user with exercise result data (e.g., the evaluation result of the exercise). The description of operation 2017 may apply to operation 2217.

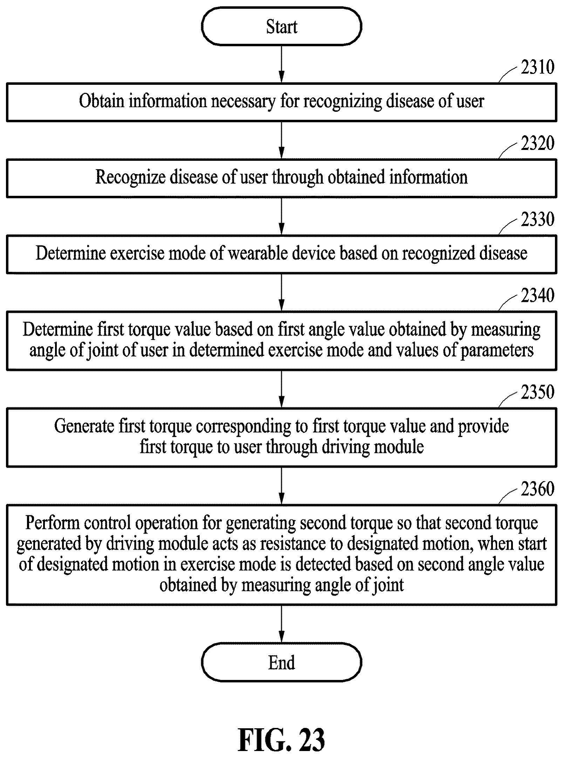

FIG. 23 is a flowchart illustrating an example of an operating method of a wearable device according to an embodiment.

Referring to FIG. 23, in operation 2310, the wearable device 110 may obtain information necessary or helpful for recognizing a disease of a user.

In operation 2320, the wearable device 110 may recognize the disease of the user through the obtained information.

In operation 2330, the wearable device 110 may determine an exercise mode of the wearable device based on the recognized disease.

In operation 2340, the wearable device 110 may determine a first torque value based on a first angle value obtained by measuring an angle of a joint (e.g., a hip joint or a knee joint) of the user in the determined exercise mode and values of parameters.

In operation 2350, the wearable device 110 may generate a first torque corresponding to the first torque value and provide the first torque to the user through the driving module 30.

In operation 2360, when a start of a designated motion in the exercise mode is detected based on a second angle value obtained by measuring the angle of the joint, the wearable device 110 may perform a control operation for generating a second torque so that the second torque generated by the driving module 30 may act as a resistance to the designated motion.

According to an embodiment, the exercise mode may include a first exercise mode (e.g., a damping exercise mode) of performing impact mitigation for a body part of the user and the control (or the control operation) for generating the second torque, and a second exercise mode (e.g., a muscle strengthening exercise mode) of performing muscle strengthening of the user and the control (or the control operation) for generating the second torque.

According to an embodiment, the control operation may include an operation of multiplying the determined torque value by a predetermined (or certain) value or changing the value of a first parameter among the parameters, when the start of a designated motion is detected.

According to an embodiment, the designated motion in the first exercise mode may include, for example, a first motion from when a rotation direction of the joint changes from a first direction (e.g., the forward direction) to a second direction (e.g., the backward direction) to when a foot of the user touches the ground. The designated motion in the second exercise mode may include, for example, a second motion from when the angle of the joint has a first value (e.g., 0 degrees) to when the rotation direction of the joint changes from the second direction to the first direction.

According to an embodiment, the processor 310 of the wearable device 110 may determine the exercise mode to be the first exercise mode through the recognized disease and the pain state of the user. When it is checked that the rotation direction of the joint of the user changes from the first direction to the second direction in the first exercise mode, the processor 310 may perform an operation of multiplying a second torque value (e.g., the torque value when the processor 310 checks that the rotation direction of the joint changes from the first direction to the second direction) by the predetermined (or certain) value, or an operation of changing the value of the first parameter among the parameters.

According to an embodiment, when it is checked that a foot of the user touches the ground, the processor 310 may perform an operation of not multiplying a third torque value (e.g., the torque value when the processor 310 checks that the foot touches the ground) by the predetermined (or certain) value, or an operation of returning the changed value of the first parameter to the original value.

According to an embodiment, the processor 310 may check (or detect) that the rotation direction of the joint changes from the first direction to the second direction through an angular velocity value of the joint.

According to an embodiment, the processor 310 may determine the exercise mode to be the second exercise mode through the recognized disease and the pain state of the user. When it is checked that the angle of the joint has a first value while the joint rotates in the second direction in the determined second exercise mode, the processor 310 may perform an operation of multiplying a fourth torque value (e.g., the torque value when the processor 310 checks that the angle of the joint has the first value while the joint rotates in the second direction) by the predetermined (or certain) value, or an operation of changing the value of the first parameter among the parameters.

According to an embodiment, when it is checked that the rotation direction of the joint changes from the second direction to the first direction, the processor 310 may perform an operation of not multiplying a fifth torque value (e.g., the torque value when the processor 310 checks that the rotation direction of the joint changes from the second direction to the first direction) by the predetermined (or certain) value, or an operation of returning the changed value of the first parameter to the original value.

According to an embodiment, the processor 310 may check (or detect) that the rotation direction of the joint changes from the first direction to the second direction through an angular velocity value of the joint.

According to an embodiment, the processor 310 may determine a walking symmetry of the user performing a walking exercise. When the determined walking symmetry is greater than or equal to a predetermined (or certain) level (e.g., when the determined walking symmetry is out of the range of balance), the processor 310 may control to provide the user with an inquiry about the pain state of the body part of the user. The processor 310 may obtain the pain state of the user as a response to the inquiry, and determine the exercise mode to be the first exercise mode based on the obtained pain state.

According to an embodiment, when it is recognized that the user has a musculoskeletal disorder and pain in a body part through the information necessary or helpful for recognizing the disease of the user, the processor 310 may determine the exercise mode to be the first exercise mode or the second exercise mode. When it is recognized that the user has a cardiovascular disease through the information necessary or helpful for recognizing the disease of the user, the processor 310 may obtain biometric information (e.g., at least one of blood glucose data, blood pressure data, or heart rate data), determine whether the user is capable of performing an exercise based on the obtained biometric information, and control to provide the user with a guide to a first exercise (e.g., an interval walking exercise suitable for cardiovascular diseases) when it is determined that the user is capable of performing an exercise.

According to an embodiment, the processor 310 may determine the exercise mode to be the first exercise mode when it is recognized that the user has pain in a first body part (e.g., the knees), and determine the exercise mode to be the second exercise mode when it is recognized that the user has pain in a second body part (e.g., the lower back).

According to an embodiment, the processor 310 may obtain heart rate information of the user while the user performs the first exercise (e.g., an interval walking exercise), and adjust an exercise intensity when the obtained heart rate information satisfies a predetermined (or certain) level (e.g., the heart rate is stable).

According to an embodiment, the wearable device 110 may further include a communication module to communicate with at least one of the server 140 or a user terminal (e.g., the electronic device 120 and/or the other wearable device 130). The processor 310 may receive the information necessary or helpful for recognizing the disease of the user from at least one of the server or the user terminal through the communication module.

The embodiments described with reference to FIGS. 1 to 22 may apply to the operating method of the wearable device 110 of FIG. 23.

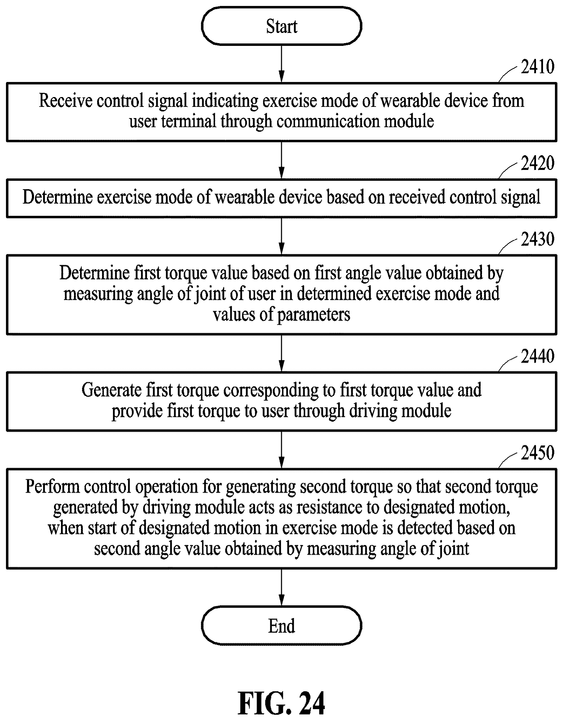

FIG. 24 is a flowchart illustrating an example of an operating method of a wearable device according to an embodiment.

Referring to FIG. 24, in operation 2410, the wearable device 110 (e.g., the processor 310) may receive a control signal indicating an exercise mode of the wearable device 110 from a user terminal (e.g., the electronic device 120 and/or the other wearable device 130) (or the server 140) through the communication module 390. For example, the user terminal (or the server 140) may obtain the information necessary or helpful for recognizing the disease of the user, recognize the disease (e.g., a cardiovascular disease, a musculoskeletal disorder, etc.) of the user through the obtained information, and determine the exercise mode of the wearable device 110 based on the recognized disease.

In operation 2420, the wearable device 110 (e.g., the processor 310) may determine the exercise mode (e.g., a first exercise mode or a second exercise mode) of the wearable device 110 based on the received control signal.

In operation 2430, the wearable device 110 may determine a first torque value based on a first angle value obtained by measuring an angle of a joint of the user in the determined exercise mode and values of parameters.

In operation 2440, the wearable device 110 may generate a first torque corresponding to the first torque value and provide the first torque to the user through the driving module 30.

In operation 2450, when a start of a designated motion in the exercise mode is detected based on a second angle value obtained by measuring the angle of the joint, the wearable device 110 may perform a control operation for generating a second torque so that the second torque generated by the driving module 30 may act as a resistance to the designated motion.

According to an embodiment, if the control signal indicates the first exercise mode, the processor 310 may determine the exercise mode to be the first exercise mode, and perform an operation of multiplying a second torque value (e.g., the torque value when it is checked that the rotation direction of the joint changes from the first direction to the second direction) by the predetermined (or certain) value, or an operation of changing the value of the first parameter among the parameters, when it is checked that the rotation direction of the joint of the user changes from the first direction to the second direction in the determined first exercise mode.

According to an embodiment, if the control signal indicates the second exercise mode, the processor 310 may determine the exercise mode to be the second exercise mode, and perform an operation of multiplying a third torque value (e.g., the torque value when it is checked that the angle of the joint has the first value while the joint rotates in the second direction) by the predetermined (or certain) value, or an operation of changing the value of the first parameter among the parameters, when it is checked that the angle of the joint has a first value while the joint rotates in the second direction in the determined second exercise mode.

The embodiments described with reference to FIGS. 1 to 22 may apply to the operating method of the wearable device 110 of FIG. 24.

The units described herein may be implemented using a hardware component, a software component and/or a combination thereof. A processing device may be implemented using one or more general-purpose or special-purpose computers, such as, for example, a processor, a controller and an arithmetic logic unit (ALU), a digital signal processor (DSP), a microcomputer, a field-programmable gate array (FPGA), a programmable logic unit (PLU), a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciate that a processing device may include multiple processing elements and multiple types of processing elements. For example, the processing device may include a plurality of processors, or a single processor and a single controller. In addition, different processing configurations are possible, such as parallel processors.

The software may include a computer program, a piece of code, an instruction, or some combination thereof, to independently or uniformly instruct or configure the processing device to operate as desired. Software and data may be embodied permanently or temporarily in any type of machine, component, physical or virtual equipment, computer storage medium or device, or in a propagated signal wave capable of providing instructions or data to or being interpreted by the processing device. The software also may be distributed over network-coupled computer systems so that the software is stored and executed in a distributed fashion. The software and data may be stored by one or more non-transitory computer-readable recording mediums.

The methods according to the above-described embodiments may be recorded in non-transitory computer-readable media including program instructions to implement various operations of the above-described embodiments. The media may also include, alone or in combination with the program instructions, data files, data structures, and the like. The program instructions recorded on the media may be those specially designed and constructed for the purposes of embodiments, or they may be of the kind well-known and available to those having skill in the computer software arts. Examples of non-transitory computer-readable media include magnetic media such as hard disks, floppy disks, and magnetic tape; optical media such as CD-ROM discs, DVDs, and/or Blue-ray discs; magneto-optical media such as optical discs; and hardware devices that are specially configured to store and perform program instructions, such as read-only memory (ROM), random access memory (RAM), flash memory (e.g., USB flash drives, memory cards, memory sticks, etc.), and the like. Examples of program instructions include both machine code, such as produced by a compiler, and files containing higher-level code that may be executed by the computer using an interpreter.

The above-described hardware devices may be configured to act as one or more software modules in order to perform the operations of the above-described examples, or vice versa.

A number of embodiments have been described above. Nevertheless, it should be understood that various modifications may be made to these embodiments. For example, suitable results may be achieved if the described techniques are performed in a different order, and/or if components in a described system, architecture, device, or circuit are combined in a different manner, or replaced or supplemented by other components or their equivalents. While the disclosure has been illustrated and described with reference to various embodiments, it will be understood that the various embodiments are intended to be illustrative, not limiting. It will further be understood by those skilled in the art that various changes in form and detail may be made without departing from the true spirit and full scope of the disclosure, including the appended claims and their equivalents. It will also be understood that any of the embodiment(s) described herein may be used in conjunction with any other embodiment(s) described herein.

Therefore, other implementations, other embodiments, and/or equivalents of the claims are within the scope of the following claims.

## Claims

1. A wearable device (110) comprising:
an angle sensor (320, 320-1) configured to measure an angle of a joint of a user;
a driving module (30), comprising a motor and/or circuitry, configured to generate a torque and provide the torque to the user; and
at least one processor (310), comprising processing circuitry, individually and/or collectively configured to:
obtain information for recognizing a disease of the user, and recognize a disease of the user based on the obtained information,
determine an exercise mode of the wearable device based on the recognized disease,
receive a first angle value based on the angle in the determined exercise mode from the angle sensor,
determine a first torque value based on the received first angle value and values of parameters,
control the driving module to generate a first torque based on the determined first torque value, and
when a start of a designated motion in the determined exercise mode is detected based on a second angle value obtained by measuring the angle, perform a control operation for generating a second torque so that the second torque generated by the driving module acts as a resistance to the designated motion.

2. The wearable device of claim 1, wherein the exercise mode comprises a first exercise mode of performing impact mitigation for a body part of the user and the control operation, and a second exercise mode of performing muscle strengthening of the user and the control operation.

3. The wearable device of claim 2, wherein
the designated motion in the first exercise mode comprises a first motion from when a rotation direction of the joint changes from a first direction to a second direction to when a foot of the user touches a ground, and
the designated motion in the second exercise mode comprises a second motion from when the angle of the joint has a first value to when the rotation direction of the joint changes from the second direction to the first direction.

4. The wearable device of any one of claims 1 to 3, wherein at least one processor, comprising processing circuitry, is individually and/or collectively configured to determine the exercise mode to be a first exercise mode based on the recognized disease and a pain state of the user, and when it is determined that a rotation direction of the joint of the user changes from a first direction to a second direction in the determined first exercise mode, perform an operation of multiplying a second torque value by a predetermined value and/or an operation of changing a value of a first parameter among the parameters.

5. The wearable device of claim 4, wherein at least one processor, comprising processing circuitry, is individually and/or collectively configured to, when it is determined that a foot of the user touches a ground, perform an operation of not multiplying a third torque value by the predetermined value and/or an operation of returning the changed value of the first parameter to the original value.

6. The wearable device of claim 4, wherein at least one processor, comprising processing circuitry, is individually and/or collectively configured to determine that the rotation direction changes from the first direction to the second direction through an angular velocity value of the joint.

7. The wearable device of any one of claims 1 to 6, wherein at least one processor, comprising processing circuitry, is individually and/or collectively configured to determine the exercise mode to be a second exercise mode based on the recognized disease and a pain state of the user, and when it is determined that the angle of the joint has a first value while the joint rotates in a second direction in the determined second exercise mode, perform an operation of multiplying a fourth torque value by a predetermined value and/or an operation of changing a value of a first parameter among the parameters.

8. The wearable device of claim 7, wherein at least one processor, comprising processing circuitry, is individually and/or collectively configured to, when it is determined that a rotation direction of the joint changes from the second direction to a first direction, perform an operation of not multiplying a fifth torque value by the predetermined value and/or an operation of returning the changed value of the first parameter to the original value.

9. The wearable device of claim 8, wherein at least one processor, comprising processing circuitry, is individually and/or collectively configured to determine that the rotation direction of the joint changes from the second direction to the first direction through an angular velocity value of the joint.

10. The wearable device of any one of claims 1 to 9, wherein at least one processor, comprising processing circuitry, is individually and/or collectively configured to determine a walking symmetry of the user performing a walking exercise, and when the determined walking symmetry is greater than or equal to a predetermined level, control to provide an inquiry about a pain state of a body part of the user to the user, obtain the pain state as a response to the inquiry, and determine the exercise mode to be a first exercise mode based on the obtained pain state.

11. The wearable device of any one of claims 1 to 10, wherein at least one processor, comprising processing circuitry, is individually and/or collectively configured to, when it is recognized that the user has a musculoskeletal disorder and pain in a body part through the obtained information, determine the exercise mode to be a first exercise mode and/or a second exercise mode, and when it is recognized that the user has a cardiovascular disease through the obtained information, obtain biometric information of the user, determine whether the user is capable of performing an exercise based on the obtained biometric information, and control to provide a guide to a first exercise to the user in response to the determination that the user is capable of performing the exercise.

12. The wearable device of claim 11, wherein at least one processor, comprising processing circuitry, is individually and/or collectively configured to, when it is recognized that the user has pain in a first body part, determine the exercise mode to be the first exercise mode, and when it is recognized that the user has pain in a second body part, determine the exercise mode to be the second exercise mode.

13. The wearable device of claim 11, wherein at least one processor, comprising processing circuitry, is individually and/or collectively configured to obtain heart rate information of the user while the user is performing the first exercise, and adjust an exercise intensity when the obtained heart rate information satisfies a predetermined level.

14. The wearable device of any one of claims 1 to 13, further comprising:
a communication module, comprising communication circuitry, configured to communicate with at least one of a server or a user terminal,
wherein at least one processor, comprising processing circuitry, is individually and/or collectively configured to receive the information for recognizing the disease of the user from at least one of the server or the user terminal through the communication module.

15. An operating method of a wearable device (110), the operating method comprising:
receiving a control signal indicating an exercise mode of the wearable device from the user terminal;
determining an exercise mode of the wearable device based on the received control signal;
determining a first torque value based on a first angle value obtained by measuring an angle of a joint of the user in the determined exercise mode and values of parameters
generating a first torque based on the determined first torque value, and
when a start of a designated motion in the determined exercise mode is detected based on a second angle value obtained by measuring the angle, performing a control operation for generating a second torque so that the second torque acts as a resistance to the designated motion.
